# EUROPÄISCHE PATENTSCHRIFT

(11) **EP 3 145 918 B1**
(45) Veröffentlichungstag und Bekanntmachung des Hinweises auf die Patenterteilung: **03.01.2018**
(21) Anmeldenummer: 15723916.1
(22) Anmeldetag: 19.05.2015
(51) Int. Cl.: C07D 237/18

(54) **2-(HETERO)ARYL-PYRIDAZINONE UND IHRE VERWENDUNG ALS HERBIZIDE**
2-(HETERO)ARYL PYRIDAZINONES AND THEIR USE AS HERBICIDES
2-(HÉTÉRO)ARYL-PYRIDAZINONES ET LEUR UTILISATION EN TANT QU'HERBICIDES

(30) Priorität: 21.05.2014 EP 14169229
(43) Veröffentlichungstag der Anmeldung: 29.03.2017
(73) Patentinhaber: Bayer CropScience Aktiengesellschaft, 40789 Monheim am Rhein (DE)
(72) Erfinder: AHRENS, Hartmut, 63329 Egelsbach (DE); TIEBES, Jörg, 60431 Frankfurt am Main (DE); WALDRAFF, Christian, 61118 Bad Vilbel (DE); DIETRICH, Hansjörg, 65835 Liederbach am Taunus (DE); SCHMUTZLER, Dirk, 65795 Hattersheim (DE); GATZWEILER, Elmar, 61231 Bad Nauheim (DE); ROSINGER, Christopher, 65719 Hofheim am Taunus (DE)
(74) Vertreter: BIP Patents
(86) Internationale Anmeldenummer: PCT/EP2015/060934
(87) Internationale Veröffentlichungsnummer: WO 2015/177108

(56) Entgegenhaltungen:
- EP-A1- 0 003 805
- EP-A1- 0 085 785
- EP-A1- 0 128 530
- WO-A1-2013/083774

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2013/083774 A1 sind Pyridazinone als Herbizide beschrieben. Dort werden auch Pyridazinone beschrieben, die in einer bestimmten Position eines Heteroaryl-Rings unter anderem einen Sulfonylrest tragen. Allerdings zeigen diese Wirkstoffe nicht immer eine ausreichende Wirkung gegen Schadpflanzen und/oder sie sind zum Teil nicht ausreichend verträglich mit einigen wichtigen Kulturpflanzen, wie Getreidearten, Mais und Reis.

Aufgabe der vorliegenden Erfindung ist es, alternative herbizid wirksame Wirkstoffe bereitzustellen. Diese Aufgabe wird durch Bereitstellung von 2-(Hetero)aryl-Pyridazinonen, die in einer bestimmten Position des (Hetero)aryl-Rings einen Schwefelrest tragen, gelöst.

Ein Gegenstand der vorliegenden Erfindung sind somit 2-(Hetero)aryl-Pyridazinone der Formel (I) oder deren Salze 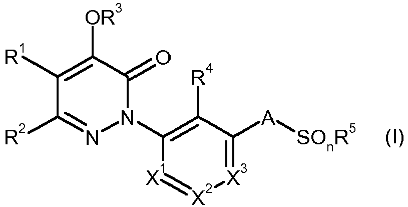 worin
R¹ bedeutet Wasserstoff, Halogen, Cyano, (C₁-C₆)-Alkyl, (C₃-C₆)-Cycloalkyl, (C₂-C₆)-Alkenyl, (C₄-C₆)-Cycloalkenyl, (C₂-C₆)-Alkinyl, Halogen-(C₁-C₆)-alkyl, (C₁-C₆)-Alkoxy, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, (C₃-C₆)-Cycloalkyl-(C₁-C₃)-alkyl, Amino, (C₁-C₆)-Alkylamino, Di-(C₁-C₆)-alkylamino, (C₁-C₃)-Alkyl-(O)C-amino-(C₁-C₄)-alkyl, (C₁-C₆)-Alkyl-(O)ₙS, (C₁-C₆)-Alkyl-(O)ₙS-(C₁-C₃)-alkyl, Halogen-(C₁-C₆)-alkyl-(O)ₙS oder Halogen-(C₁-C₆)-alkyl-(O)ₙS-(C₁-C₃)-alkyl;
R² bedeutet Wasserstoff, Hydroxy, Halogen, Nitro, Amino, Cyano, (C₁-C₆)-Alkyl, (C₁-C₃)-Alkoxy, (C₃-C₆)-Cycloalkyl, (C₂-C₆)-Alkenyl, (C₂-C₆)-Alkinyl, Halogen-(C₁-C₆)-alkyl, (C₁-C₆)-Alkoxy-(C₁-C₃)-alkyl, (C₃-C₆)-Cycloalkyl-(C₁-C₃)-alkyl, (C₁-C₆)-Alkyl-(O)ₙS, (C₁-C₆)-Alkyl-(O)ₙS-(C₁-C₃)-alkyl, Halogen-(C₁-C₆)-alkyl-(O)ₙS, Halogen-(C₁-C₆)-alkyl-(O)ₙS-(C₁-C₃)-alkyl, (C₁-C₃)-Alkylamino oder Di-(C₁-C₃)-alkylamino;
R³ bedeutet Wasserstoff, (C₁-C₆)-Alkyl-(O)C, Aryl-(O)C, (C₁-C₆)-Alkoxy-(O)C, (C₁-C₆)-Alkyl-(O)ₙS, (C₁-C₆)-Alkyl-(O)ₙS(O)C oder Aryl-(O)ₙS, wobei die Arylgruppen jeweils durch s Reste R⁹ substituiert sind;
R⁴ bedeutet Hydroxy, Halogen, Cyano, Nitro, (C₁-C₆)-Alkyl, (C₃-C₆)-Cycloalkyl, Halogen-(C₁-C₆)-alkyl, (C₂-C₆)-Alkenyl, Halogen-(C₂-C₆)-alkenyl, (C₂-C₆)-Alkinyl, (C₁-C₆)-Alkoxy, (C₂-C₆)-Alkenyloxy, (C₃-C₆)-Cycloalkyl-(C₁-C₃)-alkyl, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, Halogen-(C₁-C₆)-alkoxy, Halogen-(C₁-C₆)-alkoxy-(C₁-C₃)-alkyl, (C₁-C₆)-Alkyl-(O)ₙS, Halogen-(C₁-C₆)-alkyl-(O)ₙS, Aryl, Aryl-(O)ₙS, Heterocyclyl, Heterocyclyl-(O)ₙS, Aryloxy, Aryl-(C₂-C₆)-alkyl, Aryl-(C₁-C₆)-alkoxy, Heterocyclyloxy, Heterocyclyl-(C₁-C₃)-alkoxy-(C₁-C₃)-alkyl, HO(O)C, HO(O)C-(C₁-C₃)-alkoxy, (C₁-C₃)-Alkoxy-(O)C, (C₁-C₃)-Alkoxy-(O)C-(C₁-C₃)-alkoxy, (C₁-C₃)-Alkylamino, Di-(C₁-C₃)-alkylamino, (C₁-C₃)-Alkylamino-(O)ₙS, (C₁-C₃)-Alkylamino-(O)ₙS-(C₁-C₃)-alkyl, Di-(C₁-C₃)-alkylamino-(O)ₙS, Di-(C₁-C₃)-alkylamino-(O)ₙS-(C₁-C₃)-alkyl, (C₁-C₃)-Alkylamino-(O)C, (C₁-C₃)-Alkylamino-(O)C-(C₁-C₃)-alkyl, Di-(C₁-C₃)-alkylamino-(O)C, Di-(C₁-C₃)-alkylamino-(O)C-(C₁-C₃)-alkyl, (C₁-C₃)-Alkyl-(O)C-amino, (C₁-C₃)-Alkyl-(O)ₙS-amino, (C₁-C₃)-Alkyl-(O)ₙS-(C₁-C₃)-alkylamino oder (C₁-C₃)-Alkyl-(O)ₙS-amino-(C₁-C₃)-alkyl, wobei die Heterocyclylgruppen und Arylgruppen durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, Phenyl, Cyano, Nitro und Halogen substituiert sind;
A bedeutet eine direkte Bindung oder (C₁-C₄)-Alkylen, wobei die Methylengruppen in (C₁-C₄)-Alkylen unabhängig voneinander n Reste aus der Gruppe bestehend aus Halogen, (C₁-C₄)-Alkyl, Halogen-(C₁-C₄)-alkyl, (C₁-C₄)-Alkoxy, Halogen-(C₁-C₄)-alkoxy oder (C₁-C₄)-Alkoxy-(C₁-C₄)-alkyl tragen können;
R⁵ bedeutet (C₁-C₆)-Alkyl, (C₃-C₆)-Cycloalkyl, (C₃-C₆)-Cycloalkyl-(C₁-C₆)-alkyl, (C₁-C₆)-Alkoxy-(C₁-C₆)-alkyl;
X¹ bedeutet N oder CR⁶;
X² bedeutet N oder CR⁷;
X³ bedeutet N oder CR⁸;
R⁶ bedeutet Wasserstoff, Halogen, (C₁-C₃)-Alkyl, (C₁-C₃)-Alkoxy, (C₂-C₃)-Alkenyl, (C₂-C₃)-Alkinyl, Halogen-(C₁-C₃)-alkyl, Halogen-(C₁-C₃)-alkoxy;
R⁷ bedeutet Wasserstoff, Halogen, (C₁-C₃)-Alkyl;
R⁸ bedeutet Wasserstoff, Hydroxy, Halogen, Cyano, Nitro, (C₁-C₆)-Alkyl, (C₃-C₆)-Cycloalkyl, Halogen-(C₁-C₆)-alkyl, (C₂-C₆)-Alkenyl, Halogen-(C₂-C₆)-alkenyl, (C₂-C₆)-Alkinyl, (C₁-C₆)-Alkoxy, (C₂-C₆)-Alkenyloxy, (C₃-C₆)-Cycloalkyl-(C₁-C₃)-alkyl, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, Halogen-(C₁-C₆)-alkoxy, Halogen-(C₁-C₆)-alkoxy-(C₁-C₃)-alkyl, (C₁-C₆)-Alkyl-(O)ₙS, Halogen-(C₁-C₆)-alkyl-(O)ₙS, Aryl, Aryl-(O)ₙS, Heterocyclyl, Heterocyclyl-(O)ₙS, Aryloxy, Aryl-(C₂-C₆)-alkyl, Aryl-(C₁-C₆)-alkoxy, Heterocyclyloxy, Heterocyclyl-(C₁-C₃)-alkoxy-(C₁-C₃)-alkyl, HO(O)C, HO(O)C-(C₁-C₃)-alkoxy, (C₁-C₃)-Alkoxy-(O)C, (C₁-C₃)-Alkoxy-(O)C-(C₁-C₃)-alkoxy, (C₁-C₃)-Alkylamino, Di-(C₁-C₃)-alkylamino, (C₁-C₃)-Alkylamino-(O)ₙS, (C₁-C₃)-Alkylamino-(O)ₙS-(C₁-C₃)-alkyl, Di-(C₁-C₃)-alkylamino-(O)ₙS, Di-(C₁-C₃)-alkylamino-(O)ₙS-(C₁-C₃)-alkyl, (C₁-C₃)-Alkylamino-(O)C, (C₁-C₃)-Alkylamino-(O)C-(C₁-C₃)-alkyl, Di-(C₁-C₃)-alkylamino-(O)C, Di-(C₁-C₃)-alkylamino-(O)C-(C₁-C₃)-alkyl, (C₁-C₃)-Alkyl-(O)C-amino, (C₁-C₃)-Alkyl-(O)ₙS-amino, (C₁-C₃)-Alkyl-(O)ₙS-(C₁-C₃)-alkylamino oder (C₁-C₃)-Alkyl-(O)ₙS-amino-(C₁-C₃)-alkyl, wobei die Heterocyclylgruppen und Arylgruppen durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, (C₁-C₆)-Alkyl-(O)ₙS, Phenyl, Cyano, Nitro und Halogen substituiert sind,
   oder
R⁷ und R⁸ bilden gemeinsam mit den Kohlenstoffatomen, an denen sie gebunden sind, einen ungesättigten, fünf- oder sechsgliedrigen Ring, der s Stickstoffatome enthält und durch s Reste R¹⁰ substituiert ist;
R⁹ bedeutet Halogen, (C₁-C₃)-Alkyl, Halogen-(C₁-C₃)-alkyl, (C₁-C₆)-Alkoxy;
R¹⁰ bedeutet Cyano, Halogen, (C₁-C₃)-Alkyl-(O)ₙS, (C₁-C₃)-Alkyl, (C₂-C₃)-Alkenyl, (C₂-C₃)-Alkinyl, Halogen-(C₁-C₃)-alkyl oder Morpholinyl;
n bedeutet 0, 1 oder 2;
s bedeutet 0, 1, 2 oder 3,
mit der Maßgabe, dass R⁵ nicht (C₁-C₆)-Alkyl bedeutet, wenn A eine direkte Bindung ist.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten, vollständig ungesättigten oder aromatischen cyclischen Rest, der 3 bis 6 Ringatome enthält, von denen 1 bis 4 aus der Gruppe bestehend aus Sauerstoff, Stickstoff und Schwefel stammen, und der zusätzlich durch einen Benzoring annelliert sein kann. Beispielsweise steht Heterocyclyl für Piperidinyl, Pyrrolidinyl, Morpholinyl, Tetrahydrofuranyl, Dihydrofuranyl, Oxetanyl, Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Aryl bedeutet Phenyl oder Naphthyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf Verbindungen der Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Bevorzugt sind Verbindungen der allgemeinen Formel (I), worin
R¹ bedeutet Wasserstoff, Halogen, Cyano, (C₁-C₆)-Alkyl, (C₃-C₆)-Cycloalkyl, (C₂-C₆)-Alkenyl, (C₂-C₆)-Alkinyl, Halogen-(C₁-C₆)-alkyl, (C₁-C₆)-Alkoxy-(C₁-C₃)-alkyl, (C₃-C₆)-Cycloalkyl-(C₁-C₃)-alkyl, Amino oder (C₁-C₆)-Alkyl-(O)ₙS;
R² bedeutet Wasserstoff, Halogen, Cyano, (C₁-C₆)-Alkyl, (C₂-C₆)-Alkenyl, (C₂-C₆)-Alkinyl, Halogen-(C₁-C₆)-alkyl oder (C₁-C₆)-Alkyl-(O)ₙS;
R³ bedeutet Wasserstoff;
R⁴ bedeutet Hydroxy, Halogen, Cyano, Nitro, (C₁-C₆)-Alkyl, (C₃-C₆)-Cycloalkyl, Halogen-(C₁-C₆)-alkyl, (C₂-C₆)-Alkenyl, (C₂-C₆)-Alkinyl, (C₁-C₆)-Alkoxy, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, Halogen-(C₁-C₆)-alkoxy, Halogen-(C₁-C₆)-alkoxy-(C₁-C₃)-alkyl, (C₁-C₆)-Alkyl-(O)ₙS, Halogen-(C₁-C₆)-alkyl-(O)ₙS, Aryl, Heterocyclyl, Aryloxy, Heterocyclyl-(C₁-C₃)-alkoxy-(C₁-C₃)-alkyl, (C₁-C₃)-Alkylamino, Di-(C₁-C₃)-alkylamino, (C₁-C₃)-Alkylamino-(O)ₙS, (C₁-C₃)-Alkylamino-(O)ₙS-(C₁-C₃)-alkyl, Di-(C₁-C₃)-alkylamino-(O)ₙS, Di-(C₁-C₃)-alkylamino-(O)ₙS-(C₁-C₃)-alkyl, (C₁-C₃)-Alkylamino-(O)C, Di-(C₁-C₃)-alkylamino-(O)C, Di-(C₁-C₃)-alkylamino-(O)C-(C₁-C₃)-alkyl, (C₁-C₃)-Alkyl-(O)C-amino oder (C₁-C₃)-Alkyl-(O)ₙS-amino, wobei die Heterocyclylgruppen und Arylgruppen durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, Cyano, Nitro und Halogen substituiert sind;
A bedeutet eine direkte Bindung oder (C₁-C₄)-Alkylen;
R⁵ bedeutet (C₁-C₆)-Alkyl, (C₃-C₆)-Cycloalkyl, (C₃-C₆)-Cycloalkyl-(C₁-C₆)-alkyl, (C₁-C₆)-Alkoxy-(C₁-C₆)-alkyl;
X¹ bedeutet CR⁶;
X² bedeutet CR⁷;
X³ bedeutet CR⁸;
R⁶ und R⁷ bedeuten unabhängig voneinander Wasserstoff, Halogen oder (C₁-C₃)-Alkyl;
R⁸ bedeutet Wasserstoff, Halogen, Nitro, (C₁-C₆)-Alkyl, (C₃-C₆)-Cycloalkyl, Halogen-(C₁-C₆)-alkyl, (C₂-C₆)-Alkenyl, Halogen-(C₂-C₆)-alkenyl, (C₂-C₆)-Alkinyl, (C₁-C₆)-Alkoxy, (C₂-C₆)-Alkenyloxy, (C₃-C₆)-Cycloalkyl-(C₁-C₃)-alkyl, (C₁-C₆)-Alkoxy-(C₁-C₃)-alkyl, (C₁-C₆)-Alkoxy-(C₂-C₆)-alkoxy, Halogen-(C₁-C₆)-alkoxy, (C₁-C₆)-Alkyl-(O)ₙS oder Phenyl, wobei die Phenylgruppe durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, (C₁-C₆)-Alkyl-(O)ₙS, Phenyl, Cyano, Nitro und Halogen substituiert ist;
n bedeutet 0, 1 oder 2;
s bedeutet 0, 1, 2 oder 3,
mit der Maßgabe, dass R⁵ nicht (C₁-C₆)-Alkyl bedeutet, wenn A eine direkte Bindung ist.

Besonders bevorzugt sind Verbindungen der allgemeinen Formel (I), worin
R¹ bedeutet Wasserstoff, Amino, Chlor, Brom, Cyano, Methyl, Ethyl, Isopropyl, Cyclopropyl, Vinyl, Propargyl, Isopropenyl oder Methyl-(O)ₙS;
R² bedeutet Wasserstoff, Halogen oder (C₁-C₆)-Alkyl;
R³ bedeutet Wasserstoff;
R⁴ bedeutet Fluor, Chlor, Cyano, Nitro, Methyl, Trifluormethyl, 2-Fluorethyl, Methoxyethoxymethyl, Trifluormethoxymethyl, Methyl-(O)ₙS, Aryl, Isoxazolinyl, Morpholinyl oder Methyl-(O)ₙS-amino, wobei die Heterocyclylgruppen und Arylgruppen durch s Reste aus der Gruppe bestehend aus Methyl, Trifluormethyl und Chlor substituiert sind;
A bedeutet eine direkte Bindung oder (C₁-C₄)-Alkylen;
R⁵ bedeutet (C₁-C₆)-Alkyl, (C₃-C₆)-Cycloalkyl, (C₃-C₆)-Cycloalkyl-(C₁-C₆)-alkyl, (C₁-C₆)-Alkoxy-(C₁-C₆)-alkyl;
X¹ bedeutet CR⁶;
X² bedeutet CR⁷;
X³ bedeutet CR⁸;
R⁶ und R⁷ bedeuten Wasserstoff;
R⁸ bedeutet Wasserstoff, Halogen, (C₁-C₆)-Alkyl, Halogen-(C₁-C₆)-alkyl, (C₂-C₆)-Alkenyl, (C₂-C₆)-Alkinyl, oder (C₁-C₆)-Alkyl-(O)ₙS;
n bedeutet 0, 1 oder 2;
s bedeutet 0, 1, 2 oder 3,
mit der Maßgabe, dass R⁵ nicht (C₁-C₆)-Alkyl bedeutet, wenn A eine direkte Bindung ist.

Ganz besonders bevorzugt sind Verbindungen der allgemeinen Formel (I), worin
R¹ bedeutet Methyl oder Vinyl;
R² bedeutet Wasserstoff;
R³ bedeutet Wasserstoff;
R⁴ bedeutet Methyl, Chlor, Trifluormethyl oder Methyl-(O)ₙS;
A bedeutet eine direkte Bindung, -CH₂- oder -CH₂CH₂-;
R⁵ bedeutet Methyl, Ethyl, Cyclopropyl, Cyclopropylmethyl, Methoxyethyl;
X¹ bedeutet CR⁶;
X² bedeutet CR⁷;
X³ bedeutet CR⁸;
R⁶ und R⁷ bedeuten Wasserstoff;
R⁸ bedeutet Methyl, Ethyl, Chlor, Trifluormethyl oder Methyl-(O)ₙS;
n bedeutet 0, 1 oder 2,
mit der Maßgabe, dass R⁵ nicht Methyl oder Ethyl, bedeutet, wenn A eine direkte Bindung ist.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise analog den in WO 2013/083774 A1 angegebenen Methoden hergestellt werden.

Die den erfindungsgemäßen Verbindungen zugrunde liegenden Hydrazine können nach in der Literatur bekannten Methoden synthetisiert werden. Eine Übersicht findet sich beispielsweise in Houben-Weyl, Methoden der Organischen Chemie, Georg Thieme Verlag Stuttgart, Bd. E 16a, Teil 1, Erweiterungs- und Folgebände zur vierten Auflage 1990, S. 648 ff. sowie S. 678 ff.

Thioether der Formel (I) mit n = 0 können zu den entsprechenden Sulfoxiden oder Sulfonen oxidiert werden. Oxidationsmethoden, die gezielt zum Sulfoxid oder Sulfon führen, sind in der Literatur bekannt. Es bietet sich eine Anzahl an Oxidationssystemen an, beispielsweise Persäuren wie meta-Chlorperbenzoesäure, die gegebenenfalls in situ erzeugt werden (zum Beispiel Peressigsäure im System Essigsäure/Wasserstoffperoxid/Natriumwolframat(VI)) (Houben-Weyl, Methoden der Organischen Chemie, Georg Thieme Verlag Stuttgart, Bd. E 11, Erweiterungs- und Folgebände zur vierten Auflage 1985, S. 702 ff., S. 718 ff. sowie S. 1194 ff.). Unter anderem hängt es vom Substitutionsmuster und vom Oxidationsmittel ab, an welcher Stelle der Synthesekaskade die Oxidation des Thioethers zweckmäßig ist.

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.
Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria und Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola und Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen, abhängig von ihrer jeweiligen chemischen Struktur und der ausgebrachten Aufwandmenge, hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Maniok, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl-oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die in nachfolgender Tabelle aufgeführten Beispiele sind ganz besonders bevorzugt.

Die verwendeten Abkürzungen bedeuten:
Me = Methyl c-Pr = cyclo-Propyl

**Tabelle 1: Erfindungsgemäße Verbindungen der allgemeinen Formel (I), worin R² und R³ jeweils Wasserstoff bedeuten, und A für eine direkte Bindung, X¹ für CH, X² für CR⁷ und X³ für CR⁸ stehen**

| | | | | | | |
|---|---|---|---|---|---|---|
| 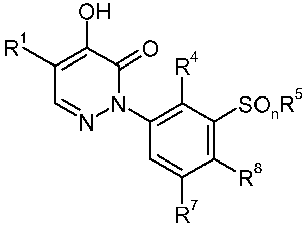 | | | | | | |

| **Nr.** | **R¹** | **R⁴** | **n** | **R⁵** | **R⁷** | **R⁸** |
|---|---|---|---|---|---|---|
| 1-1 | Me | Me | 0 | c-Pr | H | H |
| 1-2 | Me | Me | 1 | c-Pr | H | H |
| 1-3 | Me | Me | 2 | c-Pr | H | H |
| 1-4 | Me | Cl | 0 | c-Pr | H | H |
| 1-5 | Me | Cl | 1 | c-Pr | H | H |
| 1-6 | Me | Cl | 2 | c-Pr | H | H |
| 1-7 | Me | Me | 0 | c-Pr | H | Me |
| 1-8 | Me | Me | 1 | c-Pr | H | Me |
| 1-9 | Me | Me | 2 | c-Pr | H | Me |
| 1-10 | Me | Cl | 0 | c-Pr | H | Me |
| 1-11 | Me | Cl | 1 | c-Pr | H | Me |
| 1-12 | Me | Cl | 2 | c-Pr | H | Me |
| 1-13 | Me | Me | 0 | c-Pr | H | Cl |
| 1-14 | Me | Me | 1 | c-Pr | H | Cl |
| 1-15 | Me | Me | 2 | c-Pr | H | Cl |
| 1-16 | Me | Cl | 0 | c-Pr | H | Cl |
| 1-17 | Me | Cl | 1 | c-Pr | H | Cl |
| 1-18 | Me | Cl | 2 | c-Pr | H | Cl |
| 1-19 | Me | Me | 0 | c-Pr | H | CF₃ |
| 1-20 | Me | Me | 1 | c-Pr | H | CF₃ |
| 1-21 | Me | Me | 2 | c-Pr | H | CF₃ |
| 1-22 | Me | Cl | 0 | c-Pr | H | CF₃ |
| 1-23 | Me | Cl | 1 | c-Pr | H | CF₃ |
| 1-24 | Me | Cl | 2 | c-Pr | H | CF₃ |
| 1-25 | Me | Me | 0 | c-Pr | H | SO₂Me |
| 1-26 | Me | Me | 1 | c-Pr | H | SO₂Me |
| 1-27 | Me | Me | 2 | c-Pr | H | SO₂Me |
| 1-28 | Me | Cl | 0 | c-Pr | H | SO₂Me |
| 1-29 | Me | Cl | 1 | c-Pr | H | SO₂Me |
| 1-30 | Me | Cl | 2 | c-Pr | H | SO₂Me |
| 1-31 | Me | Me | 0 | c-Pr | Me | H |
| 1-32 | Me | Me | 1 | c-Pr | Me | H |
| 1-33 | Me | Me | 2 | c-Pr | Me | H |
| 1-34 | Me | Cl | 0 | c-Pr | Me | H |
| 1-35 | Me | Cl | 1 | c-Pr | Me | H |
| 1-36 | Me | Cl | 2 | c-Pr | Me | H |
| 1-37 | Me | Me | 0 | c-Pr | Me | Me |
| 1-38 | Me | Me | 1 | c-Pr | Me | Me |
| 1-39 | Me | Me | 2 | c-Pr | Me | Me |
| 1-40 | Me | Cl | 0 | c-Pr | Me | Me |
| 1-41 | Me | Cl | 1 | c-Pr | Me | Me |
| 1-42 | Me | Cl | 2 | c-Pr | Me | Me |
| 1-43 | Me | Me | 0 | c-Pr | Me | Cl |
| 1-44 | Me | Me | 1 | c-Pr | Me | Cl |
| 1-45 | Me | Me | 2 | c-Pr | Me | Cl |
| 1-46 | Me | Cl | 0 | c-Pr | Me | Cl |
| 1-47 | Me | Cl | 1 | c-Pr | Me | Cl |
| 1-48 | Me | Cl | 2 | c-Pr | Me | Cl |
| 1-49 | Me | Me | 0 | c-Pr | Me | CF₃ |
| 1-50 | Me | Me | 1 | c-Pr | Me | CF₃ |
| 1-51 | Me | Me | 2 | c-Pr | Me | CF₃ |
| 1-52 | Me | Cl | 0 | c-Pr | Me | CF₃ |
| 1-53 | Me | Cl | 1 | c-Pr | Me | CF₃ |
| 1-54 | Me | Cl | 2 | c-Pr | Me | CF₃ |
| 1-55 | Me | Me | 0 | c-Pr | Me | SO₂Me |
| 1-56 | Me | Me | 1 | c-Pr | Me | SO₂Me |
| 1-57 | Me | Me | 2 | c-Pr | Me | SO₂Me |
| 1-58 | Me | Cl | 0 | c-Pr | Me | SO₂Me |
| 1-59 | Me | Cl | 1 | c-Pr | Me | SO₂Me |
| 1-60 | Me | Cl | 2 | c-Pr | Me | SO₂Me |
| 1-61 | c-Pr | Me | 0 | c-Pr | H | H |
| 1-62 | c-Pr | Me | 1 | c-Pr | H | H |
| 1-63 | c-Pr | Me | 2 | c-Pr | H | H |
| 1-64 | c-Pr | Cl | 0 | c-Pr | H | H |
| 1-65 | c-Pr | Cl | 1 | c-Pr | H | H |
| 1-66 | c-Pr | Cl | 2 | c-Pr | H | H |
| 1-67 | c-Pr | Me | 0 | c-Pr | H | Me |
| 1-68 | c-Pr | Me | 1 | c-Pr | H | Me |
| 1-69 | c-Pr | Me | 2 | c-Pr | H | Me |
| 1-70 | c-Pr | Cl | 0 | c-Pr | H | Me |
| 1-71 | c-Pr | Cl | 1 | c-Pr | H | Me |
| 1-72 | c-Pr | Cl | 2 | c-Pr | H | Me |
| 1-73 | c-Pr | Me | 0 | c-Pr | H | Cl |
| 1-74 | c-Pr | Me | 1 | c-Pr | H | Cl |
| 1-75 | c-Pr | Me | 2 | c-Pr | H | Cl |
| 1-76 | c-Pr | Cl | 0 | c-Pr | H | Cl |
| 1-77 | c-Pr | Cl | 1 | c-Pr | H | Cl |
| 1-78 | c-Pr | Cl | 2 | c-Pr | H | Cl |
| 1-79 | c-Pr | Me | 0 | c-Pr | H | CF₃ |
| 1-80 | c-Pr | Me | 1 | c-Pr | H | CF₃ |
| 1-81 | c-Pr | Me | 2 | c-Pr | H | CF₃ |
| 1-82 | c-Pr | Cl | 0 | c-Pr | H | CF₃ |
| 1-83 | c-Pr | Cl | 1 | c-Pr | H | CF₃ |
| 1-84 | c-Pr | Cl | 2 | c-Pr | H | CF₃ |
| 1-85 | c-Pr | Me | 0 | c-Pr | H | SO₂Me |
| 1-86 | c-Pr | Me | 1 | c-Pr | H | SO₂Me |
| 1-87 | c-Pr | Me | 2 | c-Pr | H | SO₂Me |
| 1-88 | c-Pr | Cl | 0 | c-Pr | H | SO₂Me |
| 1-89 | c-Pr | Cl | 1 | c-Pr | H | SO₂Me |
| 1-90 | c-Pr | Cl | 2 | c-Pr | H | SO₂Me |
| 1-91 | c-Pr | Me | 0 | c-Pr | Me | H |
| 1-92 | c-Pr | Me | 1 | c-Pr | Me | H |
| 1-93 | c-Pr | Me | 2 | c-Pr | Me | H |
| 1-94 | c-Pr | Cl | 0 | c-Pr | Me | H |
| 1-95 | c-Pr | Cl | 1 | c-Pr | Me | H |
| 1-96 | c-Pr | Cl | 2 | c-Pr | Me | H |
| 1-97 | c-Pr | Me | 0 | c-Pr | Me | Me |
| 1-98 | c-Pr | Me | 1 | c-Pr | Me | Me |
| 1-99 | c-Pr | Me | 2 | c-Pr | Me | Me |
| 1-100 | c-Pr | Cl | 0 | c-Pr | Me | Me |
| 1-101 | c-Pr | Cl | 1 | c-Pr | Me | Me |
| 1-102 | c-Pr | Cl | 2 | c-Pr | Me | Me |
| 1-103 | c-Pr | Me | 0 | c-Pr | Me | Cl |
| 1-104 | c-Pr | Me | 1 | c-Pr | Me | Cl |
| 1-105 | c-Pr | Me | 2 | c-Pr | Me | Cl |
| 1-106 | c-Pr | Cl | 0 | c-Pr | Me | Cl |
| 1-107 | c-Pr | Cl | 1 | c-Pr | Me | Cl |
| 1-108 | c-Pr | Cl | 2 | c-Pr | Me | Cl |
| 1-109 | c-Pr | Me | 0 | c-Pr | Me | CF₃ |
| 1-110 | c-Pr | Me | 1 | c-Pr | Me | CF₃ |
| 1-111 | c-Pr | Me | 2 | c-Pr | Me | CF₃ |
| 1-112 | c-Pr | Cl | 0 | c-Pr | Me | CF₃ |
| 1-113 | c-Pr | Cl | 1 | c-Pr | Me | CF₃ |
| 1-114 | c-Pr | Cl | 2 | c-Pr | Me | CF₃ |
| 1-115 | c-Pr | Me | 0 | c-Pr | Me | SO₂Me |
| 1-116 | c-Pr | Me | 1 | c-Pr | Me | SO₂Me |
| 1-117 | c-Pr | Me | 2 | c-Pr | Me | SO₂Me |
| 1-118 | c-Pr | Cl | 0 | c-Pr | Me | SO₂Me |
| 1-119 | c-Pr | Cl | 1 | c-Pr | Me | SO₂Me |
| 1-120 | c-Pr | Cl | 2 | c-Pr | Me | SO₂Me |
| 1-121 | Propen-2-yl | Me | 0 | c-Pr | H | H |
| 1-122 | Propen-2-yl | Me | 1 | c-Pr | H | H |
| 1-123 | Propen-2-yl | Me | 2 | c-Pr | H | H |
| 1-124 | Propen-2-yl | Cl | 0 | c-Pr | H | H |
| 1-125 | Propen-2-yl | Cl | 1 | c-Pr | H | H |
| 1-126 | Propen-2-yl | Cl | 2 | c-Pr | H | H |
| 1-127 | Propen-2-yl | Me | 0 | c-Pr | H | Me |
| 1-128 | Propen-2-yl | Me | 1 | c-Pr | H | Me |
| 1-129 | Propen-2-yl | Me | 2 | c-Pr | H | Me |
| 1-130 | Propen-2-yl | Cl | 0 | c-Pr | H | Me |
| 1-131 | Propen-2-yl | Cl | 1 | c-Pr | H | Me |
| 1-132 | Propen-2-yl | Cl | 2 | c-Pr | H | Me |
| 1-133 | Propen-2-yl | Me | 0 | c-Pr | H | Cl |
| 1-134 | Propen-2-yl | Me | 1 | c-Pr | H | Cl |
| 1-135 | Propen-2-yl | Me | 2 | c-Pr | H | Cl |
| 1-136 | Propen-2-yl | Cl | 0 | c-Pr | H | Cl |
| 1-137 | Propen-2-yl | Cl | 1 | c-Pr | H | Cl |
| 1-138 | Propen-2-yl | Cl | 2 | c-Pr | H | Cl |
| 1-139 | Propen-2-yl | Me | 0 | c-Pr | H | CF₃ |
| 1-140 | Propen-2-yl | Me | 1 | c-Pr | H | CF₃ |
| 1-141 | Propen-2-yl | Me | 2 | c-Pr | H | CF₃ |
| 1-142 | Propen-2-yl | Cl | 0 | c-Pr | H | CF₃ |
| 1-143 | Propen-2-yl | Cl | 1 | c-Pr | H | CF₃ |
| 1-144 | Propen-2-yl | Cl | 2 | c-Pr | H | CF₃ |
| 1-145 | Propen-2-yl | Me | 0 | c-Pr | H | SO₂Me |
| 1-146 | Propen-2-yl | Me | 1 | c-Pr | H | SO₂Me |
| 1-147 | Propen-2-yl | Me | 2 | c-Pr | H | SO₂Me |
| 1-148 | Propen-2-yl | Cl | 0 | c-Pr | H | SO₂Me |
| 1-149 | Propen-2-yl | Cl | 1 | c-Pr | H | SO₂Me |
| 1-150 | Propen-2-yl | Cl | 2 | c-Pr | H | SO₂Me |
| 1-151 | Propen-2-yl | Me | 0 | c-Pr | Me | H |
| 1-152 | Propen-2-yl | Me | 1 | c-Pr | Me | H |
| 1-153 | Propen-2-yl | Me | 2 | c-Pr | Me | H |
| 1-154 | Propen-2-yl | Cl | 0 | c-Pr | Me | H |
| 1-155 | Propen-2-yl | Cl | 1 | c-Pr | Me | H |
| 1-156 | Propen-2-yl | Cl | 2 | c-Pr | Me | H |
| 1-157 | Propen-2-yl | Me | 0 | c-Pr | Me | Me |
| 1-158 | Propen-2-yl | Me | 1 | c-Pr | Me | Me |
| 1-159 | Propen-2-yl | Me | 2 | c-Pr | Me | Me |
| 1-160 | Propen-2-yl | Cl | 0 | c-Pr | Me | Me |
| 1-161 | Propen-2-yl | Cl | 1 | c-Pr | Me | Me |
| 1-162 | Propen-2-yl | Cl | 2 | c-Pr | Me | Me |
| 1-163 | Propen-2-yl | Me | 0 | c-Pr | Me | Cl |
| 1-164 | Propen-2-yl | Me | 1 | c-Pr | Me | Cl |
| 1-165 | Propen-2-yl | Me | 2 | c-Pr | Me | Cl |
| 1-166 | Propen-2-yl | Cl | 0 | c-Pr | Me | Cl |
| 1-167 | Propen-2-yl | Cl | 1 | c-Pr | Me | Cl |
| 1-168 | Propen-2-yl | Cl | 2 | c-Pr | Me | Cl |
| 1-169 | Propen-2-yl | Me | 0 | c-Pr | Me | CF₃ |
| 1-170 | Propen-2-yl | Me | 1 | c-Pr | Me | CF₃ |
| 1-171 | Propen-2-yl | Me | 2 | c-Pr | Me | CF₃ |
| 1-172 | Propen-2-yl | Cl | 0 | c-Pr | Me | CF₃ |
| 1-173 | Propen-2-yl | Cl | 1 | c-Pr | Me | CF₃ |
| 1-174 | Propen-2-yl | Cl | 2 | c-Pr | Me | CF₃ |
| 1-175 | Propen-2-yl | Me | 0 | c-Pr | Me | SO₂Me |
| 1-176 | Propen-2-yl | Me | 1 | c-Pr | Me | SO₂Me |
| 1-177 | Propen-2-yl | Me | 2 | c-Pr | Me | SO₂Me |
| 1-178 | Propen-2-yl | Cl | 0 | c-Pr | Me | SO₂Me |
| 1-179 | Propen-2-yl | Cl | 1 | c-Pr | Me | SO₂Me |
| 1-180 | Propen-2-yl | Cl | 2 | c-Pr | Me | SO₂Me |
| 1-181 | Cl | Me | 0 | c-Pr | H | H |
| 1-182 | Cl | Me | 1 | c-Pr | H | H |
| 1-183 | Cl | Me | 2 | c-Pr | H | H |
| 1-184 | Cl | Cl | 0 | c-Pr | H | H |
| 1-185 | Cl | Cl | 1 | c-Pr | H | H |
| 1-186 | Cl | Cl | 2 | c-Pr | H | H |
| 1-187 | Cl | Me | 0 | c-Pr | H | Me |
| 1-188 | Cl | Me | 1 | c-Pr | H | Me |
| 1-189 | Cl | Me | 2 | c-Pr | H | Me |
| 1-190 | Cl | Cl | 0 | c-Pr | H | Me |
| 1-191 | Cl | Cl | 1 | c-Pr | H | Me |
| 1-192 | Cl | Cl | 2 | c-Pr | H | Me |
| 1-193 | Cl | Me | 0 | c-Pr | H | Cl |
| 1-194 | Cl | Me | 1 | c-Pr | H | Cl |
| 1-195 | Cl | Me | 2 | c-Pr | H | Cl |
| 1-196 | Cl | Cl | 0 | c-Pr | H | Cl |
| 1-197 | Cl | Cl | 1 | c-Pr | H | Cl |
| 1-198 | Cl | Cl | 2 | c-Pr | H | Cl |
| 1-199 | Cl | Me | 0 | c-Pr | H | CF₃ |
| 1-200 | Cl | Me | 1 | c-Pr | H | CF₃ |
| 1-201 | Cl | Me | 2 | c-Pr | H | CF₃ |
| 1-202 | Cl | Cl | 0 | c-Pr | H | CF₃ |
| 1-203 | Cl | Cl | 1 | c-Pr | H | CF₃ |
| 1-204 | Cl | Cl | 2 | c-Pr | H | CF₃ |
| 1-205 | Cl | Me | 0 | c-Pr | H | SO₂Me |
| 1-206 | Cl | Me | 1 | c-Pr | H | SO₂Me |
| 1-207 | Cl | Me | 2 | c-Pr | H | SO₂Me |
| 1-208 | Cl | Cl | 0 | c-Pr | H | SO₂Me |
| 1-209 | Cl | Cl | 1 | c-Pr | H | SO₂Me |
| 1-210 | Cl | Cl | 2 | c-Pr | H | SO₂Me |
| 1-211 | Cl | Me | 0 | c-Pr | Me | H |
| 1-212 | Cl | Me | 1 | c-Pr | Me | H |
| 1-213 | Cl | Me | 2 | c-Pr | Me | H |
| 1-214 | Cl | Cl | 0 | c-Pr | Me | H |
| 1-215 | Cl | Cl | 1 | c-Pr | Me | H |
| 1-216 | Cl | Cl | 2 | c-Pr | Me | H |
| 1-217 | Cl | Me | 0 | c-Pr | Me | Me |
| 1-218 | Cl | Me | 1 | c-Pr | Me | Me |
| 1-219 | Cl | Me | 2 | c-Pr | Me | Me |
| 1-220 | Cl | Cl | 0 | c-Pr | Me | Me |
| 1-221 | Cl | Cl | 1 | c-Pr | Me | Me |
| 1-222 | Cl | Cl | 2 | c-Pr | Me | Me |
| 1-223 | Cl | Me | 0 | c-Pr | Me | Cl |
| 1-224 | Cl | Me | 1 | c-Pr | Me | Cl |
| 1-225 | Cl | Me | 2 | c-Pr | Me | Cl |
| 1-226 | Cl | Cl | 0 | c-Pr | Me | Cl |
| 1-227 | Cl | Cl | 1 | c-Pr | Me | Cl |
| 1-228 | Cl | Cl | 2 | c-Pr | Me | Cl |
| 1-229 | Cl | Me | 0 | c-Pr | Me | CF₃ |
| 1-230 | Cl | Me | 1 | c-Pr | Me | CF₃ |
| 1-231 | Cl | Me | 2 | c-Pr | Me | CF₃ |
| 1-232 | Cl | Cl | 0 | c-Pr | Me | CF₃ |
| 1-233 | Cl | Cl | 1 | c-Pr | Me | CF₃ |
| 1-234 | Cl | Cl | 2 | c-Pr | Me | CF₃ |
| 1-235 | Cl | Me | 0 | c-Pr | Me | SO₂Me |
| 1-236 | Cl | Me | 1 | c-Pr | Me | SO₂Me |
| 1-237 | Cl | Me | 2 | c-Pr | Me | SO₂Me |
| 1-238 | Cl | Cl | 0 | c-Pr | Me | SO₂Me |
| 1-239 | Cl | Cl | 1 | c-Pr | Me | SO₂Me |
| 1-240 | Cl | Cl | 2 | c-Pr | Me | SO₂Me |
| 1-241 | SO₂Me | Me | 0 | c-Pr | H | H |
| 1-242 | SO₂Me | Me | 1 | c-Pr | H | H |
| 1-243 | SO₂Me | Me | 2 | c-Pr | H | H |
| 1-244 | SO₂Me | Cl | 0 | c-Pr | H | H |
| 1-245 | SO₂Me | Cl | 1 | c-Pr | H | H |
| 1-246 | SO₂Me | Cl | 2 | c-Pr | H | H |
| 1-247 | SO₂Me | Me | 0 | c-Pr | H | Me |
| 1-248 | SO₂Me | Me | 1 | c-Pr | H | Me |
| 1-249 | SO₂Me | Me | 2 | c-Pr | H | Me |
| 1-250 | SO₂Me | Cl | 0 | c-Pr | H | Me |
| 1-251 | SO₂Me | Cl | 1 | c-Pr | H | Me |
| 1-252 | SO₂Me | Cl | 2 | c-Pr | H | Me |
| 1-253 | SO₂Me | Me | 0 | c-Pr | H | Cl |
| 1-254 | SO₂Me | Me | 1 | c-Pr | H | Cl |
| 1-255 | SO₂Me | Me | 2 | c-Pr | H | Cl |
| 1-256 | SO₂Me | Cl | 0 | c-Pr | H | Cl |
| 1-257 | SO₂Me | Cl | 1 | c-Pr | H | Cl |
| 1-258 | SO₂Me | Cl | 2 | c-Pr | H | Cl |
| 1-259 | SO₂Me | Me | 0 | c-Pr | H | CF₃ |
| 1-260 | SO₂Me | Me | 1 | c-Pr | H | CF₃ |
| 1-261 | SO₂Me | Me | 2 | c-Pr | H | CF₃ |
| 1-262 | SO₂Me | Cl | 0 | c-Pr | H | CF₃ |
| 1-263 | SO₂Me | Cl | 1 | c-Pr | H | CF₃ |
| 1-264 | SO₂Me | Cl | 2 | c-Pr | H | CF₃ |
| 1-265 | SO₂Me | Me | 0 | c-Pr | H | SO₂Me |
| 1-266 | SO₂Me | Me | 1 | c-Pr | H | SO₂Me |
| 1-267 | SO₂Me | Me | 2 | c-Pr | H | SO₂Me |
| 1-268 | SO₂Me | Cl | 0 | c-Pr | H | SO₂Me |
| 1-269 | SO₂Me | Cl | 1 | c-Pr | H | SO₂Me |
| 1-270 | SO₂Me | Cl | 2 | c-Pr | H | SO₂Me |
| 1-271 | SO₂Me | Me | 0 | c-Pr | Me | H |
| 1-272 | SO₂Me | Me | 1 | c-Pr | Me | H |
| 1-273 | SO₂Me | Me | 2 | c-Pr | Me | H |
| 1-274 | SO₂Me | Cl | 0 | c-Pr | Me | H |
| 1-275 | SO₂Me | Cl | 1 | c-Pr | Me | H |
| 1-276 | SO₂Me | Cl | 2 | c-Pr | Me | H |
| 1-277 | SO₂Me | Me | 0 | c-Pr | Me | Me |
| 1-278 | SO₂Me | Me | 1 | c-Pr | Me | Me |
| 1-279 | SO₂Me | Me | 2 | c-Pr | Me | Me |
| 1-280 | SO₂Me | Cl | 0 | c-Pr | Me | Me |
| 1-281 | SO₂Me | Cl | 1 | c-Pr | Me | Me |
| 1-282 | SO₂Me | Cl | 2 | c-Pr | Me | Me |
| 1-283 | SO₂Me | Me | 0 | c-Pr | Me | Cl |
| 1-284 | SO₂Me | Me | 1 | c-Pr | Me | Cl |
| 1-285 | SO₂Me | Me | 2 | c-Pr | Me | Cl |
| 1-286 | SO₂Me | Cl | 0 | c-Pr | Me | Cl |
| 1-287 | SO₂Me | Cl | 1 | c-Pr | Me | Cl |
| 1-288 | SO₂Me | Cl | 2 | c-Pr | Me | Cl |
| 1-289 | SO₂Me | Me | 0 | c-Pr | Me | CF₃ |
| 1-290 | SO₂Me | Me | 1 | c-Pr | Me | CF₃ |
| 1-291 | SO₂Me | Me | 2 | c-Pr | Me | CF₃ |
| 1-292 | SO₂Me | Cl | 0 | c-Pr | Me | CF₃ |
| 1-293 | SO₂Me | Cl | 1 | c-Pr | Me | CF₃ |
| 1-294 | SO₂Me | Cl | 2 | c-Pr | Me | CF₃ |
| 1-295 | SO₂Me | Me | 0 | c-Pr | Me | SO₂Me |
| 1-296 | SO₂Me | Me | 1 | c-Pr | Me | SO₂Me |
| 1-297 | SO₂Me | Me | 2 | c-Pr | Me | SO₂Me |
| 1-298 | SO₂Me | Cl | 0 | c-Pr | Me | SO₂Me |
| 1-299 | SO₂Me | Cl | 1 | c-Pr | Me | SO₂Me |
| 1-300 | SO₂Me | Cl | 2 | c-Pr | Me | SO₂Me |
| 1-301 | Me | Me | 0 | CH₂-c-Pr | H | H |
| 1-302 | Me | Me | 1 | CH₂-c-Pr | H | H |
| 1-303 | Me | Me | 2 | CH₂-c-Pr | H | H |
| 1-304 | Me | Cl | 0 | CH₂-c-Pr | H | H |
| 1-305 | Me | Cl | 1 | CH₂-c-Pr | H | H |
| 1-306 | Me | Cl | 2 | CH₂-c-Pr | H | H |
| 1-307 | Me | Me | 0 | CH₂-c-Pr | H | Me |
| 1-308 | Me | Me | 1 | CH₂-c-Pr | H | Me |
| 1-309 | Me | Me | 2 | CH₂-c-Pr | H | Me |
| 1-310 | Me | Cl | 0 | CH₂-c-Pr | H | Me |
| 1-311 | Me | Cl | 1 | CH₂-c-Pr | H | Me |
| 1-312 | Me | Cl | 2 | CH₂-c-Pr | H | Me |
| 1-313 | Me | Me | 0 | CH₂-c-Pr | H | Cl |
| 1-314 | Me | Me | 1 | CH₂-c-Pr | H | Cl |
| 1-315 | Me | Me | 2 | CH₂-c-Pr | H | Cl |
| 1-316 | Me | Cl | 0 | CH₂-c-Pr | H | Cl |
| 1-317 | Me | Cl | 1 | CH₂-c-Pr | H | Cl |
| 1-318 | Me | Cl | 2 | CH₂-c-Pr | H | Cl |
| 1-319 | Me | Me | 0 | CH₂-c-Pr | H | CF₃ |
| 1-320 | Me | Me | 1 | CH₂-c-Pr | H | CF₃ |
| 1-321 | Me | Me | 2 | CH₂-c-Pr | H | CF₃ |
| 1-322 | Me | Cl | 0 | CH₂-c-Pr | H | CF₃ |
| 1-323 | Me | Cl | 1 | CH₂-c-Pr | H | CF₃ |
| 1-324 | Me | Cl | 2 | CH₂-c-Pr | H | CF₃ |
| 1-325 | Me | Me | 0 | CH₂-c-Pr | H | SO₂Me |
| 1-326 | Me | Me | 1 | CH₂-c-Pr | H | SO₂Me |
| 1-327 | Me | Me | 2 | CH₂-c-Pr | H | SO₂Me |
| 1-328 | Me | Cl | 0 | CH₂-c-Pr | H | SO₂Me |
| 1-329 | Me | Cl | 1 | CH₂-c-Pr | H | SO₂Me |
| 1-330 | Me | Cl | 2 | CH₂-c-Pr | H | SO₂Me |
| 1-331 | Me | Me | 0 | CH₂-c-Pr | Me | H |
| 1-332 | Me | Me | 1 | CH₂-c-Pr | Me | H |
| 1-333 | Me | Me | 2 | CH₂-c-Pr | Me | H |
| 1-334 | Me | Cl | 0 | CH₂-c-Pr | Me | H |
| 1-335 | Me | Cl | 1 | CH₂-c-Pr | Me | H |
| 1-336 | Me | Cl | 2 | CH₂-c-Pr | Me | H |
| 1-337 | Me | Me | 0 | CH₂-c-Pr | Me | Me |
| 1-338 | Me | Me | 1 | CH₂-c-Pr | Me | Me |
| 1-339 | Me | Me | 2 | CH₂-c-Pr | Me | Me |
| 1-340 | Me | Cl | 0 | CH₂-c-Pr | Me | Me |
| 1-341 | Me | Cl | 1 | CH₂-c-Pr | Me | Me |
| 1-342 | Me | Cl | 2 | CH₂-c-Pr | Me | Me |
| 1-343 | Me | Me | 0 | CH₂-c-Pr | Me | Cl |
| 1-344 | Me | Me | 1 | CH₂-c-Pr | Me | Cl |
| 1-345 | Me | Me | 2 | CH₂-c-Pr | Me | Cl |
| 1-346 | Me | Cl | 0 | CH₂-c-Pr | Me | Cl |
| 1-347 | Me | Cl | 1 | CH₂-c-Pr | Me | Cl |
| 1-348 | Me | Cl | 2 | CH₂-c-Pr | Me | Cl |
| 1-349 | Me | Me | 0 | CH₂-c-Pr | Me | CF₃ |
| 1-350 | Me | Me | 1 | CH₂-c-Pr | Me | CF₃ |
| 1-351 | Me | Me | 2 | CH₂-c-Pr | Me | CF₃ |
| 1-352 | Me | Cl | 0 | CH₂-c-Pr | Me | CF₃ |
| 1-353 | Me | Cl | 1 | CH₂-c-Pr | Me | CF₃ |
| 1-354 | Me | Cl | 2 | CH₂-c-Pr | Me | CF₃ |
| 1-355 | Me | Me | 0 | CH₂-c-Pr | Me | SO₂Me |
| 1-356 | Me | Me | 1 | CH₂-c-Pr | Me | SO₂Me |
| 1-357 | Me | Me | 2 | CH₂-c-Pr | Me | SO₂Me |
| 1-358 | Me | Cl | 0 | CH₂-c-Pr | Me | SO₂Me |
| 1-359 | Me | Cl | 1 | CH₂-c-Pr | Me | SO₂Me |
| 1-360 | Me | Cl | 2 | CH₂-c-Pr | Me | SO₂Me |
| 1-361 | c-Pr | Me | 0 | CH₂-c-Pr | H | H |
| 1-362 | c-Pr | Me | 1 | CH₂-c-Pr | H | H |
| 1-363 | c-Pr | Me | 2 | CH₂-c-Pr | H | H |
| 1-364 | c-Pr | Cl | 0 | CH₂-c-Pr | H | H |
| 1-365 | c-Pr | Cl | 1 | CH₂-c-Pr | H | H |
| 1-366 | c-Pr | Cl | 2 | CH₂-c-Pr | H | H |
| 1-367 | c-Pr | Me | 0 | CH₂-c-Pr | H | Me |
| 1-368 | c-Pr | Me | 1 | CH₂-c-Pr | H | Me |
| 1-369 | c-Pr | Me | 2 | CH₂-c-Pr | H | Me |
| 1-370 | c-Pr | Cl | 0 | CH₂-c-Pr | H | Me |
| 1-371 | c-Pr | Cl | 1 | CH₂-c-Pr | H | Me |
| 1-372 | c-Pr | Cl | 2 | CH₂-c-Pr | H | Me |
| 1-373 | c-Pr | Me | 0 | CH₂-c-Pr | H | Cl |
| 1-374 | c-Pr | Me | 1 | CH₂-c-Pr | H | Cl |
| 1-375 | c-Pr | Me | 2 | CH₂-c-Pr | H | Cl |
| 1-376 | c-Pr | Cl | 0 | CH₂-c-Pr | H | Cl |
| 1-377 | c-Pr | Cl | 1 | CH₂-c-Pr | H | Cl |
| 1-378 | c-Pr | Cl | 2 | CH₂-c-Pr | H | Cl |
| 1-379 | c-Pr | Me | 0 | CH₂-c-Pr | H | CF₃ |
| 1-380 | c-Pr | Me | 1 | CH₂-c-Pr | H | CF₃ |
| 1-381 | c-Pr | Me | 2 | CH₂-c-Pr | H | CF₃ |
| 1-382 | c-Pr | Cl | 0 | CH₂-c-Pr | H | CF₃ |
| 1-383 | c-Pr | Cl | 1 | CH₂-c-Pr | H | CF₃ |
| 1-384 | c-Pr | Cl | 2 | CH₂-c-Pr | H | CF₃ |
| 1-385 | c-Pr | Me | 0 | CH₂-c-Pr | H | SO₂Me |
| 1-386 | c-Pr | Me | 1 | CH₂-c-Pr | H | SO₂Me |
| 1-387 | c-Pr | Me | 2 | CH₂-c-Pr | H | SO₂Me |
| 1-388 | c-Pr | Cl | 0 | CH₂-c-Pr | H | SO₂Me |
| 1-389 | c-Pr | Cl | 1 | CH₂-c-Pr | H | SO₂Me |
| 1-390 | c-Pr | Cl | 2 | CH₂-c-Pr | H | SO₂Me |
| 1-391 | c-Pr | Me | 0 | CH₂-c-Pr | Me | H |
| 1-392 | c-Pr | Me | 1 | CH₂-c-Pr | Me | H |
| 1-393 | c-Pr | Me | 2 | CH₂-c-Pr | Me | H |
| 1-394 | c-Pr | Cl | 0 | CH₂-c-Pr | Me | H |
| 1-395 | c-Pr | Cl | 1 | CH₂-c-Pr | Me | H |
| 1-396 | c-Pr | Cl | 2 | CH₂-c-Pr | Me | H |
| 1-397 | c-Pr | Me | 0 | CH₂-c-Pr | Me | Me |
| 1-398 | c-Pr | Me | 1 | CH₂-c-Pr | Me | Me |
| 1-399 | c-Pr | Me | 2 | CH₂-c-Pr | Me | Me |
| 1-400 | c-Pr | Cl | 0 | CH₂-c-Pr | Me | Me |
| 1-401 | c-Pr | Cl | 1 | CH₂-c-Pr | Me | Me |
| 1-402 | c-Pr | Cl | 2 | CH₂-c-Pr | Me | Me |
| 1-403 | c-Pr | Me | 0 | CH₂-c-Pr | Me | Cl |
| 1-404 | c-Pr | Me | 1 | CH₂-c-Pr | Me | Cl |
| 1-405 | c-Pr | Me | 2 | CH₂-c-Pr | Me | Cl |
| 1-406 | c-Pr | Cl | 0 | CH₂-c-Pr | Me | Cl |
| 1-407 | c-Pr | Cl | 1 | CH₂-c-Pr | Me | Cl |
| 1-408 | c-Pr | Cl | 2 | CH₂-c-Pr | Me | Cl |
| 1-409 | c-Pr | Me | 0 | CH₂-c-Pr | Me | CF₃ |
| 1-410 | c-Pr | Me | 1 | CH₂-c-Pr | Me | CF₃ |
| 1-411 | c-Pr | Me | 2 | CH₂-c-Pr | Me | CF₃ |
| 1-412 | c-Pr | Cl | 0 | CH₂-c-Pr | Me | CF₃ |
| 1-413 | c-Pr | Cl | 1 | CH₂-c-Pr | Me | CF₃ |
| 1-414 | c-Pr | Cl | 2 | CH₂-c-Pr | Me | CF₃ |
| 1-415 | c-Pr | Me | 0 | CH₂-c-Pr | Me | SO₂Me |
| 1-416 | c-Pr | Me | 1 | CH₂-c-Pr | Me | SO₂Me |
| 1-417 | c-Pr | Me | 2 | CH₂-c-Pr | Me | SO₂Me |
| 1-418 | c-Pr | Cl | 0 | CH₂-c-Pr | Me | SO₂Me |
| 1-419 | c-Pr | Cl | 1 | CH₂-c-Pr | Me | SO₂Me |
| 1-420 | c-Pr | Cl | 2 | CH₂-c-Pr | Me | SO₂Me |
| 1-421 | Propen-2-yl | Me | 0 | CH₂-c-Pr | H | H |
| 1-422 | Propen-2-yl | Me | 1 | CH₂-c-Pr | H | H |
| 1-423 | Propen-2-yl | Me | 2 | CH₂-c-Pr | H | H |
| 1-424 | Propen-2-yl | Cl | 0 | CH₂-c-Pr | H | H |
| 1-425 | Propen-2-yl | Cl | 1 | CH₂-c-Pr | H | H |
| 1-426 | Propen-2-yl | Cl | 2 | CH₂-c-Pr | H | H |
| 1-427 | Propen-2-yl | Me | 0 | CH₂-c-Pr | H | Me |
| 1-428 | Propen-2-yl | Me | 1 | CH₂-c-Pr | H | Me |
| 1-429 | Propen-2-yl | Me | 2 | CH₂-c-Pr | H | Me |
| 1-430 | Propen-2-yl | Cl | 0 | CH₂-c-Pr | H | Me |
| 1-431 | Propen-2-yl | Cl | 1 | CH₂-c-Pr | H | Me |
| 1-432 | Propen-2-yl | Cl | 2 | CH₂-c-Pr | H | Me |
| 1-433 | Propen-2-yl | Me | 0 | CH₂-c-Pr | H | Cl |
| 1-434 | Propen-2-yl | Me | 1 | CH₂-c-Pr | H | Cl |
| 1-435 | Propen-2-yl | Me | 2 | CH₂-c-Pr | H | Cl |
| 1-436 | Propen-2-yl | Cl | 0 | CH₂-c-Pr | H | Cl |
| 1-437 | Propen-2-yl | Cl | 1 | CH₂-c-Pr | H | Cl |
| 1-438 | Propen-2-yl | Cl | 2 | CH₂-c-Pr | H | Cl |
| 1-439 | Propen-2-yl | Me | 0 | CH₂-c-Pr | H | CF₃ |
| 1-440 | Propen-2-yl | Me | 1 | CH₂-c-Pr | H | CF₃ |
| 1-441 | Propen-2-yl | Me | 2 | CH₂-c-Pr | H | CF₃ |
| 1-442 | Propen-2-yl | Cl | 0 | CH₂-c-Pr | H | CF₃ |
| 1-443 | Propen-2-yl | Cl | 1 | CH₂-c-Pr | H | CF₃ |
| 1-444 | Propen-2-yl | Cl | 2 | CH₂-c-Pr | H | CF₃ |
| 1-445 | Propen-2-yl | Me | 0 | CH₂-c-Pr | H | SO₂Me |
| 1-446 | Propen-2-yl | Me | 1 | CH₂-c-Pr | H | SO₂Me |
| 1-447 | Propen-2-yl | Me | 2 | CH₂-c-Pr | H | SO₂Me |
| 1-448 | Propen-2-yl | Cl | 0 | CH₂-c-Pr | H | SO₂Me |
| 1-449 | Propen-2-yl | Cl | 1 | CH₂-c-Pr | H | SO₂Me |
| 1-450 | Propen-2-yl | Cl | 2 | CH₂-c-Pr | H | SO₂Me |
| 1-451 | Propen-2-yl | Me | 0 | CH₂-c-Pr | Me | H |
| 1-452 | Propen-2-yl | Me | 1 | CH₂-c-Pr | Me | H |
| 1-453 | Propen-2-yl | Me | 2 | CH₂-c-Pr | Me | H |
| 1-454 | Propen-2-yl | Cl | 0 | CH₂-c-Pr | Me | H |
| 1-455 | Propen-2-yl | Cl | 1 | CH₂-c-Pr | Me | H |
| 1-456 | Propen-2-yl | Cl | 2 | CH₂-c-Pr | Me | H |
| 1-457 | Propen-2-yl | Me | 0 | CH₂-c-Pr | Me | Me |
| 1-458 | Propen-2-yl | Me | 1 | CH₂-c-Pr | Me | Me |
| 1-459 | Propen-2-yl | Me | 2 | CH₂-c-Pr | Me | Me |
| 1-460 | Propen-2-yl | Cl | 0 | CH₂-c-Pr | Me | Me |
| 1-461 | Propen-2-yl | Cl | 1 | CH₂-c-Pr | Me | Me |
| 1-462 | Propen-2-yl | Cl | 2 | CH₂-c-Pr | Me | Me |
| 1-463 | Propen-2-yl | Me | 0 | CH₂-c-Pr | Me | Cl |
| 1-464 | Propen-2-yl | Me | 1 | CH₂-c-Pr | Me | Cl |
| 1-465 | Propen-2-yl | Me | 2 | CH₂-c-Pr | Me | Cl |
| 1-466 | Propen-2-yl | Cl | 0 | CH₂-c-Pr | Me | Cl |
| 1-467 | Propen-2-yl | Cl | 1 | CH₂-c-Pr | Me | Cl |
| 1-468 | Propen-2-yl | Cl | 2 | CH₂-c-Pr | Me | Cl |
| 1-469 | Propen-2-yl | Me | 0 | CH₂-c-Pr | Me | CF₃ |
| 1-470 | Propen-2-yl | Me | 1 | CH₂-c-Pr | Me | CF₃ |
| 1-471 | Propen-2-yl | Me | 2 | CH₂-c-Pr | Me | CF₃ |
| 1-472 | Propen-2-yl | Cl | 0 | CH₂-c-Pr | Me | CF₃ |
| 1-473 | Propen-2-yl | Cl | 1 | CH₂-c-Pr | Me | CF₃ |
| 1-474 | Propen-2-yl | Cl | 2 | CH₂-c-Pr | Me | CF₃ |
| 1-475 | Propen-2-yl | Me | 0 | CH₂-c-Pr | Me | SO₂Me |
| 1-476 | Propen-2-yl | Me | 1 | CH₂-c-Pr | Me | SO₂Me |
| 1-477 | Propen-2-yl | Me | 2 | CH₂-c-Pr | Me | SO₂Me |
| 1-478 | Propen-2-yl | Cl | 0 | CH₂-c-Pr | Me | SO₂Me |
| 1-479 | Propen-2-yl | Cl | 1 | CH₂-c-Pr | Me | SO₂Me |
| 1-480 | Propen-2-yl | Cl | 2 | CH₂-c-Pr | Me | SO₂Me |
| 1-481 | Cl | Me | 0 | CH₂-c-Pr | H | H |
| 1-482 | Cl | Me | 1 | CH₂-c-Pr | H | H |
| 1-483 | Cl | Me | 2 | CH₂-c-Pr | H | H |
| 1-484 | Cl | Cl | 0 | CH₂-c-Pr | H | H |
| 1-485 | Cl | Cl | 1 | CH₂-c-Pr | H | H |
| 1-486 | Cl | Cl | 2 | CH₂-c-Pr | H | H |
| 1-487 | Cl | Me | 0 | CH₂-c-Pr | H | Me |
| 1-488 | Cl | Me | 1 | CH₂-c-Pr | H | Me |
| 1-489 | Cl | Me | 2 | CH₂-c-Pr | H | Me |
| 1-490 | Cl | Cl | 0 | CH₂-c-Pr | H | Me |
| 1-491 | Cl | Cl | 1 | CH₂-c-Pr | H | Me |
| 1-492 | Cl | Cl | 2 | CH₂-c-Pr | H | Me |
| 1-493 | Cl | Me | 0 | CH₂-c-Pr | H | Cl |
| 1-494 | Cl | Me | 1 | CH₂-c-Pr | H | Cl |
| 1-495 | Cl | Me | 2 | CH₂-c-Pr | H | Cl |
| 1-496 | Cl | Cl | 0 | CH₂-c-Pr | H | Cl |
| 1-497 | Cl | Cl | 1 | CH₂-c-Pr | H | Cl |
| 1-498 | Cl | Cl | 2 | CH₂-c-Pr | H | Cl |
| 1-499 | Cl | Me | 0 | CH₂-c-Pr | H | CF₃ |
| 1-500 | Cl | Me | 1 | CH₂-c-Pr | H | CF₃ |
| 1-501 | Cl | Me | 2 | CH₂-c-Pr | H | CF₃ |
| 1-502 | Cl | Cl | 0 | CH₂-c-Pr | H | CF₃ |
| 1-503 | Cl | Cl | 1 | CH₂-c-Pr | H | CF₃ |
| 1-504 | Cl | Cl | 2 | CH₂-c-Pr | H | CF₃ |
| 1-505 | Cl | Me | 0 | CH₂-c-Pr | H | SO₂Me |
| 1-506 | Cl | Me | 1 | CH₂-c-Pr | H | SO₂Me |
| 1-507 | Cl | Me | 2 | CH₂-c-Pr | H | SO₂Me |
| 1-508 | Cl | Cl | 0 | CH₂-c-Pr | H | SO₂Me |
| 1-509 | Cl | Cl | 1 | CH₂-c-Pr | H | SO₂Me |
| 1-510 | Cl | Cl | 2 | CH₂-c-Pr | H | SO₂Me |
| 1-511 | Cl | Me | 0 | CH₂-c-Pr | Me | H |
| 1-512 | Cl | Me | 1 | CH₂-c-Pr | Me | H |
| 1-513 | Cl | Me | 2 | CH₂-c-Pr | Me | H |
| 1-514 | Cl | Cl | 0 | CH₂-c-Pr | Me | H |
| 1-515 | Cl | Cl | 1 | CH₂-c-Pr | Me | H |
| 1-516 | Cl | Cl | 2 | CH₂-c-Pr | Me | H |
| 1-517 | Cl | Me | 0 | CH₂-c-Pr | Me | Me |
| 1-518 | Cl | Me | 1 | CH₂-c-Pr | Me | Me |
| 1-519 | Cl | Me | 2 | CH₂-c-Pr | Me | Me |
| 1-520 | Cl | Cl | 0 | CH₂-c-Pr | Me | Me |
| 1-521 | Cl | Cl | 1 | CH₂-c-Pr | Me | Me |
| 1-522 | Cl | Cl | 2 | CH₂-c-Pr | Me | Me |
| 1-523 | Cl | Me | 0 | CH₂-c-Pr | Me | Cl |
| 1-524 | Cl | Me | 1 | CH₂-c-Pr | Me | Cl |
| 1-525 | Cl | Me | 2 | CH₂-c-Pr | Me | Cl |
| 1-526 | Cl | Cl | 0 | CH₂-c-Pr | Me | Cl |
| 1-527 | Cl | Cl | 1 | CH₂-c-Pr | Me | Cl |
| 1-528 | Cl | Cl | 2 | CH₂-c-Pr | Me | Cl |
| 1-529 | Cl | Me | 0 | CH₂-c-Pr | Me | CF₃ |
| 1-530 | Cl | Me | 1 | CH₂-c-Pr | Me | CF₃ |
| 1-531 | Cl | Me | 2 | CH₂-c-Pr | Me | CF₃ |
| 1-532 | Cl | Cl | 0 | CH₂-c-Pr | Me | CF₃ |
| 1-533 | Cl | Cl | 1 | CH₂-c-Pr | Me | CF₃ |
| 1-534 | Cl | Cl | 2 | CH₂-c-Pr | Me | CF₃ |
| 1-535 | Cl | Me | 0 | CH₂-c-Pr | Me | SO₂Me |
| 1-536 | Cl | Me | 1 | CH₂-c-Pr | Me | SO₂Me |
| 1-537 | Cl | Me | 2 | CH₂-c-Pr | Me | SO₂Me |
| 1-538 | Cl | Cl | 0 | CH₂-c-Pr | Me | SO₂Me |
| 1-539 | Cl | Cl | 1 | CH₂-c-Pr | Me | SO₂Me |
| 1-540 | Cl | Cl | 2 | CH₂-c-Pr | Me | SO₂Me |
| 1-541 | SO₂Me | Me | 0 | CH₂-c-Pr | H | H |
| 1-542 | SO₂Me | Me | 1 | CH₂-c-Pr | H | H |
| 1-543 | SO₂Me | Me | 2 | CH₂-c-Pr | H | H |
| 1-544 | SO₂Me | Cl | 0 | CH₂-c-Pr | H | H |
| 1-545 | SO₂Me | Cl | 1 | CH₂-c-Pr | H | H |
| 1-546 | SO₂Me | Cl | 2 | CH₂-c-Pr | H | H |
| 1-547 | SO₂Me | Me | 0 | CH₂-c-Pr | H | Me |
| 1-548 | SO₂Me | Me | 1 | CH₂-c-Pr | H | Me |
| 1-549 | SO₂Me | Me | 2 | CH₂-c-Pr | H | Me |
| 1-550 | SO₂Me | Cl | 0 | CH₂-c-Pr | H | Me |
| 1-551 | SO₂Me | Cl | 1 | CH₂-c-Pr | H | Me |
| 1-552 | SO₂Me | Cl | 2 | CH₂-c-Pr | H | Me |
| 1-553 | SO₂Me | Me | 0 | CH₂-c-Pr | H | Cl |
| 1-554 | SO₂Me | Me | 1 | CH₂-c-Pr | H | Cl |
| 1-555 | SO₂Me | Me | 2 | CH₂-c-Pr | H | Cl |
| 1-556 | SO₂Me | Cl | 0 | CH₂-c-Pr | H | Cl |
| 1-557 | SO₂Me | Cl | 1 | CH₂-c-Pr | H | Cl |
| 1-558 | SO₂Me | Cl | 2 | CH₂-c-Pr | H | Cl |
| 1-559 | SO₂Me | Me | 0 | CH₂-c-Pr | H | CF₃ |
| 1-560 | SO₂Me | Me | 1 | CH₂-c-Pr | H | CF₃ |
| 1-561 | SO₂Me | Me | 2 | CH₂-c-Pr | H | CF₃ |
| 1-562 | SO₂Me | Cl | 0 | CH₂-c-Pr | H | CF₃ |
| 1-563 | SO₂Me | Cl | 1 | CH₂-c-Pr | H | CF₃ |
| 1-564 | SO₂Me | Cl | 2 | CH₂-c-Pr | H | CF₃ |
| 1-565 | SO₂Me | Me | 0 | CH₂-c-Pr | H | SO₂Me |
| 1-566 | SO₂Me | Me | 1 | CH₂-c-Pr | H | SO₂Me |
| 1-567 | SO₂Me | Me | 2 | CH₂-c-Pr | H | SO₂Me |
| 1-568 | SO₂Me | Cl | 0 | CH₂-c-Pr | H | SO₂Me |
| 1-569 | SO₂Me | Cl | 1 | CH₂-c-Pr | H | SO₂Me |
| 1-570 | SO₂Me | Cl | 2 | CH₂-c-Pr | H | SO₂Me |
| 1-571 | SO₂Me | Me | 0 | CH₂-c-Pr | Me | H |
| 1-572 | SO₂Me | Me | 1 | CH₂-c-Pr | Me | H |
| 1-573 | SO₂Me | Me | 2 | CH₂-c-Pr | Me | H |
| 1-574 | SO₂Me | Cl | 0 | CH₂-c-Pr | Me | H |
| 1-575 | SO₂Me | Cl | 1 | CH₂-c-Pr | Me | H |
| 1-576 | SO₂Me | Cl | 2 | CH₂-c-Pr | Me | H |
| 1-577 | SO₂Me | Me | 0 | CH₂-c-Pr | Me | Me |
| 1-578 | SO₂Me | Me | 1 | CH₂-c-Pr | Me | Me |
| 1-579 | SO₂Me | Me | 2 | CH₂-c-Pr | Me | Me |
| 1-580 | SO₂Me | Cl | 0 | CH₂-c-Pr | Me | Me |
| 1-581 | SO₂Me | Cl | 1 | CH₂-c-Pr | Me | Me |
| 1-582 | SO₂Me | Cl | 2 | CH₂-c-Pr | Me | Me |
| 1-583 | SO₂Me | Me | 0 | CH₂-c-Pr | Me | Cl |
| 1-584 | SO₂Me | Me | 1 | CH₂-c-Pr | Me | Cl |
| 1-585 | SO₂Me | Me | 2 | CH₂-c-Pr | Me | Cl |
| 1-586 | SO₂Me | Cl | 0 | CH₂-c-Pr | Me | Cl |
| 1-587 | SO₂Me | Cl | 1 | CH₂-c-Pr | Me | Cl |
| 1-588 | SO₂Me | Cl | 2 | CH₂-c-Pr | Me | Cl |
| 1-589 | SO₂Me | Me | 0 | CH₂-c-Pr | Me | CF₃ |
| 1-590 | SO₂Me | Me | 1 | CH₂-c-Pr | Me | CF₃ |
| 1-591 | SO₂Me | Me | 2 | CH₂-c-Pr | Me | CF₃ |
| 1-592 | SO₂Me | Cl | 0 | CH₂-c-Pr | Me | CF₃ |
| 1-593 | SO₂Me | Cl | 1 | CH₂-c-Pr | Me | CF₃ |
| 1-594 | SO₂Me | Cl | 2 | CH₂-c-Pr | Me | CF₃ |
| 1-595 | SO₂Me | Me | 0 | CH₂-c-Pr | Me | SO₂Me |
| 1-596 | SO₂Me | Me | 1 | CH₂-c-Pr | Me | SO₂Me |
| 1-597 | SO₂Me | Me | 2 | CH₂-c-Pr | Me | SO₂Me |
| 1-598 | SO₂Me | Cl | 0 | CH₂-c-Pr | Me | SO₂Me |
| 1-599 | SO₂Me | Cl | 1 | CH₂-c-Pr | Me | SO₂Me |
| 1-600 | SO₂Me | Cl | 2 | CH₂-c-Pr | Me | SO₂Me |
| 1-601 | Me | Me | 0 | CH₂CH₂OMe | H | H |
| 1-602 | Me | Me | 1 | CH₂CH₂OMe | H | H |
| 1-603 | Me | Me | 2 | CH₂CH₂OMe | H | H |
| 1-604 | Me | Cl | 0 | CH₂CH₂OMe | H | H |
| 1-605 | Me | Cl | 1 | CH₂CH₂OMe | H | H |
| 1-606 | Me | Cl | 2 | CH₂CH₂OMe | H | H |
| 1-607 | Me | Me | 0 | CH₂CH₂OMe | H | Me |
| 1-608 | Me | Me | 1 | CH₂CH₂OMe | H | Me |
| 1-609 | Me | Me | 2 | CH₂CH₂OMe | H | Me |
| 1-610 | Me | Cl | 0 | CH₂CH₂OMe | H | Me |
| 1-611 | Me | Cl | 1 | CH₂CH₂OMe | H | Me |
| 1-612 | Me | Cl | 2 | CH₂CH₂OMe | H | Me |
| 1-613 | Me | Me | 0 | CH₂CH₂OMe | H | Cl |
| 1-614 | Me | Me | 1 | CH₂CH₂OMe | H | Cl |
| 1-615 | Me | Me | 2 | CH₂CH₂OMe | H | Cl |
| 1-616 | Me | Cl | 0 | CH₂CH₂OMe | H | Cl |
| 1-617 | Me | Cl | 1 | CH₂CH₂OMe | H | Cl |
| 1-618 | Me | Cl | 2 | CH₂CH₂OMe | H | Cl |
| 1-619 | Me | Me | 0 | CH₂CH₂OMe | H | CF₃ |
| 1-620 | Me | Me | 1 | CH₂CH₂OMe | H | CF₃ |
| 1-621 | Me | Me | 2 | CH₂CH₂OMe | H | CF₃ |
| 1-622 | Me | Cl | 0 | CH₂CH₂OMe | H | CF₃ |
| 1-623 | Me | Cl | 1 | CH₂CH₂OMe | H | CF₃ |
| 1-624 | Me | Cl | 2 | CH₂CH₂OMe | H | CF₃ |
| 1-625 | Me | Me | 0 | CH₂CH₂OMe | H | SO₂Me |
| 1-626 | Me | Me | 1 | CH₂CH₂OMe | H | SO₂Me |
| 1-627 | Me | Me | 2 | CH₂CH₂OMe | H | SO₂Me |
| 1-628 | Me | Cl | 0 | CH₂CH₂OMe | H | SO₂Me |
| 1-629 | Me | Cl | 1 | CH₂CH₂OMe | H | SO₂Me |
| 1-630 | Me | Cl | 2 | CH₂CH₂OMe | H | SO₂Me |
| 1-631 | Me | Me | 0 | CH₂CH₂OMe | Me | H |
| 1-632 | Me | Me | 1 | CH₂CH₂OMe | Me | H |
| 1-633 | Me | Me | 2 | CH₂CH₂OMe | Me | H |
| 1-634 | Me | Cl | 0 | CH₂CH₂OMe | Me | H |
| 1-635 | Me | Cl | 1 | CH₂CH₂OMe | Me | H |
| 1-636 | Me | Cl | 2 | CH₂CH₂OMe | Me | H |
| 1-637 | Me | Me | 0 | CH₂CH₂OMe | Me | Me |
| 1-638 | Me | Me | 1 | CH₂CH₂OMe | Me | Me |
| 1-639 | Me | Me | 2 | CH₂CH₂OMe | Me | Me |
| 1-640 | Me | Cl | 0 | CH₂CH₂OMe | Me | Me |
| 1-641 | Me | Cl | 1 | CH₂CH₂OMe | Me | Me |
| 1-642 | Me | Cl | 2 | CH₂CH₂OMe | Me | Me |
| 1-643 | Me | Me | 0 | CH₂CH₂OMe | Me | Cl |
| 1-644 | Me | Me | 1 | CH₂CH₂OMe | Me | Cl |
| 1-645 | Me | Me | 2 | CH₂CH₂OMe | Me | Cl |
| 1-646 | Me | Cl | 0 | CH₂CH₂OMe | Me | Cl |
| 1-647 | Me | Cl | 1 | CH₂CH₂OMe | Me | Cl |
| 1-648 | Me | Cl | 2 | CH₂CH₂OMe | Me | Cl |
| 1-649 | Me | Me | 0 | CH₂CH₂OMe | Me | CF₃ |
| 1-650 | Me | Me | 1 | CH₂CH₂OMe | Me | CF₃ |
| 1-651 | Me | Me | 2 | CH₂CH₂OMe | Me | CF₃ |
| 1-652 | Me | Cl | 0 | CH₂CH₂OMe | Me | CF₃ |
| 1-653 | Me | Cl | 1 | CH₂CH₂OMe | Me | CF₃ |
| 1-654 | Me | Cl | 2 | CH₂CH₂OMe | Me | CF₃ |
| 1-655 | Me | Me | 0 | CH₂CH₂OMe | Me | SO₂Me |
| 1-656 | Me | Me | 1 | CH₂CH₂OMe | Me | SO₂Me |
| 1-657 | Me | Me | 2 | CH₂CH₂OMe | Me | SO₂Me |
| 1-658 | Me | Cl | 0 | CH₂CH₂OMe | Me | SO₂Me |
| 1-659 | Me | Cl | 1 | CH₂CH₂OMe | Me | SO₂Me |
| 1-660 | Me | Cl | 2 | CH₂CH₂OMe | Me | SO₂Me |
| 1-661 | c-Pr | Me | 0 | CH₂CH₂OMe | H | H |
| 1-662 | c-Pr | Me | 1 | CH₂CH₂OMe | H | H |
| 1-663 | c-Pr | Me | 2 | CH₂CH₂OMe | H | H |
| 1-664 | c-Pr | Cl | 0 | CH₂CH₂OMe | H | H |
| 1-665 | c-Pr | Cl | 1 | CH₂CH₂OMe | H | H |
| 1-666 | c-Pr | Cl | 2 | CH₂CH₂OMe | H | H |
| 1-667 | c-Pr | Me | 0 | CH₂CH₂OMe | H | Me |
| 1-668 | c-Pr | Me | 1 | CH₂CH₂OMe | H | Me |
| 1-669 | c-Pr | Me | 2 | CH₂CH₂OMe | H | Me |
| 1-670 | c-Pr | Cl | 0 | CH₂CH₂OMe | H | Me |
| 1-671 | c-Pr | Cl | 1 | CH₂CH₂OMe | H | Me |
| 1-672 | c-Pr | Cl | 2 | CH₂CH₂OMe | H | Me |
| 1-673 | c-Pr | Me | 0 | CH₂CH₂OMe | H | Cl |
| 1-674 | c-Pr | Me | 1 | CH₂CH₂OMe | H | Cl |
| 1-675 | c-Pr | Me | 2 | CH₂CH₂OMe | H | Cl |
| 1-676 | c-Pr | Cl | 0 | CH₂CH₂OMe | H | Cl |
| 1-677 | c-Pr | Cl | 1 | CH₂CH₂OMe | H | Cl |
| 1-678 | c-Pr | Cl | 2 | CH₂CH₂OMe | H | Cl |
| 1-679 | c-Pr | Me | 0 | CH₂CH₂OMe | H | CF₃ |
| 1-680 | c-Pr | Me | 1 | CH₂CH₂OMe | H | CF₃ |
| 1-681 | c-Pr | Me | 2 | CH₂CH₂OMe | H | CF₃ |
| 1-682 | c-Pr | Cl | 0 | CH₂CH₂OMe | H | CF₃ |
| 1-683 | c-Pr | Cl | 1 | CH₂CH₂OMe | H | CF₃ |
| 1-684 | c-Pr | Cl | 2 | CH₂CH₂OMe | H | CF₃ |
| 1-685 | c-Pr | Me | 0 | CH₂CH₂OMe | H | SO₂Me |
| 1-686 | c-Pr | Me | 1 | CH₂CH₂OMe | H | SO₂Me |
| 1-687 | c-Pr | Me | 2 | CH₂CH₂OMe | H | SO₂Me |
| 1-688 | c-Pr | Cl | 0 | CH₂CH₂OMe | H | SO₂Me |
| 1-689 | c-Pr | Cl | 1 | CH₂CH₂OMe | H | SO₂Me |
| 1-690 | c-Pr | Cl | 2 | CH₂CH₂OMe | H | SO₂Me |
| 1-691 | c-Pr | Me | 0 | CH₂CH₂OMe | Me | H |
| 1-692 | c-Pr | Me | 1 | CH₂CH₂OMe | Me | H |
| 1-693 | c-Pr | Me | 2 | CH₂CH₂OMe | Me | H |
| 1-694 | c-Pr | Cl | 0 | CH₂CH₂OMe | Me | H |
| 1-695 | c-Pr | Cl | 1 | CH₂CH₂OMe | Me | H |
| 1-696 | c-Pr | Cl | 2 | CH₂CH₂OMe | Me | H |
| 1-697 | c-Pr | Me | 0 | CH₂CH₂OMe | Me | Me |
| 1-698 | c-Pr | Me | 1 | CH₂CH₂OMe | Me | Me |
| 1-699 | c-Pr | Me | 2 | CH₂CH₂OMe | Me | Me |
| 1-700 | c-Pr | Cl | 0 | CH₂CH₂OMe | Me | Me |
| 1-701 | c-Pr | Cl | 1 | CH₂CH₂OMe | Me | Me |
| 1-702 | c-Pr | Cl | 2 | CH₂CH₂OMe | Me | Me |
| 1-703 | c-Pr | Me | 0 | CH₂CH₂OMe | Me | Cl |
| 1-704 | c-Pr | Me | 1 | CH₂CH₂OMe | Me | Cl |
| 1-705 | c-Pr | Me | 2 | CH₂CH₂OMe | Me | Cl |
| 1-706 | c-Pr | Cl | 0 | CH₂CH₂OMe | Me | Cl |
| 1-707 | c-Pr | Cl | 1 | CH₂CH₂OMe | Me | Cl |
| 1-708 | c-Pr | Cl | 2 | CH₂CH₂OMe | Me | Cl |
| 1-709 | c-Pr | Me | 0 | CH₂CH₂OMe | Me | CF₃ |
| 1-710 | c-Pr | Me | 1 | CH₂CH₂OMe | Me | CF₃ |
| 1-711 | c-Pr | Me | 2 | CH₂CH₂OMe | Me | CF₃ |
| 1-712 | c-Pr | Cl | 0 | CH₂CH₂OMe | Me | CF₃ |
| 1-713 | c-Pr | Cl | 1 | CH₂CH₂OMe | Me | CF₃ |
| 1-714 | c-Pr | Cl | 2 | CH₂CH₂OMe | Me | CF₃ |
| 1-715 | c-Pr | Me | 0 | CH₂CH₂OMe | Me | SO₂Me |
| 1-716 | c-Pr | Me | 1 | CH₂CH₂OMe | Me | SO₂Me |
| 1-717 | c-Pr | Me | 2 | CH₂CH₂OMe | Me | SO₂Me |
| 1-718 | c-Pr | Cl | 0 | CH₂CH₂OMe | Me | SO₂Me |
| 1-719 | c-Pr | Cl | 1 | CH₂CH₂OMe | Me | SO₂Me |
| 1-720 | c-Pr | Cl | 2 | CH₂CH₂OMe | Me | SO₂Me |
| 1-721 | Propen-2-yl | Me | 0 | CH₂CH₂OMe | H | H |
| 1-722 | Propen-2-yl | Me | 1 | CH₂CH₂OMe | H | H |
| 1-723 | Propen-2-yl | Me | 2 | CH₂CH₂OMe | H | H |
| 1-724 | Propen-2-yl | Cl | 0 | CH₂CH₂OMe | H | H |
| 1-725 | Propen-2-yl | Cl | 1 | CH₂CH₂OMe | H | H |
| 1-726 | Propen-2-yl | Cl | 2 | CH₂CH₂OMe | H | H |
| 1-727 | Propen-2-yl | Me | 0 | CH₂CH₂OMe | H | Me |
| 1-728 | Propen-2-yl | Me | 1 | CH₂CH₂OMe | H | Me |
| 1-729 | Propen-2-yl | Me | 2 | CH₂CH₂OMe | H | Me |
| 1-730 | Propen-2-yl | Cl | 0 | CH₂CH₂OMe | H | Me |
| 1-731 | Propen-2-yl | Cl | 1 | CH₂CH₂OMe | H | Me |
| 1-732 | Propen-2-yl | Cl | 2 | CH₂CH₂OMe | H | Me |
| 1-733 | Propen-2-yl | Me | 0 | CH₂CH₂OMe | H | Cl |
| 1-734 | Propen-2-yl | Me | 1 | CH₂CH₂OMe | H | Cl |
| 1-735 | Propen-2-yl | Me | 2 | CH₂CH₂OMe | H | Cl |
| 1-736 | Propen-2-yl | Cl | 0 | CH₂CH₂OMe | H | Cl |
| 1-737 | Propen-2-yl | Cl | 1 | CH₂CH₂OMe | H | Cl |
| 1-738 | Propen-2-yl | Cl | 2 | CH₂CH₂OMe | H | Cl |
| 1-739 | Propen-2-yl | Me | 0 | CH₂CH₂OMe | H | CF₃ |
| 1-740 | Propen-2-yl | Me | 1 | CH₂CH₂OMe | H | CF₃ |
| 1-741 | Propen-2-yl | Me | 2 | CH₂CH₂OMe | H | CF₃ |
| 1-742 | Propen-2-yl | Cl | 0 | CH₂CH₂OMe | H | CF₃ |
| 1-743 | Propen-2-yl | Cl | 1 | CH₂CH₂OMe | H | CF₃ |
| 1-744 | Propen-2-yl | Cl | 2 | CH₂CH₂OMe | H | CF₃ |
| 1-745 | Propen-2-yl | Me | 0 | CH₂CH₂OMe | H | SO₂Me |
| 1-746 | Propen-2-yl | Me | 1 | CH₂CH₂OMe | H | SO₂Me |
| 1-747 | Propen-2-yl | Me | 2 | CH₂CH₂OMe | H | SO₂Me |
| 1-748 | Propen-2-yl | Cl | 0 | CH₂CH₂OMe | H | SO₂Me |
| 1-749 | Propen-2-yl | Cl | 1 | CH₂CH₂OMe | H | SO₂Me |
| 1-750 | Propen-2-yl | Cl | 2 | CH₂CH₂OMe | H | SO₂Me |
| 1-751 | Propen-2-yl | Me | 0 | CH₂CH₂OMe | Me | H |
| 1-752 | Propen-2-yl | Me | 1 | CH₂CH₂OMe | Me | H |
| 1-753 | Propen-2-yl | Me | 2 | CH₂CH₂OMe | Me | H |
| 1-754 | Propen-2-yl | Cl | 0 | CH₂CH₂OMe | Me | H |
| 1-755 | Propen-2-yl | Cl | 1 | CH₂CH₂OMe | Me | H |
| 1-756 | Propen-2-yl | Cl | 2 | CH₂CH₂OMe | Me | H |
| 1-757 | Propen-2-yl | Me | 0 | CH₂CH₂OMe | Me | Me |
| 1-758 | Propen-2-yl | Me | 1 | CH₂CH₂OMe | Me | Me |
| 1-759 | Propen-2-yl | Me | 2 | CH₂CH₂OMe | Me | Me |
| 1-760 | Propen-2-yl | Cl | 0 | CH₂CH₂OMe | Me | Me |
| 1-761 | Propen-2-yl | Cl | 1 | CH₂CH₂OMe | Me | Me |
| 1-762 | Propen-2-yl | Cl | 2 | CH₂CH₂OMe | Me | Me |
| 1-763 | Propen-2-yl | Me | 0 | CH₂CH₂OMe | Me | Cl |
| 1-764 | Propen-2-yl | Me | 1 | CH₂CH₂OMe | Me | Cl |
| 1-765 | Propen-2-yl | Me | 2 | CH₂CH₂OMe | Me | Cl |
| 1-766 | Propen-2-yl | Cl | 0 | CH₂CH₂OMe | Me | Cl |
| 1-767 | Propen-2-yl | Cl | 1 | CH₂CH₂OMe | Me | Cl |
| 1-768 | Propen-2-yl | Cl | 2 | CH₂CH₂OMe | Me | Cl |
| 1-769 | Propen-2-yl | Me | 0 | CH₂CH₂OMe | Me | CF₃ |
| 1-770 | Propen-2-yl | Me | 1 | CH₂CH₂OMe | Me | CF₃ |
| 1-771 | Propen-2-yl | Me | 2 | CH₂CH₂OMe | Me | CF₃ |
| 1-772 | Propen-2-yl | Cl | 0 | CH₂CH₂OMe | Me | CF₃ |
| 1-773 | Propen-2-yl | Cl | 1 | CH₂CH₂OMe | Me | CF₃ |
| 1-774 | Propen-2-yl | Cl | 2 | CH₂CH₂OMe | Me | CF₃ |
| 1-775 | Propen-2-yl | Me | 0 | CH₂CH₂OMe | Me | SO₂Me |
| 1-776 | Propen-2-yl | Me | 1 | CH₂CH₂OMe | Me | SO₂Me |
| 1-777 | Propen-2-yl | Me | 2 | CH₂CH₂OMe | Me | SO₂Me |
| 1-778 | Propen-2-yl | Cl | 0 | CH₂CH₂OMe | Me | SO₂Me |
| 1-779 | Propen-2-yl | Cl | 1 | CH₂CH₂OMe | Me | SO₂Me |
| 1-780 | Propen-2-yl | Cl | 2 | CH₂CH₂OMe | Me | SO₂Me |
| 1-781 | Cl | Me | 0 | CH₂CH₂OMe | H | H |
| 1-782 | Cl | Me | 1 | CH₂CH₂OMe | H | H |
| 1-783 | Cl | Me | 2 | CH₂CH₂OMe | H | H |
| 1-784 | Cl | Cl | 0 | CH₂CH₂OMe | H | H |
| 1-785 | Cl | Cl | 1 | CH₂CH₂OMe | H | H |
| 1-786 | Cl | Cl | 2 | CH₂CH₂OMe | H | H |
| 1-787 | Cl | Me | 0 | CH₂CH₂OMe | H | Me |
| 1-788 | Cl | Me | 1 | CH₂CH₂OMe | H | Me |
| 1-789 | Cl | Me | 2 | CH₂CH₂OMe | H | Me |
| 1-790 | Cl | Cl | 0 | CH₂CH₂OMe | H | Me |
| 1-791 | Cl | Cl | 1 | CH₂CH₂OMe | H | Me |
| 1-792 | Cl | Cl | 2 | CH₂CH₂OMe | H | Me |
| 1-793 | Cl | Me | 0 | CH₂CH₂OMe | H | Cl |
| 1-794 | Cl | Me | 1 | CH₂CH₂OMe | H | Cl |
| 1-795 | Cl | Me | 2 | CH₂CH₂OMe | H | Cl |
| 1-796 | Cl | Cl | 0 | CH₂CH₂OMe | H | Cl |
| 1-797 | Cl | Cl | 1 | CH₂CH₂OMe | H | Cl |
| 1-798 | Cl | Cl | 2 | CH₂CH₂OMe | H | Cl |
| 1-799 | Cl | Me | 0 | CH₂CH₂OMe | H | CF₃ |
| 1-800 | Cl | Me | 1 | CH₂CH₂OMe | H | CF₃ |
| 1-801 | Cl | Me | 2 | CH₂CH₂OMe | H | CF₃ |
| 1-802 | Cl | Cl | 0 | CH₂CH₂OMe | H | CF₃ |
| 1-803 | Cl | Cl | 1 | CH₂CH₂OMe | H | CF₃ |
| 1-804 | Cl | Cl | 2 | CH₂CH₂OMe | H | CF₃ |
| 1-805 | Cl | Me | 0 | CH₂CH₂OMe | H | SO₂Me |
| 1-806 | Cl | Me | 1 | CH₂CH₂OMe | H | SO₂Me |
| 1-807 | Cl | Me | 2 | CH₂CH₂OMe | H | SO₂Me |
| 1-808 | Cl | Cl | 0 | CH₂CH₂OMe | H | SO₂Me |
| 1-809 | Cl | Cl | 1 | CH₂CH₂OMe | H | SO₂Me |
| 1-810 | Cl | Cl | 2 | CH₂CH₂OMe | H | SO₂Me |
| 1-811 | Cl | Me | 0 | CH₂CH₂OMe | Me | H |
| 1-812 | Cl | Me | 1 | CH₂CH₂OMe | Me | H |
| 1-813 | Cl | Me | 2 | CH₂CH₂OMe | Me | H |
| 1-814 | Cl | Cl | 0 | CH₂CH₂OMe | Me | H |
| 1-815 | Cl | Cl | 1 | CH₂CH₂OMe | Me | H |
| 1-816 | Cl | Cl | 2 | CH₂CH₂OMe | Me | H |
| 1-817 | Cl | Me | 0 | CH₂CH₂OMe | Me | Me |
| 1-818 | Cl | Me | 1 | CH₂CH₂OMe | Me | Me |
| 1-819 | Cl | Me | 2 | CH₂CH₂OMe | Me | Me |
| 1-820 | Cl | Cl | 0 | CH₂CH₂OMe | Me | Me |
| 1-821 | Cl | Cl | 1 | CH₂CH₂OMe | Me | Me |
| 1-822 | Cl | Cl | 2 | CH₂CH₂OMe | Me | Me |
| 1-823 | Cl | Me | 0 | CH₂CH₂OMe | Me | Cl |
| 1-824 | Cl | Me | 1 | CH₂CH₂OMe | Me | Cl |
| 1-825 | Cl | Me | 2 | CH₂CH₂OMe | Me | Cl |
| 1-826 | Cl | Cl | 0 | CH₂CH₂OMe | Me | Cl |
| 1-827 | Cl | Cl | 1 | CH₂CH₂OMe | Me | Cl |
| 1-828 | Cl | Cl | 2 | CH₂CH₂OMe | Me | Cl |
| 1-829 | Cl | Me | 0 | CH₂CH₂OMe | Me | CF₃ |
| 1-830 | Cl | Me | 1 | CH₂CH₂OMe | Me | CF₃ |
| 1-831 | Cl | Me | 2 | CH₂CH₂OMe | Me | CF₃ |
| 1-832 | Cl | Cl | 0 | CH₂CH₂OMe | Me | CF₃ |
| 1-833 | Cl | Cl | 1 | CH₂CH₂OMe | Me | CF₃ |
| 1-834 | Cl | Cl | 2 | CH₂CH₂OMe | Me | CF₃ |
| 1-835 | Cl | Me | 0 | CH₂CH₂OMe | Me | SO₂Me |
| 1-836 | Cl | Me | 1 | CH₂CH₂OMe | Me | SO₂Me |
| 1-837 | Cl | Me | 2 | CH₂CH₂OMe | Me | SO₂Me |
| 1-838 | Cl | Cl | 0 | CH₂CH₂OMe | Me | SO₂Me |
| 1-839 | Cl | Cl | 1 | CH₂CH₂OMe | Me | SO₂Me |
| 1-840 | Cl | Cl | 2 | CH₂CH₂OMe | Me | SO₂Me |
| 1-841 | SO₂Me | Me | 0 | CH₂CH₂OMe | H | H |
| 1-842 | SO₂Me | Me | 1 | CH₂CH₂OMe | H | H |
| 1-843 | SO₂Me | Me | 2 | CH₂CH₂OMe | H | H |
| 1-844 | SO₂Me | Cl | 0 | CH₂CH₂OMe | H | H |
| 1-845 | SO₂Me | Cl | 1 | CH₂CH₂OMe | H | H |
| 1-846 | SO₂Me | Cl | 2 | CH₂CH₂OMe | H | H |
| 1-847 | SO₂Me | Me | 0 | CH₂CH₂OMe | H | Me |
| 1-848 | SO₂Me | Me | 1 | CH₂CH₂OMe | H | Me |
| 1-849 | SO₂Me | Me | 2 | CH₂CH₂OMe | H | Me |
| 1-850 | SO₂Me | Cl | 0 | CH₂CH₂OMe | H | Me |
| 1-851 | SO₂Me | Cl | 1 | CH₂CH₂OMe | H | Me |
| 1-852 | SO₂Me | Cl | 2 | CH₂CH₂OMe | H | Me |
| 1-853 | SO₂Me | Me | 0 | CH₂CH₂OMe | H | Cl |
| 1-854 | SO₂Me | Me | 1 | CH₂CH₂OMe | H | Cl |
| 1-855 | SO₂Me | Me | 2 | CH₂CH₂OMe | H | Cl |
| 1-856 | SO₂Me | Cl | 0 | CH₂CH₂OMe | H | Cl |
| 1-857 | SO₂Me | Cl | 1 | CH₂CH₂OMe | H | Cl |
| 1-858 | SO₂Me | Cl | 2 | CH₂CH₂OMe | H | Cl |
| 1-859 | SO₂Me | Me | 0 | CH₂CH₂OMe | H | CF₃ |
| 1-860 | SO₂Me | Me | 1 | CH₂CH₂OMe | H | CF₃ |
| 1-861 | SO₂Me | Me | 2 | CH₂CH₂OMe | H | CF₃ |
| 1-862 | SO₂Me | Cl | 0 | CH₂CH₂OMe | H | CF₃ |
| 1-863 | SO₂Me | Cl | 1 | CH₂CH₂OMe | H | CF₃ |
| 1-864 | SO₂Me | Cl | 2 | CH₂CH₂OMe | H | CF₃ |
| 1-865 | SO₂Me | Me | 0 | CH₂CH₂OMe | H | SO₂Me |
| 1-866 | SO₂Me | Me | 1 | CH₂CH₂OMe | H | SO₂Me |
| 1-867 | SO₂Me | Me | 2 | CH₂CH₂OMe | H | SO₂Me |
| 1-868 | SO₂Me | Cl | 0 | CH₂CH₂OMe | H | SO₂Me |
| 1-869 | SO₂Me | Cl | 1 | CH₂CH₂OMe | H | SO₂Me |
| 1-870 | SO₂Me | Cl | 2 | CH₂CH₂OMe | H | SO₂Me |
| 1-871 | SO₂Me | Me | 0 | CH₂CH₂OMe | Me | H |
| 1-872 | SO₂Me | Me | 1 | CH₂CH₂OMe | Me | H |
| 1-873 | SO₂Me | Me | 2 | CH₂CH₂OMe | Me | H |
| 1-874 | SO₂Me | Cl | 0 | CH₂CH₂OMe | Me | H |
| 1-875 | SO₂Me | Cl | 1 | CH₂CH₂OMe | Me | H |
| 1-876 | SO₂Me | Cl | 2 | CH₂CH₂OMe | Me | H |
| 1-877 | SO₂Me | Me | 0 | CH₂CH₂OMe | Me | Me |
| 1-878 | SO₂Me | Me | 1 | CH₂CH₂OMe | Me | Me |
| 1-879 | SO₂Me | Me | 2 | CH₂CH₂OMe | Me | Me |
| 1-880 | SO₂Me | Cl | 0 | CH₂CH₂OMe | Me | Me |
| 1-881 | SO₂Me | Cl | 1 | CH₂CH₂OMe | Me | Me |
| 1-882 | SO₂Me | Cl | 2 | CH₂CH₂OMe | Me | Me |
| 1-883 | SO₂Me | Me | 0 | CH₂CH₂OMe | Me | Cl |
| 1-884 | SO₂Me | Me | 1 | CH₂CH₂OMe | Me | Cl |
| 1-885 | SO₂Me | Me | 2 | CH₂CH₂OMe | Me | Cl |
| 1-886 | SO₂Me | Cl | 0 | CH₂CH₂OMe | Me | Cl |
| 1-887 | SO₂Me | Cl | 1 | CH₂CH₂OMe | Me | Cl |
| 1-888 | SO₂Me | Cl | 2 | CH₂CH₂OMe | Me | Cl |
| 1-889 | SO₂Me | Me | 0 | CH₂CH₂OMe | Me | CF₃ |
| 1-890 | SO₂Me | Me | 1 | CH₂CH₂OMe | Me | CF₃ |
| 1-891 | SO₂Me | Me | 2 | CH₂CH₂OMe | Me | CF₃ |
| 1-892 | SO₂Me | Cl | 0 | CH₂CH₂OMe | Me | CF₃ |
| 1-893 | SO₂Me | Cl | 1 | CH₂CH₂OMe | Me | CF₃ |
| 1-894 | SO₂Me | Cl | 2 | CH₂CH₂OMe | Me | CF₃ |
| 1-895 | SO₂Me | Me | 0 | CH₂CH₂OMe | Me | SO₂Me |
| 1-896 | SO₂Me | Me | 1 | CH₂CH₂OMe | Me | SO₂Me |
| 1-897 | SO₂Me | Me | 2 | CH₂CH₂OMe | Me | SO₂Me |
| 1-898 | SO₂Me | Cl | 0 | CH₂CH₂OMe | Me | SO₂Me |
| 1-899 | SO₂Me | Cl | 1 | CH₂CH₂OMe | Me | SO₂Me |
| 1-900 | SO₂Me | Cl | 2 | CH₂CH₂OMe | Me | SO₂Me |

**Tabelle 2: Erfindungsgemäße Verbindungen der allgemeinen Formel (I), worin R² und R³ jeweils Wasserstoff bedeuten, und A für -CH₂-, X¹ für CH, X² für CR⁷ und X³ für CR⁸ stehen**

| | | | | | | |
|---|---|---|---|---|---|---|
| 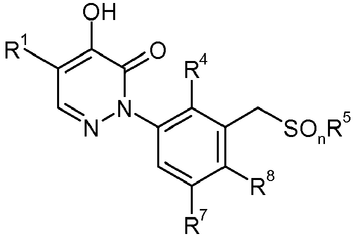 | | | | | | |

| **Nr.** | **R¹** | **R⁴** | **n** | **R⁵** | **R⁷** | **R⁸** |
|---|---|---|---|---|---|---|
| 2-1 | Me | Me | 0 | Me | H | H |
| 2-2 | Me | Me | 1 | Me | H | H |
| 2-3 | Me | Me | 2 | Me | H | H |
| 2-4 | Me | Cl | 0 | Me | H | H |
| 2-5 | Me | Cl | 1 | Me | H | H |
| 2-6 | Me | Cl | 2 | Me | H | H |
| 2-7 | Me | Me | 0 | Me | H | Me |
| 2-8 | Me | Me | 1 | Me | H | Me |
| 2-9 | Me | Me | 2 | Me | H | Me |
| 2-10 | Me | Cl | 0 | Me | H | Me |
| 2-11 | Me | Cl | 1 | Me | H | Me |
| 2-12 | Me | Cl | 2 | Me | H | Me |
| 2-13 | Me | Me | 0 | Me | H | Cl |
| 2-14 | Me | Me | 1 | Me | H | Cl |
| 2-15 | Me | Me | 2 | Me | H | Cl |
| 2-16 | Me | Cl | 0 | Me | H | Cl |
| 2-17 | Me | Cl | 1 | Me | H | Cl |
| 2-18 | Me | Cl | 2 | Me | H | Cl |
| 2-19 | Me | Me | 0 | Me | H | CF₃ |
| 2-20 | Me | Me | 1 | Me | H | CF₃ |
| 2-21 | Me | Me | 2 | Me | H | CF₃ |
| 2-22 | Me | Cl | 0 | Me | H | CF₃ |
| 2-23 | Me | Cl | 1 | Me | H | CF₃ |
| 2-24 | Me | Cl | 2 | Me | H | CF₃ |
| 2-25 | Me | Me | 0 | Me | H | SO₂Me |
| 2-26 | Me | Me | 1 | Me | H | SO₂Me |
| 2-27 | Me | Me | 2 | Me | H | SO₂Me |
| 2-28 | Me | Cl | 0 | Me | H | SO₂Me |
| 2-29 | Me | Cl | 1 | Me | H | SO₂Me |
| 2-30 | Me | Cl | 2 | Me | H | SO₂Me |
| 2-31 | Me | Me | 0 | Me | Me | H |
| 2-32 | Me | Me | 1 | Me | Me | H |
| 2-33 | Me | Me | 2 | Me | Me | H |
| 2-34 | Me | Cl | 0 | Me | Me | H |
| 2-35 | Me | Cl | 1 | Me | Me | H |
| 2-36 | Me | Cl | 2 | Me | Me | H |
| 2-37 | Me | Me | 0 | Me | Me | Me |
| 2-38 | Me | Me | 1 | Me | Me | Me |
| 2-39 | Me | Me | 2 | Me | Me | Me |
| 2-40 | Me | Cl | 0 | Me | Me | Me |
| 2-41 | Me | Cl | 1 | Me | Me | Me |
| 2-42 | Me | Cl | 2 | Me | Me | Me |
| 2-43 | Me | Me | 0 | Me | Me | Cl |
| 2-44 | Me | Me | 1 | Me | Me | Cl |
| 2-45 | Me | Me | 2 | Me | Me | Cl |
| 2-46 | Me | Cl | 0 | Me | Me | Cl |
| 2-47 | Me | Cl | 1 | Me | Me | Cl |
| 2-48 | Me | Cl | 2 | Me | Me | Cl |
| 2-49 | Me | Me | 0 | Me | Me | CF₃ |
| 2-50 | Me | Me | 1 | Me | Me | CF₃ |
| 2-51 | Me | Me | 2 | Me | Me | CF₃ |
| 2-52 | Me | Cl | 0 | Me | Me | CF₃ |
| 2-53 | Me | Cl | 1 | Me | Me | CF₃ |
| 2-54 | Me | Cl | 2 | Me | Me | CF₃ |
| 2-55 | Me | Me | 0 | Me | Me | SO₂Me |
| 2-56 | Me | Me | 1 | Me | Me | SO₂Me |
| 2-57 | Me | Me | 2 | Me | Me | SO₂Me |
| 2-58 | Me | Cl | 0 | Me | Me | SO₂Me |
| 2-59 | Me | Cl | 1 | Me | Me | SO₂Me |
| 2-60 | Me | Cl | 2 | Me | Me | SO₂Me |
| 2-61 | c-Pr | Me | 0 | Me | H | H |
| 2-62 | c-Pr | Me | 1 | Me | H | H |
| 2-63 | c-Pr | Me | 2 | Me | H | H |
| 2-64 | c-Pr | Cl | 0 | Me | H | H |
| 2-65 | c-Pr | Cl | 1 | Me | H | H |
| 2-66 | c-Pr | Cl | 2 | Me | H | H |
| 2-67 | c-Pr | Me | 0 | Me | H | Me |
| 2-68 | c-Pr | Me | 1 | Me | H | Me |
| 2-69 | c-Pr | Me | 2 | Me | H | Me |
| 2-70 | c-Pr | Cl | 0 | Me | H | Me |
| 2-71 | c-Pr | Cl | 1 | Me | H | Me |
| 2-72 | c-Pr | Cl | 2 | Me | H | Me |
| 2-73 | c-Pr | Me | 0 | Me | H | Cl |
| 2-74 | c-Pr | Me | 1 | Me | H | Cl |
| 2-75 | c-Pr | Me | 2 | Me | H | Cl |
| 2-76 | c-Pr | Cl | 0 | Me | H | Cl |
| 2-77 | c-Pr | Cl | 1 | Me | H | Cl |
| 2-78 | c-Pr | Cl | 2 | Me | H | Cl |
| 2-79 | c-Pr | Me | 0 | Me | H | CF₃ |
| 2-80 | c-Pr | Me | 1 | Me | H | CF₃ |
| 2-81 | c-Pr | Me | 2 | Me | H | CF₃ |
| 2-82 | c-Pr | Cl | 0 | Me | H | CF₃ |
| 2-83 | c-Pr | Cl | 1 | Me | H | CF₃ |
| 2-84 | c-Pr | Cl | 2 | Me | H | CF₃ |
| 2-85 | c-Pr | Me | 0 | Me | H | SO₂Me |
| 2-86 | c-Pr | Me | 1 | Me | H | SO₂Me |
| 2-87 | c-Pr | Me | 2 | Me | H | SO₂Me |
| 2-88 | c-Pr | Cl | 0 | Me | H | SO₂Me |
| 2-89 | c-Pr | Cl | 1 | Me | H | SO₂Me |
| 2-90 | c-Pr | Cl | 2 | Me | H | SO₂Me |
| 2-91 | c-Pr | Me | 0 | Me | Me | H |
| 2-92 | c-Pr | Me | 1 | Me | Me | H |
| 2-93 | c-Pr | Me | 2 | Me | Me | H |
| 2-94 | c-Pr | Cl | 0 | Me | Me | H |
| 2-95 | c-Pr | Cl | 1 | Me | Me | H |
| 2-96 | c-Pr | Cl | 2 | Me | Me | H |
| 2-97 | c-Pr | Me | 0 | Me | Me | Me |
| 2-98 | c-Pr | Me | 1 | Me | Me | Me |
| 2-99 | c-Pr | Me | 2 | Me | Me | Me |
| 2-100 | c-Pr | Cl | 0 | Me | Me | Me |
| 2-101 | c-Pr | Cl | 1 | Me | Me | Me |
| 2-102 | c-Pr | Cl | 2 | Me | Me | Me |
| 2-103 | c-Pr | Me | 0 | Me | Me | Cl |
| 2-104 | c-Pr | Me | 1 | Me | Me | Cl |
| 2-105 | c-Pr | Me | 2 | Me | Me | Cl |
| 2-106 | c-Pr | Cl | 0 | Me | Me | Cl |
| 2-107 | c-Pr | Cl | 1 | Me | Me | Cl |
| 2-108 | c-Pr | Cl | 2 | Me | Me | Cl |
| 2-109 | c-Pr | Me | 0 | Me | Me | CF₃ |
| 2-110 | c-Pr | Me | 1 | Me | Me | CF₃ |
| 2-111 | c-Pr | Me | 2 | Me | Me | CF₃ |
| 2-112 | c-Pr | Cl | 0 | Me | Me | CF₃ |
| 2-113 | c-Pr | Cl | 1 | Me | Me | CF₃ |
| 2-114 | c-Pr | Cl | 2 | Me | Me | CF₃ |
| 2-115 | c-Pr | Me | 0 | Me | Me | SO₂Me |
| 2-116 | c-Pr | Me | 1 | Me | Me | SO₂Me |
| 2-117 | c-Pr | Me | 2 | Me | Me | SO₂Me |
| 2-118 | c-Pr | Cl | 0 | Me | Me | SO₂Me |
| 2-119 | c-Pr | Cl | 1 | Me | Me | SO₂Me |
| 2-120 | c-Pr | Cl | 2 | Me | Me | SO₂Me |
| 2-121 | Cl | Me | 0 | Me | H | H |
| 2-122 | Cl | Me | 1 | Me | H | H |
| 2-123 | Cl | Me | 2 | Me | H | H |
| 2-124 | Cl | Cl | 0 | Me | H | H |
| 2-125 | Cl | Cl | 1 | Me | H | H |
| 2-126 | Cl | Cl | 2 | Me | H | H |
| 2-127 | Cl | Me | 0 | Me | H | Me |
| 2-128 | Cl | Me | 1 | Me | H | Me |
| 2-129 | Cl | Me | 2 | Me | H | Me |
| 2-130 | Cl | Cl | 0 | Me | H | Me |
| 2-131 | Cl | Cl | 1 | Me | H | Me |
| 2-132 | Cl | Cl | 2 | Me | H | Me |
| 2-133 | Cl | Me | 0 | Me | H | Cl |
| 2-134 | Cl | Me | 1 | Me | H | Cl |
| 2-135 | Cl | Me | 2 | Me | H | Cl |
| 2-136 | Cl | Cl | 0 | Me | H | Cl |
| 2-137 | Cl | Cl | 1 | Me | H | Cl |
| 2-138 | Cl | Cl | 2 | Me | H | Cl |
| 2-139 | Cl | Me | 0 | Me | H | CF₃ |
| 2-140 | Cl | Me | 1 | Me | H | CF₃ |
| 2-141 | Cl | Me | 2 | Me | H | CF₃ |
| 2-142 | Cl | Cl | 0 | Me | H | CF₃ |
| 2-143 | Cl | Cl | 1 | Me | H | CF₃ |
| 2-144 | Cl | Cl | 2 | Me | H | CF₃ |
| 2-145 | Cl | Me | 0 | Me | H | SO₂Me |
| 2-146 | Cl | Me | 1 | Me | H | SO₂Me |
| 2-147 | Cl | Me | 2 | Me | H | SO₂Me |
| 2-148 | Cl | Cl | 0 | Me | H | SO₂Me |
| 2-149 | Cl | Cl | 1 | Me | H | SO₂Me |
| 2-150 | Cl | Cl | 2 | Me | H | SO₂Me |
| 2-151 | Cl | Me | 0 | Me | Me | H |
| 2-152 | Cl | Me | 1 | Me | Me | H |
| 2-153 | Cl | Me | 2 | Me | Me | H |
| 2-154 | Cl | Cl | 0 | Me | Me | H |
| 2-155 | Cl | Cl | 1 | Me | Me | H |
| 2-156 | Cl | Cl | 2 | Me | Me | H |
| 2-157 | Cl | Me | 0 | Me | Me | Me |
| 2-158 | Cl | Me | 1 | Me | Me | Me |
| 2-159 | Cl | Me | 2 | Me | Me | Me |
| 2-160 | Cl | Cl | 0 | Me | Me | Me |
| 2-161 | Cl | Cl | 1 | Me | Me | Me |
| 2-162 | Cl | Cl | 2 | Me | Me | Me |
| 2-163 | Cl | Me | 0 | Me | Me | Cl |
| 2-164 | Cl | Me | 1 | Me | Me | Cl |
| 2-165 | Cl | Me | 2 | Me | Me | Cl |
| 2-166 | Cl | Cl | 0 | Me | Me | Cl |
| 2-167 | Cl | Cl | 1 | Me | Me | Cl |
| 2-168 | Cl | Cl | 2 | Me | Me | Cl |
| 2-169 | Cl | Me | 0 | Me | Me | CF₃ |
| 2-170 | Cl | Me | 1 | Me | Me | CF₃ |
| 2-171 | Cl | Me | 2 | Me | Me | CF₃ |
| 2-172 | Cl | Cl | 0 | Me | Me | CF₃ |
| 2-173 | Cl | Cl | 1 | Me | Me | CF₃ |
| 2-174 | Cl | Cl | 2 | Me | Me | CF₃ |
| 2-175 | Cl | Me | 0 | Me | Me | SO₂Me |
| 2-176 | Cl | Me | 1 | Me | Me | SO₂Me |
| 2-177 | Cl | Me | 2 | Me | Me | SO₂Me |
| 2-178 | Cl | Cl | 0 | Me | Me | SO₂Me |
| 2-179 | Cl | Cl | 1 | Me | Me | SO₂Me |
| 2-180 | Cl | Cl | 2 | Me | Me | SO₂Me |
| 2-181 | SO₂Me | Me | 0 | Me | H | H |
| 2-182 | SO₂Me | Me | 1 | Me | H | H |
| 2-183 | SO₂Me | Me | 2 | Me | H | H |
| 2-184 | SO₂Me | Cl | 0 | Me | H | H |
| 2-185 | SO₂Me | Cl | 1 | Me | H | H |
| 2-186 | SO₂Me | Cl | 2 | Me | H | H |
| 2-187 | SO₂Me | Me | 0 | Me | H | Me |
| 2-188 | SO₂Me | Me | 1 | Me | H | Me |
| 2-189 | SO₂Me | Me | 2 | Me | H | Me |
| 2-190 | SO₂Me | Cl | 0 | Me | H | Me |
| 2-191 | SO₂Me | Cl | 1 | Me | H | Me |
| 2-192 | SO₂Me | Cl | 2 | Me | H | Me |
| 2-193 | SO₂Me | Me | 0 | Me | H | Cl |
| 2-194 | SO₂Me | Me | 1 | Me | H | Cl |
| 2-195 | SO₂Me | Me | 2 | Me | H | Cl |
| 2-196 | SO₂Me | Cl | 0 | Me | H | Cl |
| 2-197 | SO₂Me | Cl | 1 | Me | H | Cl |
| 2-198 | SO₂Me | Cl | 2 | Me | H | Cl |
| 2-199 | SO₂Me | Me | 0 | Me | H | CF₃ |
| 2-200 | SO₂Me | Me | 1 | Me | H | CF₃ |
| 2-201 | SO₂Me | Me | 2 | Me | H | CF₃ |
| 2-202 | SO₂Me | Cl | 0 | Me | H | CF₃ |
| 2-203 | SO₂Me | Cl | 1 | Me | H | CF₃ |
| 2-204 | SO₂Me | Cl | 2 | Me | H | CF₃ |
| 2-205 | SO₂Me | Me | 0 | Me | H | SO₂Me |
| 2-206 | SO₂Me | Me | 1 | Me | H | SO₂Me |
| 2-207 | SO₂Me | Me | 2 | Me | H | SO₂Me |
| 2-208 | SO₂Me | Cl | 0 | Me | H | SO₂Me |
| 2-209 | SO₂Me | Cl | 1 | Me | H | SO₂Me |
| 2-210 | SO₂Me | Cl | 2 | Me | H | SO₂Me |
| 2-211 | SO₂Me | Me | 0 | Me | Me | H |
| 2-212 | SO₂Me | Me | 1 | Me | Me | H |
| 2-213 | SO₂Me | Me | 2 | Me | Me | H |
| 2-214 | SO₂Me | Cl | 0 | Me | Me | H |
| 2-215 | SO₂Me | Cl | 1 | Me | Me | H |
| 2-216 | SO₂Me | Cl | 2 | Me | Me | H |
| 2-217 | SO₂Me | Me | 0 | Me | Me | Me |
| 2-218 | SO₂Me | Me | 1 | Me | Me | Me |
| 2-219 | SO₂Me | Me | 2 | Me | Me | Me |
| 2-220 | SO₂Me | Cl | 0 | Me | Me | Me |
| 2-221 | SO₂Me | Cl | 1 | Me | Me | Me |
| 2-222 | SO₂Me | Cl | 2 | Me | Me | Me |
| 2-223 | SO₂Me | Me | 0 | Me | Me | Cl |
| 2-224 | SO₂Me | Me | 1 | Me | Me | Cl |
| 2-225 | SO₂Me | Me | 2 | Me | Me | Cl |
| 2-226 | SO₂Me | Cl | 0 | Me | Me | Cl |
| 2-227 | SO₂Me | Cl | 1 | Me | Me | Cl |
| 2-228 | SO₂Me | Cl | 2 | Me | Me | Cl |
| 2-229 | SO₂Me | Me | 0 | Me | Me | CF₃ |
| 2-230 | SO₂Me | Me | 1 | Me | Me | CF₃ |
| 2-231 | SO₂Me | Me | 2 | Me | Me | CF₃ |
| 2-232 | SO₂Me | Cl | 0 | Me | Me | CF₃ |
| 2-233 | SO₂Me | Cl | 1 | Me | Me | CF₃ |
| 2-234 | SO₂Me | Cl | 2 | Me | Me | CF₃ |
| 2-235 | SO₂Me | Me | 0 | Me | Me | SO₂Me |
| 2-236 | SO₂Me | Me | 1 | Me | Me | SO₂Me |
| 2-237 | SO₂Me | Me | 2 | Me | Me | SO₂Me |
| 2-238 | SO₂Me | Cl | 0 | Me | Me | SO₂Me |
| 2-239 | SO₂Me | Cl | 1 | Me | Me | SO₂Me |
| 2-240 | SO₂Me | Cl | 2 | Me | Me | SO₂Me |
| 2-241 | Me | Me | 0 | CH₂CH₂OMe | H | H |
| 2-242 | Me | Me | 1 | CH₂CH₂OMe | H | H |
| 2-243 | Me | Me | 2 | CH₂CH₂OMe | H | H |
| 2-244 | Me | Cl | 0 | CH₂CH₂OMe | H | H |
| 2-245 | Me | Cl | 1 | CH₂CH₂OMe | H | H |
| 2-246 | Me | Cl | 2 | CH₂CH₂OMe | H | H |
| 2-247 | Me | Me | 0 | CH₂CH₂OMe | H | Me |
| 2-248 | Me | Me | 1 | CH₂CH₂OMe | H | Me |
| 2-249 | Me | Me | 2 | CH₂CH₂OMe | H | Me |
| 2-250 | Me | Cl | 0 | CH₂CH₂OMe | H | Me |
| 2-251 | Me | Cl | 1 | CH₂CH₂OMe | H | Me |
| 2-252 | Me | Cl | 2 | CH₂CH₂OMe | H | Me |
| 2-253 | Me | Me | 0 | CH₂CH₂OMe | H | Cl |
| 2-254 | Me | Me | 1 | CH₂CH₂OMe | H | Cl |
| 2-255 | Me | Me | 2 | CH₂CH₂OMe | H | Cl |
| 2-256 | Me | Cl | 0 | CH₂CH₂OMe | H | Cl |
| 2-257 | Me | Cl | 1 | CH₂CH₂OMe | H | Cl |
| 2-258 | Me | Cl | 2 | CH₂CH₂OMe | H | Cl |
| 2-259 | Me | Me | 0 | CH₂CH₂OMe | H | CF₃ |
| 2-260 | Me | Me | 1 | CH₂CH₂OMe | H | CF₃ |
| 2-261 | Me | Me | 2 | CH₂CH₂OMe | H | CF₃ |
| 2-262 | Me | Cl | 0 | CH₂CH₂OMe | H | CF₃ |
| 2-263 | Me | Cl | 1 | CH₂CH₂OMe | H | CF₃ |
| 2-264 | Me | Cl | 2 | CH₂CH₂OMe | H | CF₃ |
| 2-265 | Me | Me | 0 | CH₂CH₂OMe | H | SO₂Me |
| 2-266 | Me | Me | 1 | CH₂CH₂OMe | H | SO₂Me |
| 2-267 | Me | Me | 2 | CH₂CH₂OMe | H | SO₂Me |
| 2-268 | Me | Cl | 0 | CH₂CH₂OMe | H | SO₂Me |
| 2-269 | Me | Cl | 1 | CH₂CH₂OMe | H | SO₂Me |
| 2-270 | Me | Cl | 2 | CH₂CH₂OMe | H | SO₂Me |
| 2-271 | Me | Me | 0 | CH₂CH₂OMe | Me | H |
| 2-272 | Me | Me | 1 | CH₂CH₂OMe | Me | H |
| 2-273 | Me | Me | 2 | CH₂CH₂OMe | Me | H |
| 2-274 | Me | Cl | 0 | CH₂CH₂OMe | Me | H |
| 2-275 | Me | Cl | 1 | CH₂CH₂OMe | Me | H |
| 2-276 | Me | Cl | 2 | CH₂CH₂OMe | Me | H |
| 2-277 | Me | Me | 0 | CH₂CH₂OMe | Me | Me |
| 2-278 | Me | Me | 1 | CH₂CH₂OMe | Me | Me |
| 2-279 | Me | Me | 2 | CH₂CH₂OMe | Me | Me |
| 2-280 | Me | Cl | 0 | CH₂CH₂OMe | Me | Me |
| 2-281 | Me | Cl | 1 | CH₂CH₂OMe | Me | Me |
| 2-282 | Me | Cl | 2 | CH₂CH₂OMe | Me | Me |
| 2-283 | Me | Me | 0 | CH₂CH₂OMe | Me | Cl |
| 2-284 | Me | Me | 1 | CH₂CH₂OMe | Me | Cl |
| 2-285 | Me | Me | 2 | CH₂CH₂OMe | Me | Cl |
| 2-286 | Me | Cl | 0 | CH₂CH₂OMe | Me | Cl |
| 2-287 | Me | Cl | 1 | CH₂CH₂OMe | Me | Cl |
| 2-288 | Me | Cl | 2 | CH₂CH₂OMe | Me | Cl |
| 2-289 | Me | Me | 0 | CH₂CH₂OMe | Me | CF₃ |
| 2-290 | Me | Me | 1 | CH₂CH₂OMe | Me | CF₃ |
| 2-291 | Me | Me | 2 | CH₂CH₂OMe | Me | CF₃ |
| 2-292 | Me | Cl | 0 | CH₂CH₂OMe | Me | CF₃ |
| 2-293 | Me | Cl | 1 | CH₂CH₂OMe | Me | CF₃ |
| 2-294 | Me | Cl | 2 | CH₂CH₂OMe | Me | CF₃ |
| 2-295 | Me | Me | 0 | CH₂CH₂OMe | Me | SO₂Me |
| 2-296 | Me | Me | 1 | CH₂CH₂OMe | Me | SO₂Me |
| 2-297 | Me | Me | 2 | CH₂CH₂OMe | Me | SO₂Me |
| 2-298 | Me | Cl | 0 | CH₂CH₂OMe | Me | SO₂Me |
| 2-299 | Me | Cl | 1 | CH₂CH₂OMe | Me | SO₂Me |
| 2-300 | Me | Cl | 2 | CH₂CH₂OMe | Me | SO₂Me |
| 2-301 | c-Pr | Me | 0 | CH₂CH₂OMe | H | H |
| 2-302 | c-Pr | Me | 1 | CH₂CH₂OMe | H | H |
| 2-303 | c-Pr | Me | 2 | CH₂CH₂OMe | H | H |
| 2-304 | c-Pr | Cl | 0 | CH₂CH₂OMe | H | H |
| 2-305 | c-Pr | Cl | 1 | CH₂CH₂OMe | H | H |
| 2-306 | c-Pr | Cl | 2 | CH₂CH₂OMe | H | H |
| 2-307 | c-Pr | Me | 0 | CH₂CH₂OMe | H | Me |
| 2-308 | c-Pr | Me | 1 | CH₂CH₂OMe | H | Me |
| 2-309 | c-Pr | Me | 2 | CH₂CH₂OMe | H | Me |
| 2-310 | c-Pr | Cl | 0 | CH₂CH₂OMe | H | Me |
| 2-311 | c-Pr | Cl | 1 | CH₂CH₂OMe | H | Me |
| 2-312 | c-Pr | Cl | 2 | CH₂CH₂OMe | H | Me |
| 2-313 | c-Pr | Me | 0 | CH₂CH₂OMe | H | Cl |
| 2-314 | c-Pr | Me | 1 | CH₂CH₂OMe | H | Cl |
| 2-315 | c-Pr | Me | 2 | CH₂CH₂OMe | H | Cl |
| 2-316 | c-Pr | Cl | 0 | CH₂CH₂OMe | H | Cl |
| 2-317 | c-Pr | Cl | 1 | CH₂CH₂OMe | H | Cl |
| 2-318 | c-Pr | Cl | 2 | CH₂CH₂OMe | H | Cl |
| 2-319 | c-Pr | Me | 0 | CH₂CH₂OMe | H | CF₃ |
| 2-320 | c-Pr | Me | 1 | CH₂CH₂OMe | H | CF₃ |
| 2-321 | c-Pr | Me | 2 | CH₂CH₂OMe | H | CF₃ |
| 2-322 | c-Pr | Cl | 0 | CH₂CH₂OMe | H | CF₃ |
| 2-323 | c-Pr | Cl | 1 | CH₂CH₂OMe | H | CF₃ |
| 2-324 | c-Pr | Cl | 2 | CH₂CH₂OMe | H | CF₃ |
| 2-325 | c-Pr | Me | 0 | CH₂CH₂OMe | H | SO₂Me |
| 2-326 | c-Pr | Me | 1 | CH₂CH₂OMe | H | SO₂Me |
| 2-327 | c-Pr | Me | 2 | CH₂CH₂OMe | H | SO₂Me |
| 2-328 | c-Pr | Cl | 0 | CH₂CH₂OMe | H | SO₂Me |
| 2-329 | c-Pr | Cl | 1 | CH₂CH₂OMe | H | SO₂Me |
| 2-330 | c-Pr | Cl | 2 | CH₂CH₂OMe | H | SO₂Me |
| 2-331 | c-Pr | Me | 0 | CH₂CH₂OMe | Me | H |
| 2-332 | c-Pr | Me | 1 | CH₂CH₂OMe | Me | H |
| 2-333 | c-Pr | Me | 2 | CH₂CH₂OMe | Me | H |
| 2-334 | c-Pr | Cl | 0 | CH₂CH₂OMe | Me | H |
| 2-335 | c-Pr | Cl | 1 | CH₂CH₂OMe | Me | H |
| 2-336 | c-Pr | Cl | 2 | CH₂CH₂OMe | Me | H |
| 2-337 | c-Pr | Me | 0 | CH₂CH₂OMe | Me | Me |
| 2-338 | c-Pr | Me | 1 | CH₂CH₂OMe | Me | Me |
| 2-339 | c-Pr | Me | 2 | CH₂CH₂OMe | Me | Me |
| 2-340 | c-Pr | Cl | 0 | CH₂CH₂OMe | Me | Me |
| 2-341 | c-Pr | Cl | 1 | CH₂CH₂OMe | Me | Me |
| 2-342 | c-Pr | Cl | 2 | CH₂CH₂OMe | Me | Me |
| 2-343 | c-Pr | Me | 0 | CH₂CH₂OMe | Me | Cl |
| 2-344 | c-Pr | Me | 1 | CH₂CH₂OMe | Me | Cl |
| 2-345 | c-Pr | Me | 2 | CH₂CH₂OMe | Me | Cl |
| 2-346 | c-Pr | Cl | 0 | CH₂CH₂OMe | Me | Cl |
| 2-347 | c-Pr | Cl | 1 | CH₂CH₂OMe | Me | Cl |
| 2-348 | c-Pr | Cl | 2 | CH₂CH₂OMe | Me | Cl |
| 2-349 | c-Pr | Me | 0 | CH₂CH₂OMe | Me | CF₃ |
| 2-350 | c-Pr | Me | 1 | CH₂CH₂OMe | Me | CF₃ |
| 2-351 | c-Pr | Me | 2 | CH₂CH₂OMe | Me | CF₃ |
| 2-352 | c-Pr | Cl | 0 | CH₂CH₂OMe | Me | CF₃ |
| 2-353 | c-Pr | Cl | 1 | CH₂CH₂OMe | Me | CF₃ |
| 2-354 | c-Pr | Cl | 2 | CH₂CH₂OMe | Me | CF₃ |
| 2-355 | c-Pr | Me | 0 | CH₂CH₂OMe | Me | SO₂Me |
| 2-356 | c-Pr | Me | 1 | CH₂CH₂OMe | Me | SO₂Me |
| 2-357 | c-Pr | Me | 2 | CH₂CH₂OMe | Me | SO₂Me |
| 2-358 | c-Pr | Cl | 0 | CH₂CH₂OMe | Me | SO₂Me |
| 2-359 | c-Pr | Cl | 1 | CH₂CH₂OMe | Me | SO₂Me |
| 2-360 | c-Pr | Cl | 2 | CH₂CH₂OMe | Me | SO₂Me |
| 2-361 | Cl | Me | 0 | CH₂CH₂OMe | H | H |
| 2-362 | Cl | Me | 1 | CH₂CH₂OMe | H | H |
| 2-363 | Cl | Me | 2 | CH₂CH₂OMe | H | H |
| 2-364 | Cl | Cl | 0 | CH₂CH₂OMe | H | H |
| 2-365 | Cl | Cl | 1 | CH₂CH₂OMe | H | H |
| 2-366 | Cl | Cl | 2 | CH₂CH₂OMe | H | H |
| 2-367 | Cl | Me | 0 | CH₂CH₂OMe | H | Me |
| 2-368 | Cl | Me | 1 | CH₂CH₂OMe | H | Me |
| 2-369 | Cl | Me | 2 | CH₂CH₂OMe | H | Me |
| 2-370 | Cl | Cl | 0 | CH₂CH₂OMe | H | Me |
| 2-371 | Cl | Cl | 1 | CH₂CH₂OMe | H | Me |
| 2-372 | Cl | Cl | 2 | CH₂CH₂OMe | H | Me |
| 2-373 | Cl | Me | 0 | CH₂CH₂OMe | H | Cl |
| 2-374 | Cl | Me | 1 | CH₂CH₂OMe | H | Cl |
| 2-375 | Cl | Me | 2 | CH₂CH₂OMe | H | Cl |
| 2-376 | Cl | Cl | 0 | CH₂CH₂OMe | H | Cl |
| 2-377 | Cl | Cl | 1 | CH₂CH₂OMe | H | Cl |
| 2-378 | Cl | Cl | 2 | CH₂CH₂OMe | H | Cl |
| 2-379 | Cl | Me | 0 | CH₂CH₂OMe | H | CF₃ |
| 2-380 | Cl | Me | 1 | CH₂CH₂OMe | H | CF₃ |
| 2-381 | Cl | Me | 2 | CH₂CH₂OMe | H | CF₃ |
| 2-382 | Cl | Cl | 0 | CH₂CH₂OMe | H | CF₃ |
| 2-383 | Cl | Cl | 1 | CH₂CH₂OMe | H | CF₃ |
| 2-384 | Cl | Cl | 2 | CH₂CH₂OMe | H | CF₃ |
| 2-385 | Cl | Me | 0 | CH₂CH₂OMe | H | SO₂Me |
| 2-386 | Cl | Me | 1 | CH₂CH₂OMe | H | SO₂Me |
| 2-387 | Cl | Me | 2 | CH₂CH₂OMe | H | SO₂Me |
| 2-388 | Cl | Cl | 0 | CH₂CH₂OMe | H | SO₂Me |
| 2-389 | Cl | Cl | 1 | CH₂CH₂OMe | H | SO₂Me |
| 2-390 | Cl | Cl | 2 | CH₂CH₂OMe | H | SO₂Me |
| 2-391 | Cl | Me | 0 | CH₂CH₂OMe | Me | H |
| 2-392 | Cl | Me | 1 | CH₂CH₂OMe | Me | H |
| 2-393 | Cl | Me | 2 | CH₂CH₂OMe | Me | H |
| 2-394 | Cl | Cl | 0 | CH₂CH₂OMe | Me | H |
| 2-395 | Cl | Cl | 1 | CH₂CH₂OMe | Me | H |
| 2-396 | Cl | Cl | 2 | CH₂CH₂OMe | Me | H |
| 2-397 | Cl | Me | 0 | CH₂CH₂OMe | Me | Me |
| 2-398 | Cl | Me | 1 | CH₂CH₂OMe | Me | Me |
| 2-399 | Cl | Me | 2 | CH₂CH₂OMe | Me | Me |
| 2-400 | Cl | Cl | 0 | CH₂CH₂OMe | Me | Me |
| 2-401 | Cl | Cl | 1 | CH₂CH₂OMe | Me | Me |
| 2-402 | Cl | Cl | 2 | CH₂CH₂OMe | Me | Me |
| 2-403 | Cl | Me | 0 | CH₂CH₂OMe | Me | Cl |
| 2-404 | Cl | Me | 1 | CH₂CH₂OMe | Me | Cl |
| 2-405 | Cl | Me | 2 | CH₂CH₂OMe | Me | Cl |
| 2-406 | Cl | Cl | 0 | CH₂CH₂OMe | Me | Cl |
| 2-407 | Cl | Cl | 1 | CH₂CH₂OMe | Me | Cl |
| 2-408 | Cl | Cl | 2 | CH₂CH₂OMe | Me | Cl |
| 2-409 | Cl | Me | 0 | CH₂CH₂OMe | Me | CF₃ |
| 2-410 | Cl | Me | 1 | CH₂CH₂OMe | Me | CF₃ |
| 2-411 | Cl | Me | 2 | CH₂CH₂OMe | Me | CF₃ |
| 2-412 | Cl | Cl | 0 | CH₂CH₂OMe | Me | CF₃ |
| 2-413 | Cl | Cl | 1 | CH₂CH₂OMe | Me | CF₃ |
| 2-414 | Cl | Cl | 2 | CH₂CH₂OMe | Me | CF₃ |
| 2-415 | Cl | Me | 0 | CH₂CH₂OMe | Me | SO₂Me |
| 2-416 | Cl | Me | 1 | CH₂CH₂OMe | Me | SO₂Me |
| 2-417 | Cl | Me | 2 | CH₂CH₂OMe | Me | SO₂Me |
| 2-418 | Cl | Cl | 0 | CH₂CH₂OMe | Me | SO₂Me |
| 2-419 | Cl | Cl | 1 | CH₂CH₂OMe | Me | SO₂Me |
| 2-420 | Cl | Cl | 2 | CH₂CH₂OMe | Me | SO₂Me |
| 2-421 | SO₂Me | Me | 0 | CH₂CH₂OMe | H | H |
| 2-422 | SO₂Me | Me | 1 | CH₂CH₂OMe | H | H |
| 2-423 | SO₂Me | Me | 2 | CH₂CH₂OMe | H | H |
| 2-424 | SO₂Me | Cl | 0 | CH₂CH₂OMe | H | H |
| 2-425 | SO₂Me | Cl | 1 | CH₂CH₂OMe | H | H |
| 2-426 | SO₂Me | Cl | 2 | CH₂CH₂OMe | H | H |
| 2-427 | SO₂Me | Me | 0 | CH₂CH₂OMe | H | Me |
| 2-428 | SO₂Me | Me | 1 | CH₂CH₂OMe | H | Me |
| 2-429 | SO₂Me | Me | 2 | CH₂CH₂OMe | H | Me |
| 2-430 | SO₂Me | Cl | 0 | CH₂CH₂OMe | H | Me |
| 2-431 | SO₂Me | Cl | 1 | CH₂CH₂OMe | H | Me |
| 2-432 | SO₂Me | Cl | 2 | CH₂CH₂OMe | H | Me |
| 2-433 | SO₂Me | Me | 0 | CH₂CH₂OMe | H | Cl |
| 2-434 | SO₂Me | Me | 1 | CH₂CH₂OMe | H | Cl |
| 2-435 | SO₂Me | Me | 2 | CH₂CH₂OMe | H | Cl |
| 2-436 | SO₂Me | Cl | 0 | CH₂CH₂OMe | H | Cl |
| 2-437 | SO₂Me | Cl | 1 | CH₂CH₂OMe | H | Cl |
| 2-438 | SO₂Me | Cl | 2 | CH₂CH₂OMe | H | Cl |
| 2-439 | SO₂Me | Me | 0 | CH₂CH₂OMe | H | CF₃ |
| 2-440 | SO₂Me | Me | 1 | CH₂CH₂OMe | H | CF₃ |
| 2-441 | SO₂Me | Me | 2 | CH₂CH₂OMe | H | CF₃ |
| 2-442 | SO₂Me | Cl | 0 | CH₂CH₂OMe | H | CF₃ |
| 2-443 | SO₂Me | Cl | 1 | CH₂CH₂OMe | H | CF₃ |
| 2-444 | SO₂Me | Cl | 2 | CH₂CH₂OMe | H | CF₃ |
| 2-445 | SO₂Me | Me | 0 | CH₂CH₂OMe | H | SO₂Me |
| 2-446 | SO₂Me | Me | 1 | CH₂CH₂OMe | H | SO₂Me |
| 2-447 | SO₂Me | Me | 2 | CH₂CH₂OMe | H | SO₂Me |
| 2-448 | SO₂Me | Cl | 0 | CH₂CH₂OMe | H | SO₂Me |
| 2-449 | SO₂Me | Cl | 1 | CH₂CH₂OMe | H | SO₂Me |
| 2-450 | SO₂Me | Cl | 2 | CH₂CH₂OMe | H | SO₂Me |
| 2-451 | SO₂Me | Me | 0 | CH₂CH₂OMe | Me | H |
| 2-452 | SO₂Me | Me | 1 | CH₂CH₂OMe | Me | H |
| 2-453 | SO₂Me | Me | 2 | CH₂CH₂OMe | Me | H |
| 2-454 | SO₂Me | Cl | 0 | CH₂CH₂OMe | Me | H |
| 2-455 | SO₂Me | Cl | 1 | CH₂CH₂OMe | Me | H |
| 2-456 | SO₂Me | Cl | 2 | CH₂CH₂OMe | Me | H |
| 2-457 | SO₂Me | Me | 0 | CH₂CH₂OMe | Me | Me |
| 2-458 | SO₂Me | Me | 1 | CH₂CH₂OMe | Me | Me |
| 2-459 | SO₂Me | Me | 2 | CH₂CH₂OMe | Me | Me |
| 2-460 | SO₂Me | Cl | 0 | CH₂CH₂OMe | Me | Me |
| 2-461 | SO₂Me | Cl | 1 | CH₂CH₂OMe | Me | Me |
| 2-462 | SO₂Me | Cl | 2 | CH₂CH₂OMe | Me | Me |
| 2-463 | SO₂Me | Me | 0 | CH₂CH₂OMe | Me | Cl |
| 2-464 | SO₂Me | Me | 1 | CH₂CH₂OMe | Me | Cl |
| 2-465 | SO₂Me | Me | 2 | CH₂CH₂OMe | Me | Cl |
| 2-466 | SO₂Me | Cl | 0 | CH₂CH₂OMe | Me | Cl |
| 2-467 | SO₂Me | Cl | 1 | CH₂CH₂OMe | Me | Cl |
| 2-468 | SO₂Me | Cl | 2 | CH₂CH₂OMe | Me | Cl |
| 2-469 | SO₂Me | Me | 0 | CH₂CH₂OMe | Me | CF₃ |
| 2-470 | SO₂Me | Me | 1 | CH₂CH₂OMe | Me | CF₃ |
| 2-471 | SO₂Me | Me | 2 | CH₂CH₂OMe | Me | CF₃ |
| 2-472 | SO₂Me | Cl | 0 | CH₂CH₂OMe | Me | CF₃ |
| 2-473 | SO₂Me | Cl | 1 | CH₂CH₂OMe | Me | CF₃ |
| 2-474 | SO₂Me | Cl | 2 | CH₂CH₂OMe | Me | CF₃ |
| 2-475 | SO₂Me | Me | 0 | CH₂CH₂OMe | Me | SO₂Me |
| 2-476 | SO₂Me | Me | 1 | CH₂CH₂OMe | Me | SO₂Me |
| 2-477 | SO₂Me | Me | 2 | CH₂CH₂OMe | Me | SO₂Me |
| 2-478 | SO₂Me | Cl | 0 | CH₂CH₂OMe | Me | SO₂Me |
| 2-479 | SO₂Me | Cl | 1 | CH₂CH₂OMe | Me | SO₂Me |
| 2-480 | SO₂Me | Cl | 2 | CH₂CH₂OMe | Me | SO₂Me |

**Tabelle 3: Erfindungsgemäße Verbindungen der allgemeinen Formel (I), worin R² und R³ jeweils Wasserstoff bedeuten, und A für -CH₂CH₂-, X¹ für CH, X² für CR⁷ und X³ für CR⁸ stehen**

| | | | | | | |
|---|---|---|---|---|---|---|
| 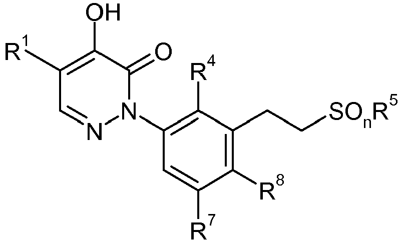 | | | | | | |

| **Nr.** | **R¹** | **R⁴** | **n** | **R⁵** | **R⁷** | **R⁸** |
|---|---|---|---|---|---|---|
| 3-1 | Me | Me | 0 | Me | H | H |
| 3-2 | Me | Me | 1 | Me | H | H |
| 3-3 | Me | Me | 2 | Me | H | H |
| 3-4 | Me | Cl | 0 | Me | H | H |
| 3-5 | Me | Cl | 1 | Me | H | H |
| 3-6 | Me | Cl | 2 | Me | H | H |
| 3-7 | Me | Me | 0 | Me | H | Me |
| 3-8 | Me | Me | 1 | Me | H | Me |
| 3-9 | Me | Me | 2 | Me | H | Me |
| 3-10 | Me | Cl | 0 | Me | H | Me |
| 3-11 | Me | Cl | 1 | Me | H | Me |
| 3-12 | Me | Cl | 2 | Me | H | Me |
| 3-13 | Me | Me | 0 | Me | H | Cl |
| 3-14 | Me | Me | 1 | Me | H | Cl |
| 3-15 | Me | Me | 2 | Me | H | Cl |
| 3-16 | Me | Cl | 0 | Me | H | Cl |
| 3-17 | Me | Cl | 1 | Me | H | Cl |
| 3-18 | Me | Cl | 2 | Me | H | Cl |
| 3-19 | Me | Me | 0 | Me | H | CF₃ |
| 3-20 | Me | Me | 1 | Me | H | CF₃ |
| 3-21 | Me | Me | 2 | Me | H | CF₃ |
| 3-22 | Me | Cl | 0 | Me | H | CF₃ |
| 3-23 | Me | Cl | 1 | Me | H | CF₃ |
| 3-24 | Me | Cl | 2 | Me | H | CF₃ |
| 3-25 | Me | Me | 0 | Me | H | SO₂Me |
| 3-26 | Me | Me | 1 | Me | H | SO₂Me |
| 3-27 | Me | Me | 2 | Me | H | SO₂Me |
| 3-28 | Me | Cl | 0 | Me | H | SO₂Me |
| 3-29 | Me | Cl | 1 | Me | H | SO₂Me |
| 3-30 | Me | Cl | 2 | Me | H | SO₂Me |
| 3-31 | Me | Me | 0 | Me | Me | H |
| 3-32 | Me | Me | 1 | Me | Me | H |
| 3-33 | Me | Me | 2 | Me | Me | H |
| 3-34 | Me | Cl | 0 | Me | Me | H |
| 3-35 | Me | Cl | 1 | Me | Me | H |
| 3-36 | Me | Cl | 2 | Me | Me | H |
| 3-37 | Me | Me | 0 | Me | Me | Me |
| 3-38 | Me | Me | 1 | Me | Me | Me |
| 3-39 | Me | Me | 2 | Me | Me | Me |
| 3-40 | Me | Cl | 0 | Me | Me | Me |
| 3-41 | Me | Cl | 1 | Me | Me | Me |
| 3-42 | Me | Cl | 2 | Me | Me | Me |
| 3-43 | Me | Me | 0 | Me | Me | Cl |
| 3-44 | Me | Me | 1 | Me | Me | Cl |
| 3-45 | Me | Me | 2 | Me | Me | Cl |
| 3-46 | Me | Cl | 0 | Me | Me | Cl |
| 3-47 | Me | Cl | 1 | Me | Me | Cl |
| 3-48 | Me | Cl | 2 | Me | Me | Cl |
| 3-49 | Me | Me | 0 | Me | Me | CF₃ |
| 3-50 | Me | Me | 1 | Me | Me | CF₃ |
| 3-51 | Me | Me | 2 | Me | Me | CF₃ |
| 3-52 | Me | Cl | 0 | Me | Me | CF₃ |
| 3-53 | Me | Cl | 1 | Me | Me | CF₃ |
| 3-54 | Me | Cl | 2 | Me | Me | CF₃ |
| 3-55 | Me | Me | 0 | Me | Me | SO₂Me |
| 3-56 | Me | Me | 1 | Me | Me | SO₂Me |
| 3-57 | Me | Me | 2 | Me | Me | SO₂Me |
| 3-58 | Me | Cl | 0 | Me | Me | SO₂Me |
| 3-59 | Me | Cl | 1 | Me | Me | SO₂Me |
| 3-60 | Me | Cl | 2 | Me | Me | SO₂Me |
| 3-61 | c-Pr | Me | 0 | Me | H | H |
| 3-62 | c-Pr | Me | 1 | Me | H | H |
| 3-63 | c-Pr | Me | 2 | Me | H | H |
| 3-64 | c-Pr | Cl | 0 | Me | H | H |
| 3-65 | c-Pr | Cl | 1 | Me | H | H |
| 3-66 | c-Pr | Cl | 2 | Me | H | H |
| 3-67 | c-Pr | Me | 0 | Me | H | Me |
| 3-68 | c-Pr | Me | 1 | Me | H | Me |
| 3-69 | c-Pr | Me | 2 | Me | H | Me |
| 3-70 | c-Pr | Cl | 0 | Me | H | Me |
| 3-71 | c-Pr | Cl | 1 | Me | H | Me |
| 3-72 | c-Pr | Cl | 2 | Me | H | Me |
| 3-73 | c-Pr | Me | 0 | Me | H | Cl |
| 3-74 | c-Pr | Me | 1 | Me | H | Cl |
| 3-75 | c-Pr | Me | 2 | Me | H | Cl |
| 3-76 | c-Pr | Cl | 0 | Me | H | Cl |
| 3-77 | c-Pr | Cl | 1 | Me | H | Cl |
| 3-78 | c-Pr | Cl | 2 | Me | H | Cl |
| 3-79 | c-Pr | Me | 0 | Me | H | CF₃ |
| 3-80 | c-Pr | Me | 1 | Me | H | CF₃ |
| 3-81 | c-Pr | Me | 2 | Me | H | CF₃ |
| 3-82 | c-Pr | Cl | 0 | Me | H | CF₃ |
| 3-83 | c-Pr | Cl | 1 | Me | H | CF₃ |
| 3-84 | c-Pr | Cl | 2 | Me | H | CF₃ |
| 3-85 | c-Pr | Me | 0 | Me | H | SO₂Me |
| 3-86 | c-Pr | Me | 1 | Me | H | SO₂Me |
| 3-87 | c-Pr | Me | 2 | Me | H | SO₂Me |
| 3-88 | c-Pr | Cl | 0 | Me | H | SO₂Me |
| 3-89 | c-Pr | Cl | 1 | Me | H | SO₂Me |
| 3-90 | c-Pr | Cl | 2 | Me | H | SO₂Me |
| 3-91 | c-Pr | Me | 0 | Me | Me | H |
| 3-92 | c-Pr | Me | 1 | Me | Me | H |
| 3-93 | c-Pr | Me | 2 | Me | Me | H |
| 3-94 | c-Pr | Cl | 0 | Me | Me | H |
| 3-95 | c-Pr | Cl | 1 | Me | Me | H |
| 3-96 | c-Pr | Cl | 2 | Me | Me | H |
| 3-97 | c-Pr | Me | 0 | Me | Me | Me |
| 3-98 | c-Pr | Me | 1 | Me | Me | Me |
| 3-99 | c-Pr | Me | 2 | Me | Me | Me |
| 3-100 | c-Pr | Cl | 0 | Me | Me | Me |
| 3-101 | c-Pr | Cl | 1 | Me | Me | Me |
| 3-102 | c-Pr | Cl | 2 | Me | Me | Me |
| 3-103 | c-Pr | Me | 0 | Me | Me | Cl |
| 3-104 | c-Pr | Me | 1 | Me | Me | Cl |
| 3-105 | c-Pr | Me | 2 | Me | Me | Cl |
| 3-106 | c-Pr | Cl | 0 | Me | Me | Cl |
| 3-107 | c-Pr | Cl | 1 | Me | Me | Cl |
| 3-108 | c-Pr | Cl | 2 | Me | Me | Cl |
| 3-109 | c-Pr | Me | 0 | Me | Me | CF₃ |
| 3-110 | c-Pr | Me | 1 | Me | Me | CF₃ |
| 3-111 | c-Pr | Me | 2 | Me | Me | CF₃ |
| 3-112 | c-Pr | Cl | 0 | Me | Me | CF₃ |
| 3-113 | c-Pr | Cl | 1 | Me | Me | CF₃ |
| 3-114 | c-Pr | Cl | 2 | Me | Me | CF₃ |
| 3-115 | c-Pr | Me | 0 | Me | Me | SO₂Me |
| 3-116 | c-Pr | Me | 1 | Me | Me | SO₂Me |
| 3-117 | c-Pr | Me | 2 | Me | Me | SO₂Me |
| 3-118 | c-Pr | Cl | 0 | Me | Me | SO₂Me |
| 3-119 | c-Pr | Cl | 1 | Me | Me | SO₂Me |
| 3-120 | c-Pr | Cl | 2 | Me | Me | SO₂Me |
| 3-121 | Cl | Me | 0 | Me | H | H |
| 3-122 | Cl | Me | 1 | Me | H | H |
| 3-123 | Cl | Me | 2 | Me | H | H |
| 3-124 | Cl | Cl | 0 | Me | H | H |
| 3-125 | Cl | Cl | 1 | Me | H | H |
| 3-126 | Cl | Cl | 2 | Me | H | H |
| 3-127 | Cl | Me | 0 | Me | H | Me |
| 3-128 | Cl | Me | 1 | Me | H | Me |
| 3-129 | Cl | Me | 2 | Me | H | Me |
| 3-130 | Cl | Cl | 0 | Me | H | Me |
| 3-131 | Cl | Cl | 1 | Me | H | Me |
| 3-132 | Cl | Cl | 2 | Me | H | Me |
| 3-133 | Cl | Me | 0 | Me | H | Cl |
| 3-134 | Cl | Me | 1 | Me | H | Cl |
| 3-135 | Cl | Me | 2 | Me | H | Cl |
| 3-136 | Cl | Cl | 0 | Me | H | Cl |
| 3-137 | Cl | Cl | 1 | Me | H | Cl |
| 3-138 | Cl | Cl | 2 | Me | H | Cl |
| 3-139 | Cl | Me | 0 | Me | H | CF₃ |
| 3-140 | Cl | Me | 1 | Me | H | CF₃ |
| 3-141 | Cl | Me | 2 | Me | H | CF₃ |
| 3-142 | Cl | Cl | 0 | Me | H | CF₃ |
| 3-143 | Cl | Cl | 1 | Me | H | CF₃ |
| 3-144 | Cl | Cl | 2 | Me | H | CF₃ |
| 3-145 | Cl | Me | 0 | Me | H | SO₂Me |
| 3-146 | Cl | Me | 1 | Me | H | SO₂Me |
| 3-147 | Cl | Me | 2 | Me | H | SO₂Me |
| 3-148 | Cl | Cl | 0 | Me | H | SO₂Me |
| 3-149 | Cl | Cl | 1 | Me | H | SO₂Me |
| 3-150 | Cl | Cl | 2 | Me | H | SO₂Me |
| 3-151 | Cl | Me | 0 | Me | Me | H |
| 3-152 | Cl | Me | 1 | Me | Me | H |
| 3-153 | Cl | Me | 2 | Me | Me | H |
| 3-154 | Cl | Cl | 0 | Me | Me | H |
| 3-155 | Cl | Cl | 1 | Me | Me | H |
| 3-156 | Cl | Cl | 2 | Me | Me | H |
| 3-157 | Cl | Me | 0 | Me | Me | Me |
| 3-158 | Cl | Me | 1 | Me | Me | Me |
| 3-159 | Cl | Me | 2 | Me | Me | Me |
| 3-160 | Cl | Cl | 0 | Me | Me | Me |
| 3-161 | Cl | Cl | 1 | Me | Me | Me |
| 3-162 | Cl | Cl | 2 | Me | Me | Me |
| 3-163 | Cl | Me | 0 | Me | Me | Cl |
| 3-164 | Cl | Me | 1 | Me | Me | Cl |
| 3-165 | Cl | Me | 2 | Me | Me | Cl |
| 3-166 | Cl | Cl | 0 | Me | Me | Cl |
| 3-167 | Cl | Cl | 1 | Me | Me | Cl |
| 3-168 | Cl | Cl | 2 | Me | Me | Cl |
| 3-169 | Cl | Me | 0 | Me | Me | CF₃ |
| 3-170 | Cl | Me | 1 | Me | Me | CF₃ |
| 3-171 | Cl | Me | 2 | Me | Me | CF₃ |
| 3-172 | Cl | Cl | 0 | Me | Me | CF₃ |
| 3-173 | Cl | Cl | 1 | Me | Me | CF₃ |
| 3-174 | Cl | Cl | 2 | Me | Me | CF₃ |
| 3-175 | Cl | Me | 0 | Me | Me | SO₂Me |
| 3-176 | Cl | Me | 1 | Me | Me | SO₂Me |
| 3-177 | Cl | Me | 2 | Me | Me | SO₂Me |
| 3-178 | Cl | Cl | 0 | Me | Me | SO₂Me |
| 3-179 | Cl | Cl | 1 | Me | Me | SO₂Me |
| 3-180 | Cl | Cl | 2 | Me | Me | SO₂Me |
| 3-181 | SO₂Me | Me | 0 | Me | H | H |
| 3-182 | SO₂Me | Me | 1 | Me | H | H |
| 3-183 | SO₂Me | Me | 2 | Me | H | H |
| 3-184 | SO₂Me | Cl | 0 | Me | H | H |
| 3-185 | SO₂Me | Cl | 1 | Me | H | H |
| 3-186 | SO₂Me | Cl | 2 | Me | H | H |
| 3-187 | SO₂Me | Me | 0 | Me | H | Me |
| 3-188 | SO₂Me | Me | 1 | Me | H | Me |
| 3-189 | SO₂Me | Me | 2 | Me | H | Me |
| 3-190 | SO₂Me | Cl | 0 | Me | H | Me |
| 3-191 | SO₂Me | Cl | 1 | Me | H | Me |
| 3-192 | SO₂Me | Cl | 2 | Me | H | Me |
| 3-193 | SO₂Me | Me | 0 | Me | H | Cl |
| 3-194 | SO₂Me | Me | 1 | Me | H | Cl |
| 3-195 | SO₂Me | Me | 2 | Me | H | Cl |
| 3-196 | SO₂Me | Cl | 0 | Me | H | Cl |
| 3-197 | SO₂Me | Cl | 1 | Me | H | Cl |
| 3-198 | SO₂Me | Cl | 2 | Me | H | Cl |
| 3-199 | SO₂Me | Me | 0 | Me | H | CF₃ |
| 3-200 | SO₂Me | Me | 1 | Me | H | CF₃ |
| 3-201 | SO₂Me | Me | 2 | Me | H | CF₃ |
| 3-202 | SO₂Me | Cl | 0 | Me | H | CF₃ |
| 3-203 | SO₂Me | Cl | 1 | Me | H | CF₃ |
| 3-204 | SO₂Me | Cl | 2 | Me | H | CF₃ |
| 3-205 | SO₂Me | Me | 0 | Me | H | SO₂Me |
| 3-206 | SO₂Me | Me | 1 | Me | H | SO₂Me |
| 3-207 | SO₂Me | Me | 2 | Me | H | SO₂Me |
| 3-208 | SO₂Me | Cl | 0 | Me | H | SO₂Me |
| 3-209 | SO₂Me | Cl | 1 | Me | H | SO₂Me |
| 3-210 | SO₂Me | Cl | 2 | Me | H | SO₂Me |
| 3-211 | SO₂Me | Me | 0 | Me | Me | H |
| 3-212 | SO₂Me | Me | 1 | Me | Me | H |
| 3-213 | SO₂Me | Me | 2 | Me | Me | H |
| 3-214 | SO₂Me | Cl | 0 | Me | Me | H |
| 3-215 | SO₂Me | Cl | 1 | Me | Me | H |
| 3-216 | SO₂Me | Cl | 2 | Me | Me | H |
| 3-217 | SO₂Me | Me | 0 | Me | Me | Me |
| 3-218 | SO₂Me | Me | 1 | Me | Me | Me |
| 3-219 | SO₂Me | Me | 2 | Me | Me | Me |
| 3-220 | SO₂Me | Cl | 0 | Me | Me | Me |
| 3-221 | SO₂Me | Cl | 1 | Me | Me | Me |
| 3-222 | SO₂Me | Cl | 2 | Me | Me | Me |
| 3-223 | SO₂Me | Me | 0 | Me | Me | Cl |
| 3-224 | SO₂Me | Me | 1 | Me | Me | Cl |
| 3-225 | SO₂Me | Me | 2 | Me | Me | Cl |
| 3-226 | SO₂Me | Cl | 0 | Me | Me | Cl |
| 3-227 | SO₂Me | Cl | 1 | Me | Me | Cl |
| 3-228 | SO₂Me | Cl | 2 | Me | Me | Cl |
| 3-229 | SO₂Me | Me | 0 | Me | Me | CF₃ |
| 3-230 | SO₂Me | Me | 1 | Me | Me | CF₃ |
| 3-231 | SO₂Me | Me | 2 | Me | Me | CF₃ |
| 3-232 | SO₂Me | Cl | 0 | Me | Me | CF₃ |
| 3-233 | SO₂Me | Cl | 1 | Me | Me | CF₃ |
| 3-234 | SO₂Me | Cl | 2 | Me | Me | CF₃ |
| 3-235 | SO₂Me | Me | 0 | Me | Me | SO₂Me |
| 3-236 | SO₂Me | Me | 1 | Me | Me | SO₂Me |
| 3-237 | SO₂Me | Me | 2 | Me | Me | SO₂Me |
| 3-238 | SO₂Me | Cl | 0 | Me | Me | SO₂Me |
| 3-239 | SO₂Me | Cl | 1 | Me | Me | SO₂Me |
| 3-240 | SO₂Me | Cl | 2 | Me | Me | SO₂Me |
| 3-241 | Me | Me | 0 | CH₂CH₂OMe | H | H |
| 3-242 | Me | Me | 1 | CH₂CH₂OMe | H | H |
| 3-243 | Me | Me | 2 | CH₂CH₂OMe | H | H |
| 3-244 | Me | Cl | 0 | CH₂CH₂OMe | H | H |
| 3-245 | Me | Cl | 1 | CH₂CH₂OMe | H | H |
| 3-246 | Me | Cl | 2 | CH₂CH₂OMe | H | H |
| 3-247 | Me | Me | 0 | CH₂CH₂OMe | H | Me |
| 3-248 | Me | Me | 1 | CH₂CH₂OMe | H | Me |
| 3-249 | Me | Me | 2 | CH₂CH₂OMe | H | Me |
| 3-250 | Me | Cl | 0 | CH₂CH₂OMe | H | Me |
| 3-251 | Me | Cl | 1 | CH₂CH₂OMe | H | Me |
| 3-252 | Me | Cl | 2 | CH₂CH₂OMe | H | Me |
| 3-253 | Me | Me | 0 | CH₂CH₂OMe | H | Cl |
| 3-254 | Me | Me | 1 | CH₂CH₂OMe | H | Cl |
| 3-255 | Me | Me | 2 | CH₂CH₂OMe | H | Cl |
| 3-256 | Me | Cl | 0 | CH₂CH₂OMe | H | Cl |
| 3-257 | Me | Cl | 1 | CH₂CH₂OMe | H | Cl |
| 3-258 | Me | Cl | 2 | CH₂CH₂OMe | H | Cl |
| 3-259 | Me | Me | 0 | CH₂CH₂OMe | H | CF₃ |
| 3-260 | Me | Me | 1 | CH₂CH₂OMe | H | CF₃ |
| 3-261 | Me | Me | 2 | CH₂CH₂OMe | H | CF₃ |
| 3-262 | Me | Cl | 0 | CH₂CH₂OMe | H | CF₃ |
| 3-263 | Me | Cl | 1 | CH₂CH₂OMe | H | CF₃ |
| 3-264 | Me | Cl | 2 | CH₂CH₂OMe | H | CF₃ |
| 3-265 | Me | Me | 0 | CH₂CH₂OMe | H | SO₂Me |
| 3-266 | Me | Me | 1 | CH₂CH₂OMe | H | SO₂Me |
| 3-267 | Me | Me | 2 | CH₂CH₂OMe | H | SO₂Me |
| 3-268 | Me | Cl | 0 | CH₂CH₂OMe | H | SO₂Me |
| 3-269 | Me | Cl | 1 | CH₂CH₂OMe | H | SO₂Me |
| 3-270 | Me | Cl | 2 | CH₂CH₂OMe | H | SO₂Me |
| 3-271 | Me | Me | 0 | CH₂CH₂OMe | Me | H |
| 3-272 | Me | Me | 1 | CH₂CH₂OMe | Me | H |
| 3-273 | Me | Me | 2 | CH₂CH₂OMe | Me | H |
| 3-274 | Me | Cl | 0 | CH₂CH₂OMe | Me | H |
| 3-275 | Me | Cl | 1 | CH₂CH₂OMe | Me | H |
| 3-276 | Me | Cl | 2 | CH₂CH₂OMe | Me | H |
| 3-277 | Me | Me | 0 | CH₂CH₂OMe | Me | Me |
| 3-278 | Me | Me | 1 | CH₂CH₂OMe | Me | Me |
| 3-279 | Me | Me | 2 | CH₂CH₂OMe | Me | Me |
| 3-280 | Me | Cl | 0 | CH₂CH₂OMe | Me | Me |
| 3-281 | Me | Cl | 1 | CH₂CH₂OMe | Me | Me |
| 3-282 | Me | Cl | 2 | CH₂CH₂OMe | Me | Me |
| 3-283 | Me | Me | 0 | CH₂CH₂OMe | Me | Cl |
| 3-284 | Me | Me | 1 | CH₂CH₂OMe | Me | Cl |
| 3-285 | Me | Me | 2 | CH₂CH₂OMe | Me | Cl |
| 3-286 | Me | Cl | 0 | CH₂CH₂OMe | Me | Cl |
| 3-287 | Me | Cl | 1 | CH₂CH₂OMe | Me | Cl |
| 3-288 | Me | Cl | 2 | CH₂CH₂OMe | Me | Cl |
| 3-289 | Me | Me | 0 | CH₂CH₂OMe | Me | CF₃ |
| 3-290 | Me | Me | 1 | CH₂CH₂OMe | Me | CF₃ |
| 3-291 | Me | Me | 2 | CH₂CH₂OMe | Me | CF₃ |
| 3-292 | Me | Cl | 0 | CH₂CH₂OMe | Me | CF₃ |
| 3-293 | Me | Cl | 1 | CH₂CH₂OMe | Me | CF₃ |
| 3-294 | Me | Cl | 2 | CH₂CH₂OMe | Me | CF₃ |
| 3-295 | Me | Me | 0 | CH₂CH₂OMe | Me | SO₂Me |
| 3-296 | Me | Me | 1 | CH₂CH₂OMe | Me | SO₂Me |
| 3-297 | Me | Me | 2 | CH₂CH₂OMe | Me | SO₂Me |
| 3-298 | Me | Cl | 0 | CH₂CH₂OMe | Me | SO₂Me |
| 3-299 | Me | Cl | 1 | CH₂CH₂OMe | Me | SO₂Me |
| 3-300 | Me | Cl | 2 | CH₂CH₂OMe | Me | SO₂Me |
| 3-301 | c-Pr | Me | 0 | CH₂CH₂OMe | H | H |
| 3-302 | c-Pr | Me | 1 | CH₂CH₂OMe | H | H |
| 3-303 | c-Pr | Me | 2 | CH₂CH₂OMe | H | H |
| 3-304 | c-Pr | Cl | 0 | CH₂CH₂OMe | H | H |
| 3-305 | c-Pr | Cl | 1 | CH₂CH₂OMe | H | H |
| 3-306 | c-Pr | Cl | 2 | CH₂CH₂OMe | H | H |
| 3-307 | c-Pr | Me | 0 | CH₂CH₂OMe | H | Me |
| 3-308 | c-Pr | Me | 1 | CH₂CH₂OMe | H | Me |
| 3-309 | c-Pr | Me | 2 | CH₂CH₂OMe | H | Me |
| 3-310 | c-Pr | Cl | 0 | CH₂CH₂OMe | H | Me |
| 3-311 | c-Pr | Cl | 1 | CH₂CH₂OMe | H | Me |
| 3-312 | c-Pr | Cl | 2 | CH₂CH₂OMe | H | Me |
| 3-313 | c-Pr | Me | 0 | CH₂CH₂OMe | H | Cl |
| 3-314 | c-Pr | Me | 1 | CH₂CH₂OMe | H | Cl |
| 3-315 | c-Pr | Me | 2 | CH₂CH₂OMe | H | Cl |
| 3-316 | c-Pr | Cl | 0 | CH₂CH₂OMe | H | Cl |
| 3-317 | c-Pr | Cl | 1 | CH₂CH₂OMe | H | Cl |
| 3-318 | c-Pr | Cl | 2 | CH₂CH₂OMe | H | Cl |
| 3-319 | c-Pr | Me | 0 | CH₂CH₂OMe | H | CF₃ |
| 3-320 | c-Pr | Me | 1 | CH₂CH₂OMe | H | CF₃ |
| 3-321 | c-Pr | Me | 2 | CH₂CH₂OMe | H | CF₃ |
| 3-322 | c-Pr | Cl | 0 | CH₂CH₂OMe | H | CF₃ |
| 3-323 | c-Pr | Cl | 1 | CH₂CH₂OMe | H | CF₃ |
| 3-324 | c-Pr | Cl | 2 | CH₂CH₂OMe | H | CF₃ |
| 3-325 | c-Pr | Me | 0 | CH₂CH₂OMe | H | SO₂Me |
| 3-326 | c-Pr | Me | 1 | CH₂CH₂OMe | H | SO₂Me |
| 3-327 | c-Pr | Me | 2 | CH₂CH₂OMe | H | SO₂Me |
| 3-328 | c-Pr | Cl | 0 | CH₂CH₂OMe | H | SO₂Me |
| 3-329 | c-Pr | Cl | 1 | CH₂CH₂OMe | H | SO₂Me |
| 3-330 | c-Pr | Cl | 2 | CH₂CH₂OMe | H | SO₂Me |
| 3-331 | c-Pr | Me | 0 | CH₂CH₂OMe | Me | H |
| 3-332 | c-Pr | Me | 1 | CH₂CH₂OMe | Me | H |
| 3-333 | c-Pr | Me | 2 | CH₂CH₂OMe | Me | H |
| 3-334 | c-Pr | Cl | 0 | CH₂CH₂OMe | Me | H |
| 3-335 | c-Pr | Cl | 1 | CH₂CH₂OMe | Me | H |
| 3-336 | c-Pr | Cl | 2 | CH₂CH₂OMe | Me | H |
| 3-337 | c-Pr | Me | 0 | CH₂CH₂OMe | Me | Me |
| 3-338 | c-Pr | Me | 1 | CH₂CH₂OMe | Me | Me |
| 3-339 | c-Pr | Me | 2 | CH₂CH₂OMe | Me | Me |
| 3-340 | c-Pr | Cl | 0 | CH₂CH₂OMe | Me | Me |
| 3-341 | c-Pr | Cl | 1 | CH₂CH₂OMe | Me | Me |
| 3-342 | c-Pr | Cl | 2 | CH₂CH₂OMe | Me | Me |
| 3-343 | c-Pr | Me | 0 | CH₂CH₂OMe | Me | Cl |
| 3-344 | c-Pr | Me | 1 | CH₂CH₂OMe | Me | Cl |
| 3-345 | c-Pr | Me | 2 | CH₂CH₂OMe | Me | Cl |
| 3-346 | c-Pr | Cl | 0 | CH₂CH₂OMe | Me | Cl |
| 3-347 | c-Pr | Cl | 1 | CH₂CH₂OMe | Me | Cl |
| 3-348 | c-Pr | Cl | 2 | CH₂CH₂OMe | Me | Cl |
| 3-349 | c-Pr | Me | 0 | CH₂CH₂OMe | Me | CF₃ |
| 3-350 | c-Pr | Me | 1 | CH₂CH₂OMe | Me | CF₃ |
| 3-351 | c-Pr | Me | 2 | CH₂CH₂OMe | Me | CF₃ |
| 3-352 | c-Pr | Cl | 0 | CH₂CH₂OMe | Me | CF₃ |
| 3-353 | c-Pr | Cl | 1 | CH₂CH₂OMe | Me | CF₃ |
| 3-354 | c-Pr | Cl | 2 | CH₂CH₂OMe | Me | CF₃ |
| 3-355 | c-Pr | Me | 0 | CH₂CH₂OMe | Me | SO₂Me |
| 3-356 | c-Pr | Me | 1 | CH₂CH₂OMe | Me | SO₂Me |
| 3-357 | c-Pr | Me | 2 | CH₂CH₂OMe | Me | SO₂Me |
| 3-358 | c-Pr | Cl | 0 | CH₂CH₂OMe | Me | SO₂Me |
| 3-359 | c-Pr | Cl | 1 | CH₂CH₂OMe | Me | SO₂Me |
| 3-360 | c-Pr | Cl | 2 | CH₂CH₂OMe | Me | SO₂Me |
| 3-361 | Cl | Me | 0 | CH₂CH₂OMe | H | H |
| 3-362 | Cl | Me | 1 | CH₂CH₂OMe | H | H |
| 3-363 | Cl | Me | 2 | CH₂CH₂OMe | H | H |
| 3-364 | Cl | Cl | 0 | CH₂CH₂OMe | H | H |
| 3-365 | Cl | Cl | 1 | CH₂CH₂OMe | H | H |
| 3-366 | Cl | Cl | 2 | CH₂CH₂OMe | H | H |
| 3-367 | Cl | Me | 0 | CH₂CH₂OMe | H | Me |
| 3-368 | Cl | Me | 1 | CH₂CH₂OMe | H | Me |
| 3-369 | Cl | Me | 2 | CH₂CH₂OMe | H | Me |
| 3-370 | Cl | Cl | 0 | CH₂CH₂OMe | H | Me |
| 3-371 | Cl | Cl | 1 | CH₂CH₂OMe | H | Me |
| 3-372 | Cl | Cl | 2 | CH₂CH₂OMe | H | Me |
| 3-373 | Cl | Me | 0 | CH₂CH₂OMe | H | Cl |
| 3-374 | Cl | Me | 1 | CH₂CH₂OMe | H | Cl |
| 3-375 | Cl | Me | 2 | CH₂CH₂OMe | H | Cl |
| 3-376 | Cl | Cl | 0 | CH₂CH₂OMe | H | Cl |
| 3-377 | Cl | Cl | 1 | CH₂CH₂OMe | H | Cl |
| 3-378 | Cl | Cl | 2 | CH₂CH₂OMe | H | Cl |
| 3-379 | Cl | Me | 0 | CH₂CH₂OMe | H | CF₃ |
| 3-380 | Cl | Me | 1 | CH₂CH₂OMe | H | CF₃ |
| 3-381 | Cl | Me | 2 | CH₂CH₂OMe | H | CF₃ |
| 3-382 | Cl | Cl | 0 | CH₂CH₂OMe | H | CF₃ |
| 3-383 | Cl | Cl | 1 | CH₂CH₂OMe | H | CF₃ |
| 3-384 | Cl | Cl | 2 | CH₂CH₂OMe | H | CF₃ |
| 3-385 | Cl | Me | 0 | CH₂CH₂OMe | H | SO₂Me |
| 3-386 | Cl | Me | 1 | CH₂CH₂OMe | H | SO₂Me |
| 3-387 | Cl | Me | 2 | CH₂CH₂OMe | H | SO₂Me |
| 3-388 | Cl | Cl | 0 | CH₂CH₂OMe | H | SO₂Me |
| 3-389 | Cl | Cl | 1 | CH₂CH₂OMe | H | SO₂Me |
| 3-390 | Cl | Cl | 2 | CH₂CH₂OMe | H | SO₂Me |
| 3-391 | Cl | Me | 0 | CH₂CH₂OMe | Me | H |
| 3-392 | Cl | Me | 1 | CH₂CH₂OMe | Me | H |
| 3-393 | Cl | Me | 2 | CH₂CH₂OMe | Me | H |
| 3-394 | Cl | Cl | 0 | CH₂CH₂OMe | Me | H |
| 3-395 | Cl | Cl | 1 | CH₂CH₂OMe | Me | H |
| 3-396 | Cl | Cl | 2 | CH₂CH₂OMe | Me | H |
| 3-397 | Cl | Me | 0 | CH₂CH₂OMe | Me | Me |
| 3-398 | Cl | Me | 1 | CH₂CH₂OMe | Me | Me |
| 3-399 | Cl | Me | 2 | CH₂CH₂OMe | Me | Me |
| 3-400 | Cl | Cl | 0 | CH₂CH₂OMe | Me | Me |
| 3-401 | Cl | Cl | 1 | CH₂CH₂OMe | Me | Me |
| 3-402 | Cl | Cl | 2 | CH₂CH₂OMe | Me | Me |
| 3-403 | Cl | Me | 0 | CH₂CH₂OMe | Me | Cl |
| 3-404 | Cl | Me | 1 | CH₂CH₂OMe | Me | Cl |
| 3-405 | Cl | Me | 2 | CH₂CH₂OMe | Me | Cl |
| 3-406 | Cl | Cl | 0 | CH₂CH₂OMe | Me | Cl |
| 3-407 | Cl | Cl | 1 | CH₂CH₂OMe | Me | Cl |
| 3-408 | Cl | Cl | 2 | CH₂CH₂OMe | Me | Cl |
| 3-409 | Cl | Me | 0 | CH₂CH₂OMe | Me | CF₃ |
| 3-410 | Cl | Me | 1 | CH₂CH₂OMe | Me | CF₃ |
| 3-411 | Cl | Me | 2 | CH₂CH₂OMe | Me | CF₃ |
| 3-412 | Cl | Cl | 0 | CH₂CH₂OMe | Me | CF₃ |
| 3-413 | Cl | Cl | 1 | CH₂CH₂OMe | Me | CF₃ |
| 3-414 | Cl | Cl | 2 | CH₂CH₂OMe | Me | CF₃ |
| 3-415 | Cl | Me | 0 | CH₂CH₂OMe | Me | SO₂Me |
| 3-416 | Cl | Me | 1 | CH₂CH₂OMe | Me | SO₂Me |
| 3-417 | Cl | Me | 2 | CH₂CH₂OMe | Me | SO₂Me |
| 3-418 | Cl | Cl | 0 | CH₂CH₂OMe | Me | SO₂Me |
| 3-419 | Cl | Cl | 1 | CH₂CH₂OMe | Me | SO₂Me |
| 3-420 | Cl | Cl | 2 | CH₂CH₂OMe | Me | SO₂Me |
| 3-421 | SO₂Me | Me | 0 | CH₂CH₂OMe | H | H |
| 3-422 | SO₂Me | Me | 1 | CH₂CH₂OMe | H | H |
| 3-423 | SO₂Me | Me | 2 | CH₂CH₂OMe | H | H |
| 3-424 | SO₂Me | Cl | 0 | CH₂CH₂OMe | H | H |
| 3-425 | SO₂Me | Cl | 1 | CH₂CH₂OMe | H | H |
| 3-426 | SO₂Me | Cl | 2 | CH₂CH₂OMe | H | H |
| 3-427 | SO₂Me | Me | 0 | CH₂CH₂OMe | H | Me |
| 3-428 | SO₂Me | Me | 1 | CH₂CH₂OMe | H | Me |
| 3-429 | SO₂Me | Me | 2 | CH₂CH₂OMe | H | Me |
| 3-430 | SO₂Me | Cl | 0 | CH₂CH₂OMe | H | Me |
| 3-431 | SO₂Me | Cl | 1 | CH₂CH₂OMe | H | Me |
| 3-432 | SO₂Me | Cl | 2 | CH₂CH₂OMe | H | Me |
| 3-433 | SO₂Me | Me | 0 | CH₂CH₂OMe | H | Cl |
| 3-434 | SO₂Me | Me | 1 | CH₂CH₂OMe | H | Cl |
| 3-435 | SO₂Me | Me | 2 | CH₂CH₂OMe | H | Cl |
| 3-436 | SO₂Me | Cl | 0 | CH₂CH₂OMe | H | Cl |
| 3-437 | SO₂Me | Cl | 1 | CH₂CH₂OMe | H | Cl |
| 3-438 | SO₂Me | Cl | 2 | CH₂CH₂OMe | H | Cl |
| 3-439 | SO₂Me | Me | 0 | CH₂CH₂OMe | H | CF₃ |
| 3-440 | SO₂Me | Me | 1 | CH₂CH₂OMe | H | CF₃ |
| 3-441 | SO₂Me | Me | 2 | CH₂CH₂OMe | H | CF₃ |
| 3-442 | SO₂Me | Cl | 0 | CH₂CH₂OMe | H | CF₃ |
| 3-443 | SO₂Me | Cl | 1 | CH₂CH₂OMe | H | CF₃ |
| 3-444 | SO₂Me | Cl | 2 | CH₂CH₂OMe | H | CF₃ |
| 3-445 | SO₂Me | Me | 0 | CH₂CH₂OMe | H | SO₂Me |
| 3-446 | SO₂Me | Me | 1 | CH₂CH₂OMe | H | SO₂Me |
| 3-447 | SO₂Me | Me | 2 | CH₂CH₂OMe | H | SO₂Me |
| 3-448 | SO₂Me | Cl | 0 | CH₂CH₂OMe | H | SO₂Me |
| 3-449 | SO₂Me | Cl | 1 | CH₂CH₂OMe | H | SO₂Me |
| 3-450 | SO₂Me | Cl | 2 | CH₂CH₂OMe | H | SO₂Me |
| 3-451 | SO₂Me | Me | 0 | CH₂CH₂OMe | Me | H |
| 3-452 | SO₂Me | Me | 1 | CH₂CH₂OMe | Me | H |
| 3-453 | SO₂Me | Me | 2 | CH₂CH₂OMe | Me | H |
| 3-454 | SO₂Me | Cl | 0 | CH₂CH₂OMe | Me | H |
| 3-455 | SO₂Me | Cl | 1 | CH₂CH₂OMe | Me | H |
| 3-456 | SO₂Me | Cl | 2 | CH₂CH₂OMe | Me | H |
| 3-457 | SO₂Me | Me | 0 | CH₂CH₂OMe | Me | Me |
| 3-458 | SO₂Me | Me | 1 | CH₂CH₂OMe | Me | Me |
| 3-459 | SO₂Me | Me | 2 | CH₂CH₂OMe | Me | Me |
| 3-460 | SO₂Me | Cl | 0 | CH₂CH₂OMe | Me | Me |
| 3-461 | SO₂Me | Cl | 1 | CH₂CH₂OMe | Me | Me |
| 3-462 | SO₂Me | Cl | 2 | CH₂CH₂OMe | Me | Me |
| 3-463 | SO₂Me | Me | 0 | CH₂CH₂OMe | Me | Cl |
| 3-464 | SO₂Me | Me | 1 | CH₂CH₂OMe | Me | Cl |
| 3-465 | SO₂Me | Me | 2 | CH₂CH₂OMe | Me | Cl |
| 3-466 | SO₂Me | Cl | 0 | CH₂CH₂OMe | Me | Cl |
| 3-467 | SO₂Me | Cl | 1 | CH₂CH₂OMe | Me | Cl |
| 3-468 | SO₂Me | Cl | 2 | CH₂CH₂OMe | Me | Cl |
| 3-469 | SO₂Me | Me | 0 | CH₂CH₂OMe | Me | CF₃ |
| 3-470 | SO₂Me | Me | 1 | CH₂CH₂OMe | Me | CF₃ |
| 3-471 | SO₂Me | Me | 2 | CH₂CH₂OMe | Me | CF₃ |
| 3-472 | SO₂Me | Cl | 0 | CH₂CH₂OMe | Me | CF₃ |
| 3-473 | SO₂Me | Cl | 1 | CH₂CH₂OMe | Me | CF₃ |
| 3-474 | SO₂Me | Cl | 2 | CH₂CH₂OMe | Me | CF₃ |
| 3-475 | SO₂Me | Me | 0 | CH₂CH₂OMe | Me | SO₂Me |
| 3-476 | SO₂Me | Me | 1 | CH₂CH₂OMe | Me | SO₂Me |
| 3-477 | SO₂Me | Me | 2 | CH₂CH₂OMe | Me | SO₂Me |
| 3-478 | SO₂Me | Cl | 0 | CH₂CH₂OMe | Me | SO₂Me |
| 3-479 | SO₂Me | Cl | 1 | CH₂CH₂OMe | Me | SO₂Me |
| 3-480 | SO₂Me | Cl | 2 | CH₂CH₂OMe | Me | SO₂Me |

**Tabelle 4: Erfindungsgemäße Verbindungen der allgemeinen Formel (I), worin R² Wasserstoff und R³ Acetyl bedeuten, A für eine direkte Bindung, X¹ und X² jeweils für CH und X³ für CR⁸ stehen**

| | | | | | |
|---|---|---|---|---|---|
| 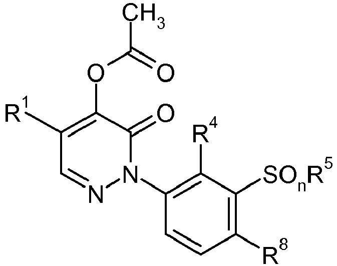 | | | | | |

| **Nr.** | **R¹** | **R⁴** | **n** | **R⁵** | **R⁸** |
|---|---|---|---|---|---|
| 4-1 | Me | Me | 0 | CH₂-c-Pr | CF₃ |
| 4-2 | Me | Me | 1 | CH₂-c-Pr | CF₃ |
| 4-3 | Me | Me | 2 | CH₂-c-Pr | CF₃ |
| 4-4 | Me | Cl | 0 | CH₂-c-Pr | CF₃ |
| 4-5 | Me | Cl | 1 | CH₂-c-Pr | CF₃ |
| 4-6 | Me | Cl | 2 | CH₂-c-Pr | CF₃ |
| 4-7 | Me | Me | 0 | CH₂-c-Pr | SO₂Me |
| 4-8 | Me | Me | 1 | CH₂-c-Pr | SO₂Me |
| 4-9 | Me | Me | 2 | CH₂-c-Pr | SO₂Me |
| 4-10 | Me | Cl | 0 | CH₂-c-Pr | SO₂Me |
| 4-11 | Me | Cl | 1 | CH₂-c-Pr | SO₂Me |
| 4-12 | Me | Cl | 2 | CH₂-c-Pr | SO₂Me |
| 4-13 | Cl | Me | 0 | CH₂-c-Pr | CF₃ |
| 4-14 | Cl | Me | 1 | CH₂-c-Pr | CF₃ |
| 4-15 | Cl | Me | 2 | CH₂-c-Pr | CF₃ |
| 4-16 | Cl | Cl | 0 | CH₂-c-Pr | CF₃ |
| 4-17 | Cl | Cl | 1 | CH₂-c-Pr | CF₃ |
| 4-18 | Cl | Cl | 2 | CH₂-c-Pr | CF₃ |
| 4-19 | Cl | Me | 0 | CH₂-c-Pr | SO₂Me |
| 4-20 | Cl | Me | 1 | CH₂-c-Pr | SO₂Me |
| 4-21 | Cl | Me | 2 | CH₂-c-Pr | SO₂Me |
| 4-22 | Cl | Cl | 0 | CH₂-c-Pr | SO₂Me |
| 4-23 | Cl | Cl | 1 | CH₂-c-Pr | SO₂Me |
| 4-24 | Cl | Cl | 2 | CH₂-c-Pr | SO₂Me |
| 4-25 | SO₂Me | Me | 0 | CH₂-c-Pr | CF₃ |
| 4-26 | SO₂Me | Me | 1 | CH₂-c-Pr | CF₃ |
| 4-27 | SO₂Me | Me | 2 | CH₂-c-Pr | CF₃ |
| 4-28 | SO₂Me | Cl | 0 | CH₂-c-Pr | CF₃ |
| 4-29 | SO₂Me | Cl | 1 | CH₂-c-Pr | CF₃ |
| 4-30 | SO₂Me | Cl | 2 | CH₂-c-Pr | CF₃ |
| 4-31 | SO₂Me | Me | 0 | CH₂-c-Pr | SO₂Me |
| 4-32 | SO₂Me | Me | 1 | CH₂-c-Pr | SO₂Me |
| 4-33 | SO₂Me | Me | 2 | CH₂-c-Pr | SO₂Me |
| 4-34 | SO₂Me | Cl | 0 | CH₂-c-Pr | SO₂Me |
| 4-35 | SO₂Me | Cl | 1 | CH₂-c-Pr | SO₂Me |
| 4-36 | SO₂Me | Cl | 2 | CH₂-c-Pr | SO₂Me |
| 4-37 | Me | Me | 0 | CH₂CH₂OMe | CF₃ |
| 4-38 | Me | Me | 1 | CH₂CH₂OMe | CF₃ |
| 4-39 | Me | Me | 2 | CH₂CH₂OMe | CF₃ |
| 4-40 | Me | Cl | 0 | CH₂CH₂OMe | CF₃ |
| 4-41 | Me | Cl | 1 | CH₂CH₂OMe | CF₃ |
| 4-42 | Me | Cl | 2 | CH₂CH₂OMe | CF₃ |
| 4-43 | Me | Me | 0 | CH₂CH₂OMe | SO₂Me |
| 4-44 | Me | Me | 1 | CH₂CH₂OMe | SO₂Me |
| 4-45 | Me | Me | 2 | CH₂CH₂OMe | SO₂Me |
| 4-46 | Me | Cl | 0 | CH₂CH₂OMe | SO₂Me |
| 4-47 | Me | Cl | 1 | CH₂CH₂OMe | SO₂Me |
| 4-48 | Me | Cl | 2 | CH₂CH₂OMe | SO₂Me |
| 4-49 | Cl | Me | 0 | CH₂CH₂OMe | CF₃ |
| 4-50 | Cl | Me | 1 | CH₂CH₂OMe | CF₃ |
| 4-51 | Cl | Me | 2 | CH₂CH₂OMe | CF₃ |
| 4-52 | Cl | Cl | 0 | CH₂CH₂OMe | CF₃ |
| 4-53 | Cl | Cl | 1 | CH₂CH₂OMe | CF₃ |
| 4-54 | Cl | Cl | 2 | CH₂CH₂OMe | CF₃ |
| 4-55 | Cl | Me | 0 | CH₂CH₂OMe | SO₂Me |
| 4-56 | Cl | Me | 1 | CH₂CH₂OMe | SO₂Me |
| 4-57 | Cl | Me | 2 | CH₂CH₂OMe | SO₂Me |
| 4-58 | Cl | Cl | 0 | CH₂CH₂OMe | SO₂Me |
| 4-59 | Cl | Cl | 1 | CH₂CH₂OMe | SO₂Me |
| 4-60 | Cl | Cl | 2 | CH₂CH₂OMe | SO₂Me |
| 4-61 | SO₂Me | Me | 0 | CH₂CH₂OMe | CF₃ |
| 4-62 | SO₂Me | Me | 1 | CH₂CH₂OMe | CF₃ |
| 4-63 | SO₂Me | Me | 2 | CH₂CH₂OMe | CF₃ |
| 4-64 | SO₂Me | Cl | 0 | CH₂CH₂OMe | CF₃ |
| 4-65 | SO₂Me | Cl | 1 | CH₂CH₂OMe | CF₃ |
| 4-66 | SO₂Me | Cl | 2 | CH₂CH₂OMe | CF₃ |
| 4-67 | SO₂Me | Me | 0 | CH₂CH₂OMe | SO₂Me |
| 4-68 | SO₂Me | Me | 1 | CH₂CH₂OMe | SO₂Me |
| 4-69 | SO₂Me | Me | 2 | CH₂CH₂OMe | SO₂Me |
| 4-70 | SO₂Me | Cl | 0 | CH₂CH₂OMe | SO₂Me |
| 4-71 | SO₂Me | Cl | 1 | CH₂CH₂OMe | SO₂Me |
| 4-72 | SO₂Me | Cl | 2 | CH₂CH₂OMe | SO₂Me |

**Tabelle 5: Erfindungsgemäße Verbindungen der allgemeinen Formel (I), worin R² Wasserstoff und R³ Acetyl bedeuten, A für -CH₂-, X¹ und X² jeweils für CH und X³ für CR⁸ stehen**

| | | | | | |
|---|---|---|---|---|---|
| 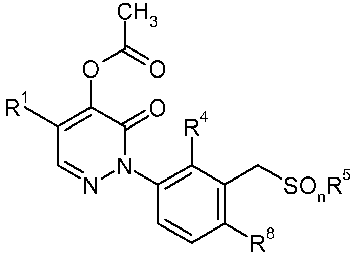 | | | | | |

| **Nr.** | **R¹** | **R⁴** | **n** | **R⁵** | **R⁸** |
|---|---|---|---|---|---|
| 5-1 | Me | Me | 0 | Me | CF₃ |
| 5-2 | Me | Me | 1 | Me | CF₃ |
| 5-3 | Me | Me | 2 | Me | CF₃ |
| 5-4 | Me | Cl | 0 | Me | CF₃ |
| 5-5 | Me | Cl | 1 | Me | CF₃ |
| 5-6 | Me | Cl | 2 | Me | CF₃ |
| 5-7 | Me | Me | 0 | Me | SO₂Me |
| 5-8 | Me | Me | 1 | Me | SO₂Me |
| 5-9 | Me | Me | 2 | Me | SO₂Me |
| 5-10 | Me | Cl | 0 | Me | SO₂Me |
| 5-11 | Me | Cl | 1 | Me | SO₂Me |
| 5-12 | Me | Cl | 2 | Me | SO₂Me |
| 5-13 | Cl | Me | 0 | Me | CF₃ |
| 5-14 | Cl | Me | 1 | Me | CF₃ |
| 5-15 | Cl | Me | 2 | Me | CF₃ |
| 5-16 | Cl | Cl | 0 | Me | CF₃ |
| 5-17 | Cl | Cl | 1 | Me | CF₃ |
| 5-18 | Cl | Cl | 2 | Me | CF₃ |
| 5-19 | Cl | Me | 0 | Me | SO₂Me |
| 5-20 | Cl | Me | 1 | Me | SO₂Me |
| 5-21 | Cl | Me | 2 | Me | SO₂Me |
| 5-22 | Cl | Cl | 0 | Me | SO₂Me |
| 5-23 | Cl | Cl | 1 | Me | SO₂Me |
| 5-24 | Cl | Cl | 2 | Me | SO₂Me |
| 5-25 | SO₂Me | Me | 0 | Me | CF₃ |
| 5-26 | SO₂Me | Me | 1 | Me | CF₃ |
| 5-27 | SO₂Me | Me | 2 | Me | CF₃ |
| 5-28 | SO₂Me | Cl | 0 | Me | CF₃ |
| 5-29 | SO₂Me | Cl | 1 | Me | CF₃ |
| 5-30 | SO₂Me | Cl | 2 | Me | CF₃ |
| 5-31 | SO₂Me | Me | 0 | Me | SO₂Me |
| 5-32 | SO₂Me | Me | 1 | Me | SO₂Me |
| 5-33 | SO₂Me | Me | 2 | Me | SO₂Me |
| 5-34 | SO₂Me | Cl | 0 | Me | SO₂Me |
| 5-35 | SO₂Me | Cl | 1 | Me | SO₂Me |
| 5-36 | SO₂Me | Cl | 2 | Me | SO₂Me |
| 5-37 | Me | Me | 0 | CH₂CH₂OMe | CF₃ |
| 5-38 | Me | Me | 1 | CH₂CH₂OMe | CF₃ |
| 5-39 | Me | Me | 2 | CH₂CH₂OMe | CF₃ |
| 5-40 | Me | Cl | 0 | CH₂CH₂OMe | CF₃ |
| 5-41 | Me | Cl | 1 | CH₂CH₂OMe | CF₃ |
| 5-42 | Me | Cl | 2 | CH₂CH₂OMe | CF₃ |
| 5-43 | Me | Me | 0 | CH₂CH₂OMe | SO₂Me |
| 5-44 | Me | Me | 1 | CH₂CH₂OMe | SO₂Me |
| 5-45 | Me | Me | 2 | CH₂CH₂OMe | SO₂Me |
| 5-46 | Me | Cl | 0 | CH₂CH₂OMe | SO₂Me |
| 5-47 | Me | Cl | 1 | CH₂CH₂OMe | SO₂Me |
| 5-48 | Me | Cl | 2 | CH₂CH₂OMe | SO₂Me |
| 5-49 | Cl | Me | 0 | CH₂CH₂OMe | CF₃ |
| 5-50 | Cl | Me | 1 | CH₂CH₂OMe | CF₃ |
| 5-51 | Cl | Me | 2 | CH₂CH₂OMe | CF₃ |
| 5-52 | Cl | Cl | 0 | CH₂CH₂OMe | CF₃ |
| 5-53 | Cl | Cl | 1 | CH₂CH₂OMe | CF₃ |
| 5-54 | Cl | Cl | 2 | CH₂CH₂OMe | CF₃ |
| 5-55 | Cl | Me | 0 | CH₂CH₂OMe | SO₂Me |
| 5-56 | Cl | Me | 1 | CH₂CH₂OMe | SO₂Me |
| 5-57 | Cl | Me | 2 | CH₂CH₂OMe | SO₂Me |
| 5-58 | Cl | Cl | 0 | CH₂CH₂OMe | SO₂Me |
| 5-59 | Cl | Cl | 1 | CH₂CH₂OMe | SO₂Me |
| 5-60 | Cl | Cl | 2 | CH₂CH₂OMe | SO₂Me |
| 5-61 | SO₂Me | Me | 0 | CH₂CH₂OMe | CF₃ |
| 5-62 | SO₂Me | Me | 1 | CH₂CH₂OMe | CF₃ |
| 5-63 | SO₂Me | Me | 2 | CH₂CH₂OMe | CF₃ |
| 5-64 | SO₂Me | Cl | 0 | CH₂CH₂OMe | CF₃ |
| 5-65 | SO₂Me | Cl | 1 | CH₂CH₂OMe | CF₃ |
| 5-66 | SO₂Me | Cl | 2 | CH₂CH₂OMe | CF₃ |
| 5-67 | SO₂Me | Me | 0 | CH₂CH₂OMe | SO₂Me |
| 5-68 | SO₂Me | Me | 1 | CH₂CH₂OMe | SO₂Me |
| 5-69 | SO₂Me | Me | 2 | CH₂CH₂OMe | SO₂Me |
| 5-70 | SO₂Me | Cl | 0 | CH₂CH₂OMe | SO₂Me |
| 5-71 | SO₂Me | Cl | 1 | CH₂CH₂OMe | SO₂Me |
| 5-72 | SO₂Me | Cl | 2 | CH₂CH₂OMe | SO₂Me |

**Tabelle 6: Erfindungsgemäße Verbindungen der allgemeinen Formel (I), worin R² Wasserstoff und R³ Acetyl bedeuten, A für -CH₂CH₂-, X¹ und X² jeweils für CH und X³ für CR⁸ stehen**

| | | | | | |
|---|---|---|---|---|---|
| 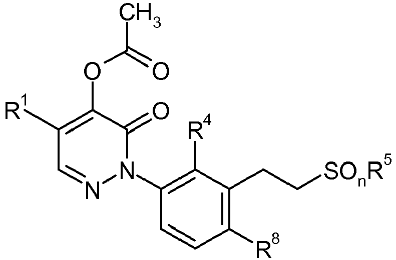 | | | | | |

| **Nr.** | **R¹** | **R⁴** | **n** | **R⁵** | **R⁸** |
|---|---|---|---|---|---|
| 6-1 | Me | Me | 0 | Me | CF₃ |
| 6-2 | Me | Me | 1 | Me | CF₃ |
| 6-3 | Me | Me | 2 | Me | CF₃ |
| 6-4 | Me | Cl | 0 | Me | CF₃ |
| 6-5 | Me | Cl | 1 | Me | CF₃ |
| 6-6 | Me | Cl | 2 | Me | CF₃ |
| 6-7 | Me | Me | 0 | Me | SO₂Me |
| 6-8 | Me | Me | 1 | Me | SO₂Me |
| 6-9 | Me | Me | 2 | Me | SO₂Me |
| 6-10 | Me | Cl | 0 | Me | SO₂Me |
| 6-11 | Me | Cl | 1 | Me | SO₂Me |
| 6-12 | Me | Cl | 2 | Me | SO₂Me |
| 6-13 | Cl | Me | 0 | Me | CF₃ |
| 6-14 | Cl | Me | 1 | Me | CF₃ |
| 6-15 | Cl | Me | 2 | Me | CF₃ |
| 6-16 | Cl | Cl | 0 | Me | CF₃ |
| 6-17 | Cl | Cl | 1 | Me | CF₃ |
| 6-18 | Cl | Cl | 2 | Me | CF₃ |
| 6-19 | Cl | Me | 0 | Me | SO₂Me |
| 6-20 | Cl | Me | 1 | Me | SO₂Me |
| 6-21 | Cl | Me | 2 | Me | SO₂Me |
| 6-22 | Cl | Cl | 0 | Me | SO₂Me |
| 6-23 | Cl | Cl | 1 | Me | SO₂Me |
| 6-24 | Cl | Cl | 2 | Me | SO₂Me |
| 6-25 | SO₂Me | Me | 0 | Me | CF₃ |
| 6-26 | SO₂Me | Me | 1 | Me | CF₃ |
| 6-27 | SO₂Me | Me | 2 | Me | CF₃ |
| 6-28 | SO₂Me | Cl | 0 | Me | CF₃ |
| 6-29 | SO₂Me | Cl | 1 | Me | CF₃ |
| 6-30 | SO₂Me | Cl | 2 | Me | CF₃ |
| 6-31 | SO₂Me | Me | 0 | Me | SO₂Me |
| 6-32 | SO₂Me | Me | 1 | Me | SO₂Me |
| 6-33 | SO₂Me | Me | 2 | Me | SO₂Me |
| 6-34 | SO₂Me | Cl | 0 | Me | SO₂Me |
| 6-35 | SO₂Me | Cl | 1 | Me | SO₂Me |
| 6-36 | SO₂Me | Cl | 2 | Me | SO₂Me |
| 6-37 | Me | Me | 0 | CH₂CH₂OMe | CF₃ |
| 6-38 | Me | Me | 1 | CH₂CH₂OMe | CF₃ |
| 6-39 | Me | Me | 2 | CH₂CH₂OMe | CF₃ |
| 6-40 | Me | Cl | 0 | CH₂CH₂OMe | CF₃ |
| 6-41 | Me | Cl | 1 | CH₂CH₂OMe | CF₃ |
| 6-42 | Me | Cl | 2 | CH₂CH₂OMe | CF₃ |
| 6-43 | Me | Me | 0 | CH₂CH₂OMe | SO₂Me |
| 6-44 | Me | Me | 1 | CH₂CH₂OMe | SO₂Me |
| 6-45 | Me | Me | 2 | CH₂CH₂OMe | SO₂Me |
| 6-46 | Me | Cl | 0 | CH₂CH₂OMe | SO₂Me |
| 6-47 | Me | Cl | 1 | CH₂CH₂OMe | SO₂Me |
| 6-48 | Me | Cl | 2 | CH₂CH₂OMe | SO₂Me |
| 6-49 | Cl | Me | 0 | CH₂CH₂OMe | CF₃ |
| 6-50 | Cl | Me | 1 | CH₂CH₂OMe | CF₃ |
| 6-51 | Cl | Me | 2 | CH₂CH₂OMe | CF₃ |
| 6-52 | Cl | Cl | 0 | CH₂CH₂OMe | CF₃ |
| 6-53 | Cl | Cl | 1 | CH₂CH₂OMe | CF₃ |
| 6-54 | Cl | Cl | 2 | CH₂CH₂OMe | CF₃ |
| 6-55 | Cl | Me | 0 | CH₂CH₂OMe | SO₂Me |
| 6-56 | Cl | Me | 1 | CH₂CH₂OMe | SO₂Me |
| 6-57 | Cl | Me | 2 | CH₂CH₂OMe | SO₂Me |
| 6-58 | Cl | Cl | 0 | CH₂CH₂OMe | SO₂Me |
| 6-59 | Cl | Cl | 1 | CH₂CH₂OMe | SO₂Me |
| 6-60 | Cl | Cl | 2 | CH₂CH₂OMe | SO₂Me |
| 6-61 | SO₂Me | Me | 0 | CH₂CH₂OMe | CF₃ |
| 6-62 | SO₂Me | Me | 1 | CH₂CH₂OMe | CF₃ |
| 6-63 | SO₂Me | Me | 2 | CH₂CH₂OMe | CF₃ |
| 6-64 | SO₂Me | Cl | 0 | CH₂CH₂OMe | CF₃ |
| 6-65 | SO₂Me | Cl | 1 | CH₂CH₂OMe | CF₃ |
| 6-66 | SO₂Me | Cl | 2 | CH₂CH₂OMe | CF₃ |
| 6-67 | SO₂Me | Me | 0 | CH₂CH₂OMe | SO₂Me |
| 6-68 | SO₂Me | Me | 1 | CH₂CH₂OMe | SO₂Me |
| 6-69 | SO₂Me | Me | 2 | CH₂CH₂OMe | SO₂Me |
| 6-70 | SO₂Me | Cl | 0 | CH₂CH₂OMe | SO₂Me |
| 6-71 | SO₂Me | Cl | 1 | CH₂CH₂OMe | SO₂Me |
| 6-72 | SO₂Me | Cl | 2 | CH₂CH₂OMe | SO₂Me |

**Tabelle 7: Erfindungsgemäße Verbindungen der allgemeinen Formel (I) in Form der Natriumsalze, worin R² Wasserstoff bedeutet, A für eine direkte Bindung, X¹ und X² jeweils für CH und X³ für CR⁸ stehen**

| | | | | | |
|---|---|---|---|---|---|
| 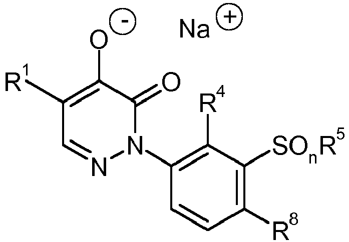 | | | | | |

| **Nr.** | **R¹** | **R⁴** | **n** | **R⁵** | **R⁸** |
|---|---|---|---|---|---|
| 7-1 | Me | Me | 0 | CH₂-c-Pr | CF₃ |
| 7-2 | Me | Me | 1 | CH₂-c-Pr | CF₃ |
| 7-3 | Me | Me | 2 | CH₂-c-Pr | CF₃ |
| 7-4 | Me | Cl | 0 | CH₂-c-Pr | CF₃ |
| 7-5 | Me | Cl | 1 | CH₂-c-Pr | CF₃ |
| 7-6 | Me | Cl | 2 | CH₂-c-Pr | CF₃ |
| 7-7 | Me | Me | 0 | CH₂-c-Pr | SO₂Me |
| 7-8 | Me | Me | 1 | CH₂-c-Pr | SO₂Me |
| 7-9 | Me | Me | 2 | CH₂-c-Pr | SO₂Me |
| 7-10 | Me | Cl | 0 | CH₂-c-Pr | SO₂Me |
| 7-11 | Me | Cl | 1 | CH₂-c-Pr | SO₂Me |
| 7-12 | Me | Cl | 2 | CH₂-c-Pr | SO₂Me |
| 7-13 | SO₂Me | Me | 0 | CH₂-c-Pr | CF₃ |
| 7-14 | SO₂Me | Me | 1 | CH₂-c-Pr | CF₃ |
| 7-15 | SO₂Me | Me | 2 | CH₂-c-Pr | CF₃ |
| 7-16 | SO₂Me | Cl | 0 | CH₂-c-Pr | CF₃ |
| 7-17 | SO₂Me | Cl | 1 | CH₂-c-Pr | CF₃ |
| 7-18 | SO₂Me | Cl | 2 | CH₂-c-Pr | CF₃ |
| 7-19 | SO₂Me | Me | 0 | CH₂-c-Pr | SO₂Me |
| 7-20 | SO₂Me | Me | 1 | CH₂-c-Pr | SO₂Me |
| 7-21 | SO₂Me | Me | 2 | CH₂-c-Pr | SO₂Me |
| 7-22 | SO₂Me | Cl | 0 | CH₂-c-Pr | SO₂Me |
| 7-23 | SO₂Me | Cl | 1 | CH₂-c-Pr | SO₂Me |
| 7-24 | SO₂Me | Cl | 2 | CH₂-c-Pr | SO₂Me |
| 7-25 | Me | Me | 0 | CH₂CH₂OMe | CF₃ |
| 7-26 | Me | Me | 1 | CH₂CH₂OMe | CF₃ |
| 7-27 | Me | Me | 2 | CH₂CH₂OMe | CF₃ |
| 7-28 | Me | Cl | 0 | CH₂CH₂OMe | CF₃ |
| 7-29 | Me | Cl | 1 | CH₂CH₂OMe | CF₃ |
| 7-30 | Me | Cl | 2 | CH₂CH₂OMe | CF₃ |
| 7-31 | Me | Me | 0 | CH₂CH₂OMe | SO₂Me |
| 7-32 | Me | Me | 1 | CH₂CH₂OMe | SO₂Me |
| 7-33 | Me | Me | 2 | CH₂CH₂OMe | SO₂Me |
| 7-34 | Me | Cl | 0 | CH₂CH₂OMe | SO₂Me |
| 7-35 | Me | Cl | 1 | CH₂CH₂OMe | SO₂Me |
| 7-36 | Me | Cl | 2 | CH₂CH₂OMe | SO₂Me |
| 7-37 | SO₂Me | Me | 0 | CH₂CH₂OMe | CF₃ |
| 7-38 | SO₂Me | Me | 1 | CH₂CH₂OMe | CF₃ |
| 7-39 | SO₂Me | Me | 2 | CH₂CH₂OMe | CF₃ |
| 7-40 | SO₂Me | Cl | 0 | CH₂CH₂OMe | CF₃ |
| 7-41 | SO₂Me | Cl | 1 | CH₂CH₂OMe | CF₃ |
| 7-42 | SO₂Me | Cl | 2 | CH₂CH₂OMe | CF₃ |
| 7-43 | SO₂Me | Me | 0 | CH₂CH₂OMe | SO₂Me |
| 7-44 | SO₂Me | Me | 1 | CH₂CH₂OMe | SO₂Me |
| 7-45 | SO₂Me | Me | 2 | CH₂CH₂OMe | SO₂Me |
| 7-46 | SO₂Me | Cl | 0 | CH₂CH₂OMe | SO₂Me |
| 7-47 | SO₂Me | Cl | 1 | CH₂CH₂OMe | SO₂Me |
| 7-48 | SO₂Me | Cl | 2 | CH₂CH₂OMe | SO₂Me |

**Tabelle 8: Erfindungsgemäße Verbindungen der allgemeinen Formel (I) in Form der Natriumsalze, worin R² Wasserstoff bedeutet, A für -CH₂-, X¹ und X² jeweils für CH und X³ für CR⁸ stehen**

| | | | | | |
|---|---|---|---|---|---|
| 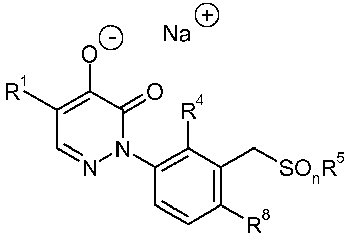 | | | | | |

| **Nr.** | **R¹** | **R⁴** | **n** | **R⁵** | **R⁸** |
|---|---|---|---|---|---|
| 8-1 | Me | Me | 0 | Me | CF₃ |
| 8-2 | Me | Me | 1 | Me | CF₃ |
| 8-3 | Me | Me | 2 | Me | CF₃ |
| 8-4 | Me | Cl | 0 | Me | CF₃ |
| 8-5 | Me | Cl | 1 | Me | CF₃ |
| 8-6 | Me | Cl | 2 | Me | CF₃ |
| 8-7 | Me | Me | 0 | Me | SO₂Me |
| 8-8 | Me | Me | 1 | Me | SO₂Me |
| 8-9 | Me | Me | 2 | Me | SO₂Me |
| 8-10 | Me | Cl | 0 | Me | SO₂Me |
| 8-11 | Me | Cl | 1 | Me | SO₂Me |
| 8-12 | Me | Cl | 2 | Me | SO₂Me |
| 8-13 | SO₂Me | Me | 0 | Me | CF₃ |
| 8-14 | SO₂Me | Me | 1 | Me | CF₃ |
| 8-15 | SO₂Me | Me | 2 | Me | CF₃ |
| 8-16 | SO₂Me | Cl | 0 | Me | CF₃ |
| 8-17 | SO₂Me | Cl | 1 | Me | CF₃ |
| 8-18 | SO₂Me | Cl | 2 | Me | CF₃ |
| 8-19 | SO₂Me | Me | 0 | Me | SO₂Me |
| 8-20 | SO₂Me | Me | 1 | Me | SO₂Me |
| 8-21 | SO₂Me | Me | 2 | Me | SO₂Me |
| 8-22 | SO₂Me | Cl | 0 | Me | SO₂Me |
| 8-23 | SO₂Me | Cl | 1 | Me | SO₂Me |
| 8-24 | SO₂Me | Cl | 2 | Me | SO₂Me |
| 8-25 | Me | Me | 0 | CH₂CH₂OMe | CF₃ |
| 8-26 | Me | Me | 1 | CH₂CH₂OMe | CF₃ |
| 8-27 | Me | Me | 2 | CH₂CH₂OMe | CF₃ |
| 8-28 | Me | Cl | 0 | CH₂CH₂OMe | CF₃ |
| 8-29 | Me | Cl | 1 | CH₂CH₂OMe | CF₃ |
| 8-30 | Me | Cl | 2 | CH₂CH₂OMe | CF₃ |
| 8-31 | Me | Me | 0 | CH₂CH₂OMe | SO₂Me |
| 8-32 | Me | Me | 1 | CH₂CH₂OMe | SO₂Me |
| 8-33 | Me | Me | 2 | CH₂CH₂OMe | SO₂Me |
| 8-34 | Me | Cl | 0 | CH₂CH₂OMe | SO₂Me |
| 8-35 | Me | Cl | 1 | CH₂CH₂OMe | SO₂Me |
| 8-36 | Me | Cl | 2 | CH₂CH₂OMe | SO₂Me |
| 8-37 | SO₂Me | Me | 0 | CH₂CH₂OMe | CF₃ |
| 8-38 | SO₂Me | Me | 1 | CH₂CH₂OMe | CF₃ |
| 8-39 | SO₂Me | Me | 2 | CH₂CH₂OMe | CF₃ |
| 8-40 | SO₂Me | Cl | 0 | CH₂CH₂OMe | CF₃ |
| 8-41 | SO₂Me | Cl | 1 | CH₂CH₂OMe | CF₃ |
| 8-42 | SO₂Me | Cl | 2 | CH₂CH₂OMe | CF₃ |
| 8-43 | SO₂Me | Me | 0 | CH₂CH₂OMe | SO₂Me |
| 8-44 | SO₂Me | Me | 1 | CH₂CH₂OMe | SO₂Me |
| 8-45 | SO₂Me | Me | 2 | CH₂CH₂OMe | SO₂Me |
| 8-46 | SO₂Me | Cl | 0 | CH₂CH₂OMe | SO₂Me |
| 8-47 | SO₂Me | Cl | 1 | CH₂CH₂OMe | SO₂Me |
| 8-48 | SO₂Me | Cl | 2 | CH₂CH₂OMe | SO₂Me |

**Tabelle 9: Erfindungsgemäße Verbindungen der allgemeinen Formel (I) in Form der Natriumsalze, worin R² Wasserstoff bedeutet, A für -CH₂CH₂-, X¹ und X² jeweils für CH und X³ für CR⁸ stehen**

| | | | | | |
|---|---|---|---|---|---|
| 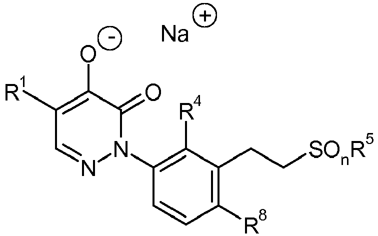 | | | | | |

| **Nr.** | **R¹** | **R⁴** | **n** | **R⁵** | **R⁸** |
|---|---|---|---|---|---|
| 9-1 | Me | Me | 0 | Me | CF₃ |
| 9-2 | Me | Me | 1 | Me | CF₃ |
| 9-3 | Me | Me | 2 | Me | CF₃ |
| 9-4 | Me | Cl | 0 | Me | CF₃ |
| 9-5 | Me | Cl | 1 | Me | CF₃ |
| 9-6 | Me | Cl | 2 | Me | CF₃ |
| 9-7 | Me | Me | 0 | Me | SO₂Me |
| 9-8 | Me | Me | 1 | Me | SO₂Me |
| 9-9 | Me | Me | 2 | Me | SO₂Me |
| 9-10 | Me | Cl | 0 | Me | SO₂Me |
| 9-11 | Me | Cl | 1 | Me | SO₂Me |
| 9-12 | Me | Cl | 2 | Me | SO₂Me |
| 9-13 | SO₂Me | Me | 0 | Me | CF₃ |
| 9-14 | SO₂Me | Me | 1 | Me | CF₃ |
| 9-15 | SO₂Me | Me | 2 | Me | CF₃ |
| 9-16 | SO₂Me | Cl | 0 | Me | CF₃ |
| 9-17 | SO₂Me | Cl | 1 | Me | CF₃ |
| 9-18 | SO₂Me | Cl | 2 | Me | CF₃ |
| 9-19 | SO₂Me | Me | 0 | Me | SO₂Me |
| 9-20 | SO₂Me | Me | 1 | Me | SO₂Me |
| 9-21 | SO₂Me | Me | 2 | Me | SO₂Me |
| 9-22 | SO₂Me | Cl | 0 | Me | SO₂Me |
| 9-23 | SO₂Me | Cl | 1 | Me | SO₂Me |
| 9-24 | SO₂Me | Cl | 2 | Me | SO₂Me |
| 9-25 | Me | Me | 0 | CH₂CH₂OMe | CF₃ |
| 9-26 | Me | Me | 1 | CH₂CH₂OMe | CF₃ |
| 9-27 | Me | Me | 2 | CH₂CH₂OMe | CF₃ |
| 9-28 | Me | Cl | 0 | CH₂CH₂OMe | CF₃ |
| 9-29 | Me | Cl | 1 | CH₂CH₂OMe | CF₃ |
| 9-30 | Me | Cl | 2 | CH₂CH₂OMe | CF₃ |
| 9-31 | Me | Me | 0 | CH₂CH₂OMe | SO₂Me |
| 9-32 | Me | Me | 1 | CH₂CH₂OMe | SO₂Me |
| 9-33 | Me | Me | 2 | CH₂CH₂OMe | SO₂Me |
| 9-34 | Me | Cl | 0 | CH₂CH₂OMe | SO₂Me |
| 9-35 | Me | Cl | 1 | CH₂CH₂OMe | SO₂Me |
| 9-36 | Me | Cl | 2 | CH₂CH₂OMe | SO₂Me |
| 9-37 | SO₂Me | Me | 0 | CH₂CH₂OMe | CF₃ |
| 9-38 | SO₂Me | Me | 1 | CH₂CH₂OMe | CF₃ |
| 9-39 | SO₂Me | Me | 2 | CH₂CH₂OMe | CF₃ |
| 9-40 | SO₂Me | Cl | 0 | CH₂CH₂OMe | CF₃ |
| 9-41 | SO₂Me | Cl | 1 | CH₂CH₂OMe | CF₃ |
| 9-42 | SO₂Me | Cl | 2 | CH₂CH₂OMe | CF₃ |
| 9-43 | SO₂Me | Me | 0 | CH₂CH₂OMe | SO₂Me |
| 9-44 | SO₂Me | Me | 1 | CH₂CH₂OMe | SO₂Me |
| 9-45 | SO₂Me | Me | 2 | CH₂CH₂OMe | SO₂Me |
| 9-46 | SO₂Me | Cl | 0 | CH₂CH₂OMe | SO₂Me |
| 9-47 | SO₂Me | Cl | 1 | CH₂CH₂OMe | SO₂Me |
| 9-48 | SO₂Me | Cl | 2 | CH₂CH₂OMe | SO₂Me |

### A. Chemische Beispiele

### Herstellung von 5-Chlor-2-{3-[(cyclopropylmethyl)sulfanyl]-2-methyl-4-(trifluormethyl)phenyl}-4-hydroxypyridazin-3(2H)-on (Beispiel-Nr. 1-499)

### Schritt 1: Synthese von 6-Brom-2-fluor-3-(trifluormethyl)benzaldehyd

Zu einer Lösung von 63.9 g (452.7 mmol) 2,2,6,6-Tetramethylpiperidin in 833 ml trockenem THF wurden bei -78 °C 181.07 ml einer 2.5M (452.7 mmol) Lösung von *n-*Butyllithium tropfenweise zugegeben. Das Gemisch wurde 30 min bei dieser Temperatur gerührt. Anschließend wurden 100.0 g (411.5 mmol) 4-Brom-2-fluor-1-(trifluormethyl)benzol bei -78 °C tropfenweise zugegeben. Die Mischung wurde 2 h bei dieser Temperatur gerührt. Danach wurden 33.1 g (452.7 mmol) DMF bei -78 °C tropfenweise zugegeben. Die Reaktionsmischung wurde anschließend 2 h gerührt. Zur Aufarbeitung wurde der Inhalt mit 300 ml Wasser versetzt. Das Gemisch wurde dreimal mit je 200 ml Dichlormethan extrahiert. Die vereinigten organischen Phasen wurden mit 300 ml 1M Salzsäure und danach mit 300 ml einer gesättigten wässrigen Kochsalzlösung gewaschen. Die organische Phase wurde getrocknet und das Filtrat wurde vom Lösungsmittel befreit. Es wurden 96.2 g des gewünschten Produkts gewonnen.

### Schritt 2: Synthese von 6-Brom-2-(tert-butylsulfanyl)-3-(trifluormethyl)benzaldehyd

Bei 0 °C wurden 30.3 g (335.8 mmol) *tert*-Butylmercaptan zu einer Lösung von 65.0 g (239.8 mmol) 6-Brom-2-fluor-3-(trifluormethyl)benzaldehyd und 66.3 g (479.7 mmol) Kaliumcarbonat in 500 ml N,N-Dimethylformamid gegeben. Das Gemisch wurde 12 h bei dieser Temperatur gerührt. Anschließend wurden 15.6 g (48.0 mmol) Cäsiumcarbonat zugegeben und das Gemisch wurde weitere 3 h gerührt. Zur Aufarbeitung wurde der Inhalt auf 1 I Wasser gegossen. Die Mischung wurde dreimal mit je 300 ml Dichlormethan extrahiert. Die vereinigten organischen Phasen wurden viermal mit je 300 ml einer gesättigten wässrigen Kochsalzlösung gewaschen. Die organische Phase wurde getrocknet und das Filtrat wurde vom Lösungsmittel befreit, wobei 68 g des gewünschten Produkts isoliert wurden.

### Schritt 3: Synthese von [6-Brom-2-(tert-butylsulfanyl)-3-(trifluormethyl)phenyl]-methanol

Bei -10 °C wurden 3.49 g (92.3 mmol) Natriumboranat langsam zu einer Lösung von 63.0 g (184.7 mmol) 6-Brom-2-(*tert*-butylsulfanyl)-3-(trifluormethyl)benzaldehyd in 500 ml Methanol gegeben. Nachdem die Reaktionskontrolle die vollständige Umsetzung angezeigt hatte wurde der Inhalt zur Aufarbeitung mit 3M Salzsäure versetzt. Das Gemisch wurde eingeengt und der Rückstand wurde auf 400 ml Wasser gegossen. Die Mischung wurde zweimal mit je 300 ml Dichlormethan extrahiert. Die vereinigten organischen Phasen wurden mit einer gesättigten wässrigen Kochsalzlösung gewaschen, getrocknet and abschließend wurde das Filtrat vom Lösungsmittel befreit. Es wurden 60.0 g des gewünschten Produkts gewonnen.

### Schritt 4: Synthese von 6-Brom-2-(tert-butylsulfanyl)-3-(trifluormethyl)benzyl-methansulfonat

Bei 0 °C wurden 31.3 g (272.8 mmol) Methansulfonsäurechlorid tropfenweise zu einer Lösung von 60.0 g (174.8 mmol) [6-Brom-2-(*tert*-butylsulfanyl)-3-(trifluormethyl)phenyl]methanol und 44.2 g (437.1 mmol) Triethylamin in 500 ml Dichlormethan gegeben. Nachdem die Reaktionskontrolle die vollständige Umsetzung angezeigt hatte wurde die Lösung zur Aufarbeitung zweimal mit je 300 ml Wasser gewaschen, getrocknet, und das Filtrat wurde vom Lösungsmittel befreit. Es wurden 70.0 g des gewünschten Produkts gewonnen.

### Schritt 5: Synthese von 1-Brom-3-(tert-butylsulfanyl)-2-methyl-4-(trifluormethyl)-benzol

Eine Lösung von 70.0 g (166.2 mmol) 6-Brom-2-(*tert*-butylsulfanyl)-3-(trifluormethyl)benzylmethansulfonat in 100 ml trockenem THF wurde bei -10 °C tropfenweise zu einer Lösung von 6.94 g (182.8 mmol) Lithiumalanat in 500 ml trockenem THF gegeben. Der Inhalt wurde 1 h gerührt. Zur Aufarbeitung wurde Natriumsulfat-Decahydrat zugegeben bis keine Gasentwicklung mehr zu beobachten war. Das Gemisch wurde filtriert und das Filtrat wurde getrocknet. Danach wurde das Filtrat vom Lösungsmittel befreit und der Rückstand wurde chromatographisch gereinigt, wobei 45.0 g des gewünschten Produkts gewonnen wurden.

### Schritt 6: Synthese von 3-Brom-2-methyl-6-(trifluormethyl)benzolthiol

23.7 g (137.5 mmol) 4-Methylbenzolsulfonsäure wurden zu einer Lösung von 45.0 g (137.5 mmol) 1-Brom-3-(tert-butylsulfanyl)-2-methyl-4-(trifluormethyl)benzol in 175 ml Toluol gegeben. Die Mischung wurde 2 h unter Rückfluss erhitzt. Das Lösungsmittel wurde am Rotationsverdampfer entfernt und der Rückstand wurde in 200 ml Dichlormethan gelöst. Die Lösung wurde viermal mit 15 proz. wässriger Kalilauge extrahiert. Die vereinigten wässrigen Phasen wurden mit konzentrierter Salzsäure angesäuert und anschließend wurde das Produkt mit Dichlormethan extrahiert. Die organische Phase wurde getrocknet, filtriert und das Filtrat wurde vom Lösungsmittel befreit, wobei 32.0 g des gewünschten Produkts gewonnen wurden.

### Schritt 7: Synthese von 1-Brom-3-[(cyclopropylmethyl)sulfanyl]-2-methyl-4-(trifluormethyl)benzol

Eine Mischung aus 20 g (74.1 mmol) 3-Brom-2-methyl-6-(trifluormethyl)benzolthiol und 36 g (111.1 mmol) Cäsiumcarbonat in 80 ml Acetonitril wurde mit 14.0 g (103.6 mmol) (Brommethyl)cyclopropan versetzt. Der Inhalt wurde bei 80 °C 2 h lang gerührt. Zur Aufarbeitung wurde das Gemisch filtriert und das Filtrat wurde vom Lösungsmittel befreit. Der Rückstand wurde chromatographisch gereinigt, wobei 20.0 g des gewünschten Produkts erhalten wurden.

### Schritt 8: Synthese von 1-{3-[(Cyclopropylmethyl)sulfanyl]-2-methyl-4-(trifluormethyl)phenyl}-2-(diphenylmethylen)hydrazin

Eine Mischung aus 20 mg (0.2 mmol) Natrium-*tert*-butanolat und 33 mg (0.17 mmol) Benzophenonhydrazon wurden zu einer Lösung von 50 mg (0.15 mmol) 1-Brom-3-[(cyclopropylmethyl)sulfanyl]-2-methyl-4-(trifluormethyl)benzol in 1 ml Toluol gegeben. Anschließend wurde das Gemisch 10 min lang zur Entfernung von Sauerstoff entgast. Danach wurde unter Schutzgas 1 mg (0.002 mmol) 2,2'-Bis(diphenylphosphino)-1,1'-binaphthyl zugegeben. Die Mischung wurde 15 min lang zur Entfernung von Sauerstoff entgast. Anschließend wurden unter Schutzgas 0.22 mg (0.001 mmol) Palladium(II)acetat zugegeben. Der Inhalt wurde unter Schutzgas 3 h lang auf eine Temperatur von 90 °C erhitzt. Nach Aufarbeitung und Reinigung wurden 35 mg des gewünschten Produkts gewonnen.

### Schritt 9: Synthese von {3-[(Cyclopropylmethyl)sulfanyl]-2-methyl-4-(trifluormethyl)-phenyl}hydrazin

Eine Lösung von 30 mg (0.11 mmol) 1-{3-[(Cyclopropylmethyl)sulfanyl]-2-methyl-4-(trifluormethyl)phenyl}-2-(diphenylmethylen)hydrazin in 2 ml Isopropylalkohol und 2 ml konzentrierter Salzsäure wurde 48 h bei Raumtemperatur gerührt. Nach Aufarbeitung und Reinigung wurden 10 mg des gewünschten Produkts gewonnen.

### Schritt 10: Synthese von 4,5-Dichlor-2-{3-[(cyclopropylmethyl)sulfanyl]-2-methyl-4-(trifluormethyl)phenyl}pyridazin-3(2H)-on

34 mg (0.2 mmol, 1.1 eq) 3,4-dichloro-5-hydroxyfuran-2(5H)-on wurden zu einer Lösung von 50 mg (0.18 mmol) {3-[(Cyclopropylmethyl)sulfanyl]-2-methyl-4-(trifluormethyl)phenyl}hydrazin in 1 ml Ethanol gegeben. Die Mischung wurde 3 h bei Raumtemperatur gerührt. Danach wurde 1 ml Essigsäure zugegeben und das Gemisch wurde 3 h unter Rückfluss erhitzt. Nach Aufarbeitung und Reinigung wurden 40 mg des gewünschten Produkts gewonnen.

### Schritt 11: Synthese von 5-Chlor-2-{3-[(cyclopropylmethyl)sulfanyl]-2-methyl-4-(trifluormethyl)phenyl}-4-methoxypyridazin-3(2H)-on

0.033 ml (20 proz., 0.12 mmol) einer Lösung von Natriummethanolat in Methanol wurden zu einer Lösung von 50 mg (0.12 mmol) 4,5-Dichlor-2-{3-[(cyclopropylmethyl)sulfanyl]-2-methyl-4-(trifluormethyl)phenyl}pyridazin-3(2H)-on in 2 ml trockenem Dioxan gegeben. Bei einer Temperatur von 15 °C wurde das Gemisch mit 5 ml trockenem Dioxan verdünnt. Das Reaktionsgemisch rührte anschließend noch 1 h bei einer Temperatur von 15 °C. Nach Aufarbeitung und Reinigung wurden 23 mg des gewünschten Produkts gewonnen.

Schritt 12: Synthese von 5-Chlor-2-{3-[(cyclopropylmethyl)sulfanyl]-2-methyl-4-(trifluormethyl)phenyl}-4-hydroxypyridazin-3(2H)-on (Beispiel-Nr. 1-499) Bei einer Temperatur von 0 °C wurden 18.6 mg (0.074 mmol) Bortribromid als 1M Lösung in Dichlormethan zu einer Lösung von 10 mg (0.02 mmol) 5-Chlor-2-{3-[(cyclopropylmethyl)sulfanyl]-2-methyl-4-(trifluormethyl)phenyl}-4-methoxypyridazin-3(2H)-on in 1 ml Dichlormethan gegeben. Das Gemisch wurde 1 h bei Raumtemperatur gerührt. Nach Aufarbeitung und Reinigung wurden 4 mg des gewünschten Produkts gewonnen.

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);.........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel 1-499: ¹H-NMR(400,0 MHz, CDCl₃): |
| δ= 7,910(11,2);7,677(4,9);7,656(4,8);7,518(7,4);7,380(1,2);7,296(6,0);7,290(4,4);7,276 (7,9);7,259(1356,1);7,226(2,8);7,209(3,3);7,140(1,7);6,995(7,4);3,731(3,6);3,487(1,3); 2,629(2,7);2,540(4,9);2,314(2,0);2,160(14,3);1,679(2,9);1,284(2,8);1,254(16,0);0,978(1 ,6);0,877(3,9);0,861(3,4);0,503(2,6);0,345(3,7);0,146(3,3);0,120(2,6);0,008(16,4);0,000 (542,8);-0,009(21,4);-0,033(4,7);-0,150(2,3) |
| Beispiel 2-139: ¹H-NMR(400,0 MHz, CDCl₃): |
| δ= 7,910(2,8);7,783(2,2);7,578(3,7);7,518(5,6);7,259(785,6);6,995(4,1);3,878(4,5);3,73 1(2,6);2,887(1,5);2,115(16,0);2,003(6,0);1,852(4,2);1,254(3,3);0,146(1,4);0,008(12,3); 0,000(389,1);-0,009(13,9);-0,149(1,4) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Beispielsweise zeigten dabei die Verbindungen Nr. 1-499 und 2-139 bei einer Aufwandmenge von 0,32 kg Aktivsubstanz oder weniger pro Hektar eine sehr gute Wirkung (80% bis 100% herbizide Wirkung) gegen Schadpflanzen wie Amaranthus retroflexus, Echinochloa crus-galli, Setaria viridis und Abutilon theophrasti. Gleichzeitig lassen erfindungsgemäße Verbindungen Gramineenkulturen wie Gerste, Weizen, Roggen, Hirse, Mais oder Reis im Vorauflaufverfahren selbst bei hohen Wirkstoffdosierungen praktisch ungeschädigt. Einige Substanzen schonen darüber hinaus auch zweikeimblättrige Kulturen wie Soja, Baumwolle, Raps, Zuckerrüben oder Kartoffeln.

Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Beispielsweise zeigten dabei die Verbindungen Nr. 1-499 und 2-139 bei einer Aufwand-menge von 0,08 kg Aktivsubstanz oder weniger pro Hektar eine sehr gute herbizide Wirkung (80% bis 100% herbizide Wirkung) gegen Schadpflanzen wie Pharbitis purpureum, Echinochloa crus-galli, Setaria viridis, Amaranthus retroflexus, Abutilon theophrasti, Viola tricolor, Veronica persica und Stellaria media. Gleichzeitig lassen erfindungsgemäße Verbindungen Gramineenkulturen wie Gerste, Weizen, Roggen, Hirse, Mais oder Reis im Nachauflaufverfahren selbst bei hohen Wirkstoffdosierungen praktisch ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen.

## Patentansprüche

1. 2-(Hetero)aryl-Pyridazinone der Formel (I) oder deren Salze 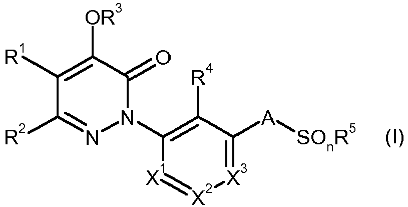 worin
R¹ bedeutet Wasserstoff, Halogen, Cyano, (C₁-C₆)-Alkyl, (C₃-C₆)-Cycloalkyl, (C₂-C₆)-Alkenyl, (C₄-C₆)-Cycloalkenyl, (C₂-C₆)-Alkinyl, Halogen-(C₁-C₆)-alkyl, (C₁-C₆)-Alkoxy, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, (C₃-C₆)-Cycloalkyl-(C₁-C₃)-alkyl, Amino, (C₁-C₆)-Alkylamino, Di-(C₁-C₆)-alkylamino, (C₁-C₃)-Alkyl-(O)C-amino-(C₁-C₄)-alkyl, (C₁-C₆)-Alkyl-(O)ₙS, (C₁-C₆)-Alkyl-(O)ₙS-(C₁-C₃)-alkyl, Halogen-(C₁-C₆)-alkyl-(O)ₙS oder Halogen-(C₁-C₆)-alkyl-(O)ₙS-(C₁-C₃)-alkyl;
R² bedeutet Wasserstoff, Hydroxy, Halogen, Nitro, Amino, Cyano, (C₁-C₆)-Alkyl, (C₁-C₃)-Alkoxy, (C₃-C₆)-Cycloalkyl, (C₂-C₆)-Alkenyl, (C₂-C₆)-Alkinyl, Halogen-(C₁-C₆)-alkyl, (C₁-C₆)-Alkoxy-(C₁-C₃)-alkyl, (C₃-C₆)-Cycloalkyl-(C₁-C₃)-alkyl, (C₁-C₆)-Alkyl-(O)ₙS, (C₁-C₆)-Alkyl-(O)ₙS-(C₁-C₃)-alkyl, Halogen-(C₁-C₆)-alkyl-(O)ₙS, Halogen-(C₁-C₆)-alkyl-(O)ₙS-(C₁-C₃)-alkyl, (C₁-C₃)-Alkylamino oder Di-(C₁-C₃)-alkylamino;
R³ bedeutet Wasserstoff, (C₁-C₆)-Alkyl-(O)C, Aryl-(O)C, (C₁-C₆)-Alkoxy-(O)C, (C₁-C₆)-Alkyl-(O)ₙS, (C₁-C₆)-Alkyl-(O)ₙS(O)C oder Aryl-(O)ₙS, wobei die Arylgruppen jeweils durch s Reste R⁹ substituiert sind;
R⁴ bedeutet Hydroxy, Halogen, Cyano, Nitro, (C₁-C₆)-Alkyl, (C₃-C₆)-Cycloalkyl, Halogen-(C₁-C₆)-alkyl, (C₂-C₆)-Alkenyl, Halogen-(C₂-C₆)-alkenyl, (C₂-C₆)-Alkinyl, (C₁-C₆)-Alkoxy, (C₂-C₆)-Alkenyloxy, (C₃-C₆)-Cycloalkyl-(C₁-C₃)-alkyl, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, Halogen-(C₁-C₆)-alkoxy, Halogen-(C₁-C₆)-alkoxy-(C₁-C₃)-alkyl, (C₁-C₆)-Alkyl-(O)ₙS, Halogen-(C₁-C₆)-alkyl-(O)ₙS, Aryl, Aryl-(O)ₙS, Heterocyclyl, Heterocyclyl-(O)ₙS, Aryloxy, Aryl-(C₂-C₆)-alkyl, Aryl-(C₁-C₆)-alkoxy, Heterocyclyloxy, Heterocyclyl-(C₁-C₃)-alkoxy-(C₁-C₃)-alkyl, HO(O)C, HO(O)C-(C₁-C₃)-alkoxy, (C₁-C₃)-Alkoxy-(O)C, (C₁-C₃)-Alkoxy-(O)C-(C₁-C₃)-alkoxy, (C₁-C₃)-Alkylamino, Oi-(C₁-C₃)-alkylamino, (C₁-C₃)-Alkylamino-(O)ₙS, (C₁-C₃)-Alkylamino-(O)ₙS-(C₁-C₃)-alkyl, Di-(C₁-C₃)-alkylamino-(O)ₙS, Di-(C₁-C₃)-alkylamino-(O)ₙS-(C₁-C₃)-alkyl, (C₁-C₃)-Alkylamino-(O)C, (C₁-C₃)-Alkylamino-(O)C-(C₁-C₃)-alkyl, Di-(C₁-C₃)-alkylamino-(O)C, Di-(C₁-C₃)-alkylamino-(O)C-(C₁-C₃)-alkyl, (C₁-C₃)-Alkyl-(O)C-amino, (C₁-C₃)-Alkyl-(O)ₙS-amino, (C₁-C₃)-Alkyl-(O)ₙS-(C₁-C₃)-alkylamino oder (C₁-C₃)-Alkyl-(O)ₙS-amino-(C₁-C₃)-alkyl, wobei die Heterocyclylgruppen und Arylgruppen durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, Phenyl, Cyano, Nitro und Halogen substituiert sind;
A bedeutet eine direkte Bindung oder (C₁-C₄)-Alkylen, wobei die Methylengruppen in (C₁-C₄)-Alkylen unabhängig voneinander n Reste aus der Gruppe bestehend aus Halogen, (C₁-C₄)-Alkyl, Halogen-(C₁-C₄)-alkyl, (C₁-C₄)-Alkoxy, Halogen-(C₁-C₄)-alkoxy oder (C₁-C₄)-Alkoxy-(C₁-C₄)-alkyl tragen können;
R⁵ bedeutet (C₁-C₆)-Alkyl, (C₃-C₆)-Cycloalkyl, (C₃-C₆)-Cycloalkyl-(C₁-C₆)-alkyl,(C₁-C₆)-Alkoxy-(C₁-C₆)-alkyl;
X¹ bedeutet N oder CR⁶;
X² bedeutet N oder CR⁷;
X³ bedeutet N oder CR⁸;
R⁶ bedeutet Wasserstoff, Halogen, (C₁-C₃)-Alkyl, (C₁-C₃)-Alkoxy, (C₂-C₃)-Alkenyl, (C₂-C₃)-Alkinyl, Halogen-(C₁-C₃)-alkyl, Halogen-(C₁-C₃)-alkoxy;
R⁷ bedeutet Wasserstoff, Halogen, (C₁-C₃)-Alkyl;
R⁸ bedeutet Wasserstoff, Hydroxy, Halogen, Cyano, Nitro, (C₁-C₆)-Alkyl, (C₃-C₆)-Cycloalkyl, Halogen-(C₁-C₆)-alkyl, (C₂-C₆)-Alkenyl, Halogen-(C₂-C₆)-alkenyl, (C₂-C₆)-Alkinyl, (C₁-C₆ )-Alkoxy, (C₂-C₆)-Alkenyloxy, (C₃-C₆)-Cycloalkyl-(C₁-C₃)-alkyl, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, Halogen-(C₁-C₆)-alkoxy, Halogen-(C₁-C₆)-alkoxy-(C₁-C₃)-alkyl, (C₁-C₆)-Alkyl-(O)ₙS, Halogen-(C₁-C₆)-alkyl-(O)ₙS, Aryl, Aryl-(O)ₙS, Heterocyclyl, Heterocyclyl-(O)ₙS, Aryloxy, Aryl-(C₂-C₆)-alkyl, Aryl-(C₁-C₆)-alkoxy, Heterocyclyloxy, Heterocyclyl-(C₁-C₃)-alkoxy-(C₁-C₃)-alkyl, HO(O)C, HO(O)C-(C₁-C₃)-alkoxy, (C₁-C₃)-Alkoxy-(O)C, (C₁-C₃)-Alkoxy-(O)C-(C₁-C₃)-alkoxy, (C₁-C₃)-Alkylamino, Oi-(C₁-C₃)-alkylamino, (C₁-C₃)-Alkylamino-(O)ₙS, (C₁-C₃)-Alkylamino-(O)ₙS-(C₁-C₃)-alkyl, Di-(C₁-C₃)-alkylamino-(O)ₙS, Di-(C₁-C₃)-alkylamino-(O)ₙS-(C₁-C₃)-alkyl, (C₁-C₃)-Alkylamino-(O)C, (C₁-C₃)-Alkylamino-(O)C-(C₁-C₃)-alkyl, Di-(C₁-C₃)-alkylamino-(O)C, Di-(C₁-C₃)-alkylamino-(O)C-(C₁-C₃)-alkyl, (C₁-C₃)-Alkyl-(O)C-amino, (C₁-C₃)-Alkyl-(O)ₙS-amino, (C₁-C₃)-Alkyl-(O)ₙS-(C₁-C₃)-alkylamino oder (C₁-C₃)-Alkyl-(O)ₙS-amino-(C₁-C₃)-alkyl, wobei die Heterocyclylgruppen und Arylgruppen durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, (C₁-C₆)-Alkyl-(O)ₙS, Phenyl, Cyano, Nitro und Halogen substituiert sind,
oder
R⁷ und R⁸ bilden gemeinsam mit den Kohlenstoffatomen, an denen sie gebunden sind, einen ungesättigten, fünf- oder sechsgliedrigen Ring, der s Stickstoffatome enthält und durch s Reste R¹⁰ substituiert ist;
R⁹ bedeutet Halogen, (C₁-C₃)-Alkyl, Halogen-(C₁-C₃)-alkyl, (C₁-C₆)-Alkoxy;
R¹⁰ bedeutet Cyano, Halogen, (C₁-C₃)-Alkyl-(O)ₙS, (C₁-C₃)-Alkyl, (C₂-C₃)-Alkenyl, (C₂-C₃)-Alkinyl, Halogen-(C₁-C₃)-alkyl oder Morpholinyl;
n bedeutet 0, 1 oder 2;
s bedeutet 0, 1, 2 oder 3,
mit der Maßgabe, dass R⁵ nicht (C₁-C₆)-Alkyl bedeutet, wenn A eine direkte Bindung ist.

2. 2-(Hetero)aryl-Pyridazinone nach Anspruch 1, worin
R¹ bedeutet Wasserstoff, Halogen, Cyano, (C₁-C₆)-Alkyl, (C₃-C₆)-Cycloalkyl, (C₂-C₆)-Alkenyl, (C₂-C₆)-Alkinyl, Halogen-(C₁-C₆)-alkyl, (C₁-C₆)-Alkoxy-(C₁-C₃)-alkyl, (C₃-C₆)-Cycloalkyl-(C₁-C₃)-alkyl, Amino oder (C₁-C₆)-Alkyl-(O)ₙS;
R² bedeutet Wasserstoff, Halogen, Cyano, (C₁-C₆)-Alkyl, (C₂-C₆)-Alkenyl, (C₂-C₆)-Alkinyl, Halogen-(C₁-C₆)-alkyl oder (C₁-C₆)-Alkyl-(O)ₙS;
R³ bedeutet Wasserstoff;
R⁴ bedeutet Hydroxy, Halogen, Cyano, Nitro, (C₁-C₆)-Alkyl, (C₃-C₆)-Cycloalkyl, Halogen-(C₁-C₆)-alkyl, (C₂-C₆)-Alkenyl, (C₂-C₆)-Alkinyl, (C₁-C₆)-Alkoxy, (C₁-C₆)-Alkoxy-(C₁-C₃)-alkyl, (C₁-C₆)-Alkoxy-(C₂-C₆)-alkoxy, (C₁-C₆)-Alkoxy-(C₂-C₆)-alkoxy-(C₁-C₃)-alkyl, Halogen-(C₁-C₆)-alkoxy, Halogen-(C₁-C₆)-alkoxy-(C₁-C₃)-alkyl, (C₁-C₆)-Alkyl-(O)ₙS, Halogen-(C₁-C₆)-alkyl-(O)ₙS, Aryl, Heterocyclyl, Aryloxy, Heterocyclyl-(C₁-C₃)-alkoxy-(C₁-C₃)-alkyl, (C₁-C₃)-Alkylamino, Oi-(C₁-C₃)-alkylamino, (C₁-C₃)-Alkylamino-(O)ₙS, (C₁-C₃)-Alkylamino-(O)ₙS-(C₁-C₃)-alkyl, Di-(C₁-C₃)-alkylamino-(O)ₙS, Di-(C₁-C₃)-alkylamino-(O)ₙS-(C₁-C₃)-alkyl, (C₁-C₃)-Alkylamino-(O)C, Di-(C₁-C₃)-alkylamino-(O)C, Di-(C₁-C₃)-alkylamino-(O)C-(C₁-C₃)-alkyl, (C₁-C₃)-Alkyl-(O)C-amino oder (C₁-C₃)-Alkyl-(O)ₙS-amino, wobei die Heterocyclylgruppen und Arylgruppen durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, Cyano, Nitro und Halogen substituiert sind;
A bedeutet eine direkte Bindung oder (C₁-C₄)-Alkylen;
R⁵ bedeutet (C₁-C₆)-Alkyl, (C₃-C₆)-Cycloalkyl, (C₃-C₆)-Cycloalkyl-(C₁-C₆)-alkyl, (C₁-C₆)-Alkoxy-(C₁-C₆)-alkyl;
X¹ bedeutet CR⁶;
X² bedeutet CR⁷;
X³ bedeutet CR⁸;
R⁶ und R⁷ bedeuten unabhängig voneinander Wasserstoff, Halogen oder (C₁-C₃)-Alkyl;
R⁸ bedeutet Wasserstoff, Halogen, Nitro, (C₁-C₆)-Alkyl, (C₃-C₆)-Cycloalkyl, Halogen-(C₁-C₆)-alkyl, (C₂-C₆)-Alkenyl, Halogen-(C₂-C₆)-alkenyl, (C₂-C₆)-Alkinyl, (C₁-C₆)-Alkoxy, (C₂-C₆)-Alkenyloxy, (C₃-C₆)-Cycloalkyl-(C₁-C₃)-alkyl, (C₁-C₆)-Alkoxy-(C₁-C₃)-alkyl, (C₁-C₆)-Alkoxy-(C₂-C₆)-alkoxy, Halogen-(C₁-C₆)-alkoxy, (C₁-C₆)-Alkyl-(O)ₙS oder Phenyl, wobei die Phenylgruppe durch s Reste aus der Gruppe bestehend aus (C₁-C₃)-Alkyl, Halogen-(C₁-C₃)-alkyl, (C₁-C₃)-Alkoxy, Halogen-(C₁-C₃)-alkoxy, (C₁-C₆)-Alkyl-(O)ₙS, Phenyl, Cyano, Nitro und Halogen substituiert ist;
n bedeutet 0, 1 oder 2;
s bedeutet 0, 1, 2 oder 3.

3. 2-(Hetero)aryl-Pyridazinone nach Anspruch 1 oder 2, worin
R¹ bedeutet Wasserstoff, Amino, Chlor, Brom, Cyano, Methyl, Ethyl, Isopropyl, Cyclopropyl, Vinyl, Propargyl, Isopropenyl oder Methyl-(O)ₙS;
R² bedeutet Wasserstoff, Halogen oder (C₁-C₆)-Alkyl;
R³ bedeutet Wasserstoff;
R⁴ bedeutet Fluor, Chlor, Cyano, Nitro, Methyl, Trifluormethyl, 2-Fluorethyl, Methoxyethoxymethyl, Trifluormethoxymethyl, Methyl-(O)ₙS, Aryl, Isoxazolinyl, Morpholinyl oder Methyl-(O)ₙS-amino, wobei die Heterocyclylgruppen und Arylgruppen durch s Reste aus der Gruppe bestehend aus Methyl, Trifluormethyl und Chlor substituiert sind;
A bedeutet eine direkte Bindung oder (C₁-C₄)-Alkylen;
R⁵ bedeutet (C₁-C₆)-Alkyl, (C₃-C₆)-Cycloalkyl, (C₃-C₆)-Cycloalkyl-(C₁-C₆)-alkyl, (C₁-C₆)-Alkoxy-(C₁-C₆)-alkyl;
X¹ bedeutet CR⁶;
X² bedeutet CR⁷;
X³ bedeutet CR⁸;
R⁶ und R⁷ bedeuten Wasserstoff;
R⁸ bedeutet Wasserstoff, Halogen, (C₁-C₆)-Alkyl, Halogen-(C₁-C₆)-alkyl, (C₂-C₆)-Alkenyl, (C₂-C₆)-Alkinyl, oder (C₁-C₆)-Alkyl-(O)ₙS;
n bedeutet 0, 1 oder 2;
s bedeutet 0, 1, 2 oder 3.

4. 2-(Hetero)aryl-Pyridazinone nach einem der Ansprüche 1 bis 3, worin
R¹ bedeutet Methyl oder Vinyl;
R² bedeutet Wasserstoff;
R³ bedeutet Wasserstoff;
R⁴ bedeutet Methyl, Chlor, Trifluormethyl oder Methyl-(O)ₙS;
A bedeutet eine direkte Bindung, -CH₂- oder -CH₂CH₂-;
R⁵ bedeutet Methyl, Ethyl, Cyclopropyl, Cyclopropylmethyl, Methoxyethyl; X¹ bedeutet CR⁶;
X² bedeutet CR⁷;
X³ bedeutet CR⁸;
R⁶ und R⁷ bedeuten Wasserstoff;
R⁸ bedeutet Methyl, Ethyl, Chlor, Trifluormethyl oder Methyl-(O)ₙS;
n bedeutet 0, 1 oder 2.

5. Herbizide Mittel, **gekennzeichnet durch** einen herbizid wirksamen Gehalt an mindestens einer Verbindung der Formel (I) gemäß einem der Ansprüche 1 bis 4.

6. Herbizide Mittel nach Anspruch 5 in Mischung mit Formulierungshilfsmitteln.

7. Herbizide Mittel gemäß Anspruch 5 oder 6 enthaltend mindestens einen weiteren pestizid wirksamen Stoff aus der Gruppe Insektizide, Akarizide, Herbizide, Fungizide, Safener und Wachstumsregulatoren.

8. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 4 oder eines herbiziden Mittels gemäß einem der Ansprüche 5 bis 7 auf die Pflanzen oder auf den Ort des unerwünschten Pflanzenwachstums appliziert.

9. Verwendung von Verbindungen der Formel (I) gemäß einem der Ansprüche 1 bis 4 oder von herbiziden Mitteln gemäß einem der Ansprüche 5 bis 7 zur Bekämpfung unerwünschter Pflanzen.

10. Verwendung gemäß Anspruch 9, **dadurch gekennzeichnet, daß** die Verbindungen der Formel (I) zur Bekämpfung unerwünschter Pflanzen in Kulturen von Nutzpflanzen eingesetzt werden.

11. Verwendung gemäß Anspruch 10, **dadurch gekennzeichnet, daß** die Nutzpflanzen transgene Nutzpflanzen sind.

## Claims

1. 2-(Hetero)arylpyridazinones of the formula (I) or salts thereof 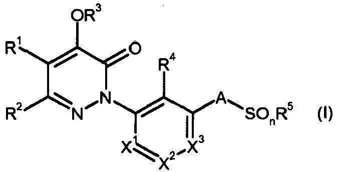 in which
R¹ represents hydrogen, halogen, cyano (C₁-C₆)-alkyl, (C₃-C₆)-cycloalkyl, (C₂-C₆)-alkenyl, (C₄-C₆)-cycloalkenyl, (C₂-C₆)-alkynyl, halo- (C₁-C₆)-alkyl, (C₁-C₆)-alkoxy, (C₁-C₆)-alkoxy- (C₁-C₃)-alkyl, (C₁-C₆)-alkoxy-(C₂-C₆)-alkoxy, (C₁-C₆) -alkoxy- (C₂-C₆)-alkoxy- (C₁-C₃)-alkyl, (C₃-C₆)-cycloalkyl- (C₁-C₃)-alkyl, amino, (C₁-C₆)-alkylamino, di-(C₁-C₆) -alkylamino, (C₁-C₃)-alkyl-(O)C-amino- (C₁-C₄) -alkyl, (C₁-C₆)-alkyl-(O)ₙS, (C₁-C₆)-alkyl-(O)ₙS-(C₁-C₃)-alkyl, halo-(C₁-C₆)-alkyl-(O)ₙS or halo-(C₁-C₆) -alkyl- (O)ₙS-(C₁-C₃)-alkyl;
R² represents hydrogen, hydroxy, halogen, nitro, amino, cyano, (C₁-C₆)-alkyl, (C₁-C₃)-alkoxy, (C₃-C₆)-cycloalkyl, (C₂-C₆)-alkenyl, (C₂-C₆)-alkynyl, halo-(C₁-C₆)-alkyl, (C₁-C₆)-alkoxy-(C₁-C₃)-alkyl, (C₃-C₆)-cycloalkyl- (C₁-C₃)-alkyl, (C₁-C₆)-alkyl- (O)ₙS, (C₁-C₆)-alkyl-(O)ₙS-(C₁-C₃)-alkyl, halo-(C₁-C₆)-alkyl-(O)ₙS, halo- (C₁-C₆) -alkyl- (O)ₙS-(C₁-C₃)-alkyl, (C₁-C₃)-alkylamino or di-(C₁-C₃)-alkylamino;
R³ represents hydrogen, (C₁-C₆)-alkyl-(O)C, aryl-(O)C, (C₁-C₆)-alkoxy-(O)C, (C₁-C₆) -alkyl- (O)ₙS, (C₁-C₆)-alkyl-(O)ₙS(O)C or aryl-(O)ₙS, where the aryl groups are in each case substituted by s radicals R⁹;
R⁴ represents hydroxy, halogen, cyano, nitro, (C₁-C₆)-alkyl, (C₃-C₆)-cycloalkyl, halo- (C₁-C₆)-alkyl, (C₂-C₆)-alkenyl, halo- (C₂-C₆)-alkenyl, (C₂-C₆)-alkynyl, (C₁-C₆)-alkoxy, (C₂-C₆)-alkenyloxy, (C₃-C₆) -cycloalkyl- (C₁-C₃)-alkyl, (C₁-C₆)-alkoxy- (C₁-C₃)-alkyl, (C₁-C₆) -alkoxy- (C₂-C₆)-alkoxy, (C₁-C₆) -alkoxy- (C₂-C₆)-alkoxy- (C₁-C₃)-alkyl, halo-(C₁-C₆)-alkoxy, halo- (C₁-C₆) -alkoxy- (C₁-C₃)-alkyl, (C₁-C₆)-alkyl-(O)ₙS, halo- (C₁-C₆)-alkyl- (O)ₙS, aryl, aryl-(0)nS, heterocyclyl, heterocyclyl-(O)ₙS, aryloxy, aryl-(C₂-C₆)-alkyl, aryl- (C₁-C₆)-alkoxy, heterocyclyloxy, heterocyclyl- (C₁-C₃)-alkoxy- (C₁-C₃)-alkyl, HO(O)C, HO(O)C-(C₁-C₃)-alkoxy, (C₁-C₃)-alkoxy-(O)C, (C₁-C₃)-alkoxy- (O)C-(C₁-C₃)-alkoxy, (C₁-C₃)-alkylamino, di-(C₁-C₃)-alkylamino, (C₁-C₃)-alkylamino-(O)ₙS, (C₁-C₃)-alkylamino-(O)ₙS-(C₁-C₃)-alkyl, di-(C₁-C₃)-alkylamino-(O)ₙS, di-(C₁-C₃)-alkylamino-(O)ₙS-(C₁-C₃)-alkyl, (C₁-C₃)-alkylamino-(O)C, (C₁-C₃)-alkylamino-(O)C-(C₁-C₃)-alkyl, di-(C₁-C₃)-alkylamino-(O)C, di-(C₁-C₃)-alkylamino-(O)C-(C₁-C₃)-alkyl, (C₁-C₃)-alkyl-(O)C-amino, (C₁-C₃)-alkyl-(O)ₙS-amino, (C₁-C₃)-alkyl-(O)ₙS- (C₁-C₃)-alkylamino or (C₁-C₃)-alkyl-(O)ₙS-amino-(C₁-C₃)-alkyl, where the heterocyclyl groups and aryl groups are substituted by s radicals from the group consisting of (C₁-C₃)-alkyl, halo- (C₁-C₃)-alkyl, (C₁-C₃)-alkoxy, halo- (C₁-C₃)-alkoxy, phenyl, cyano, nitro and halogen;
A represents a direct bond or (C₁-C₄)-alkylene, where the methylene groups in (C₁-C₄)-alkylene independently of one another may carry n radicals from the group consisting of halogen, (C₁-C₄)-alkyl, halo- (C₁-C₄)-alkyl, (C₁-C₄)-alkoxy, halo- (C₁-C₄) -alkoxy or (C₁-C₄) -alkoxy- (C₁-C₄)-alkyl;
R⁵ represents (C₁-C₆)-alkyl, (C₃-C₆)-cycloalkyl, (C₃-C₆)-cycloalkyl-(C₁-C₆)-alkyl, (C₁-C₆)-alkoxy- (C₁-C₆)-alkyl;
X¹ represents N or CR⁶;
X² represents N or CR⁷;
X³ represents N or CR⁸;
R⁶ represents hydrogen, halogen, (C₁-C₃)-alkyl, (C₁-C₃)-alkoxy, (C₂-C₃)-alkenyl, (C₂-C₃)-alkynyl, halo-(C₁-C₃)-alkyl, halo-(C₁-C₃)-alkoxy;
R⁷ represents hydrogen, halogen, (C₁-C₃)-alkyl;
R⁸ represents hydrogen, hydroxy, halogen, cyano, nitro, (C₁-C₆)-alkyl, (C₃-C₆)-cycloalkyl, halo- (C₁-C₆)-alkyl, (C₂-C₆)-alkenyl, halo- (C₂-C₆) -alkenyl, (C₂-C₆)-alkynyl, (C₁-C₆)-alkoxy, (C₂-C₆)-alkenyloxy, (C₃-C₆)-cycloalkyl- (C₁-C₃)-alkyl, (C₁-C₆)-alkoxy- (C₁-C₃)-alkyl, (C₁-C₆)-alkoxy-(C₂-C₆)-alkoxy, (C₁-C₆)-alkoxy- (C₂-C₆)-alkoxy-(C₁-C₃)-alkyl, halo- (C₁-C₆)-alkoxy, halo-(C₁-C₆)-alkoxy-(C₁-C₃)-alkyl, (C₁-C₆)-alkyl-(O)ₙS, halo-(C₁-C₆)-alkyl-(O)ₙS, aryl, aryl-(O)ₙS, heterocyclyl, heterocyclyl-(O)ₙS, aryloxy, aryl- (C₂-C₆) -alkyl, aryl-(C₁-C₆)-alkoxy, heterocyclyloxy, heterocyclyl-(C₁-C₃)-alkoxy-(C₁-C₃)-alkyl, HO(O)C, HO(O)C-(C₁-C₃)-alkoxy, (C₁-C₃)-alkoxy-(O)C, (C₁-C₃)-alkoxy-(O)C-(C₁-C₃)-alkoxy, (C₁-C₃)-alkylamino, di-(C₁-C₃)-alkylamino, (C₁-C₃)-alkylamino-(O)ₙS, (C₁-C₃)-alkylamino-(O)ₙS-(C₁-C₃)-alkyl, di-(C₁-C₃)-alkylamino-(O)ₙS, di-(C₁-C₃)-alkylamino-(O)ₙS-(C₁-C₃)-alkyl, (C₁-C₃) -alkylamino- (O)C, (C₁-C₃)-alkylamino-(O)C-(C₁-C₃)-alkyl, di-(C₁-C₃)-alkylamino-(O)C, di- (C₁-C₃) -alkylamino-(O)C-(C₁-C₃)-alkyl, (C₁-C₃)-alkyl-(O)C-amino, (C₁-C₃)-alkyl (O)ₙS-amino, (C₁-C₃)-alkyl-(O)ₙS-(C₁-C₃)-alkylamino or (C₁-C₃)-alkyl-(O)ₙS-amino-(C₁-C₃)-alkyl, where the heterocyclyl groups and aryl groups are substituted by s radicals from the group consisting of (C₁-C₃)-alkyl, halo- (C₁-C₃)-alkyl, (C₁-C₃)-alkoxy, halo- (C₁-C₃)-alkoxy, (C₁-C₆) -alkyl- (O)ₙS, phenyl, cyano, nitro and halogen,
or
R⁷ and R⁸ together with the carbon atoms to which they are attached represent an unsaturated five- or six-membered ring which contains s nitrogen atoms and is substituted by s radicals R¹⁰;
R⁹ represents halogen, (C₁-C₃)-alkyl, halo-(C₁-C₃)-alkyl, (C₁-C₆)-alkoxy,
R¹⁰ represents cyano, halogen, (C₁-C₃)-alkyl-(O)ₙS, (C₁-C₃) -alkyl, (C₂-C₃)-alkenyl, (C₂-C₃)-alkynyl, halo-(C₁-C₃)-alkyl or morpholinyl;
n represents 0, 1 or 2;
s represents 0, 1, 2 or 3,
with the proviso that R⁵ does not represent (C₁-C₆)-alkyl if A represents a direct bond.

2. 2-(Hetero)arylpyridazinones according to Claim 1 in which
R¹ represents hydrogen, halogen, cyano, (C₁-C₆)-alkyl, (C₃-C₆) -cycloalkyl, (C₂-C₆)-alkenyl, (C₂-C₆) -alkynyl, halo- (C₁-C₆)-alkyl, (C₁-C₆)-alkoxy- (C₁-C₃)-alkyl, (C₃-C₆)-cycloalkyl-(C₁-C₃)-alkyl, amino or (C₁-C₆)-alkyl-(O)nS;
R² represents hydrogen, halogen, cyano, (C₁-C₆)-alkyl, (C₂-C₆)-alkenyl, (C₂-C₆)-alkynyl, halo- (C₁-C₆)-alkyl or (C₁-C₆)-alkyl-(O)ₙS;
R³ represents hydrogen,
R⁴ represents hydroxy, halogen, cyano, nitro, (C₁-C₆)-alkyl, (C₃-C₆)-cycloalkyl, halo-(C₁-C₆)-alkyl, (C₂-C₆)-alkenyl, (C₂-C₆)-alkynyl, (C₁-C₆)-alkoxy, (C₁-C₆)-alkoxy-(C₁-C₃)-alkyl, (C₁-C₆)-alkoxy-(C₂-C₆)-alkoxy, (C₁-C₆)-alkoxy-(C₂-C₆)-alkoxy-(C₁-C₃)-alkyl, halo-(C₁-C₆)-alkoxy, halo-(C₁-C₆)-alkoxy-(C₁-C₃)-alkyl, (C₁-C₆)-alkyl-(O)ₙS, halo-(C₁-C₆)-alkyl-(O)ₙS, aryl, heterocyclyl, aryloxy, heterocyclyl-(C₁-C₃)-alkoxy-(C₁-C₃)-alkyl, (C₁-C₃)-alkylamino, di-(C₁-C₃)-alkylamino, (C₁-C₃)-alkylamino-(O)ₙS, (C₁-C₃)-alkylamino-(O)ₙS-(C₁-C₃)-alkyl, di-(C₁-C₃)-alkylamino-(O)ₙS, di-(C₁-C₃)-alkylamino-(O)ₙS-(C₁-C₃)-alkyl, (C₁-C₃)-alkylamino-(O)C, di-(C₁-C₃)-alkylamino-(O)C, di-(C₁-C₃)-alkylamino-(O)C-(C₁-C₃) -alkyl, (C₁-C₃)-alkyl-(O)C-amino or (C₁-C₃)-alkyl-(O)ₙS-amino, where the heterocyclyl groups and aryl groups are substituted by s radicals from the group consisting of (C₁-C₃) -alkyl, halo- (C₁-C₃)-alkyl, (C₁-C₃)-alkoxy, halo-(C₁-C₃) -alkoxy, cyano, nitro and halogen;
A represents a direct bond or (C₁-C₄)-alkylene;
R⁵ represents (C₁-C₆)-alkyl, (C₃-C₆)-cycloalkyl, (C₃-C₆)-cycloalkyl-(C₁-C₆)-alkyl, (C₁-C₆)-alkoxy-(C₁-C₆)-alkyl;
X¹ represents CR⁶;
X² represents CR⁷;
X³ represents CR⁸;
R⁶ and R⁷ independently of one another represent hydrogen, halogen, or (C₁-C₃)-alkyl;
R⁸ represents hydrogen, halogen, nitro, (C₁-C₆) -alkyl, (C₃-C₆) -cycloalkyl, halo- (C₁-C₆) -alkyl, (C₂-C₆) -alkenyl, halo- (C₂-C₆) -alkenyl, (C₂-C₆) -alkynyl, (C₁-C₆) -alkoxy, (C₂-C₆)-alkenyloxy, (C₃-C₆)-cycloalkyl- (C₁-C₃)-alkyl, (C₁-C₆)-alkoxy- (C₁-C₃)-alkyl, (C₁-C₆) -alkoxy- (C₂-C₆)-alkoxy, halo- (C₁-C₆)-alkoxy, (C₁-C₆)-alkyl-(O)ₙS or phenyl, where the phenyl group is substituted by s radicals from the group consisting of (C₁-C₃)-alkyl, halo- (C₁-C₃) -alkyl, (C₁-C₃) -alkoxy, halo- (C₁-C₃) -alkoxy, (C₁-C₆) -alkyl- (O) ₙS, phenyl, cyano, nitro and halogen;
n represents 0, 1 or 2;
s represents 0, 1, 2 or 3.

3. 2-(Hetero)arylpyridazinones according to Claim 1 or 2 in which
R¹ represents hydrogen, amino, chlorine, bromine, cyano, methyl, ethyl, isopropyl, cyclopropyl, vinyl, propargyl, isopropenyl or methyl-(O)ₙS;
R² represents hydrogen, halogen or (C₁-C₆)-alkyl,
R³ represents hydrogen,
R⁴ represents fluorine, chlorine, cyano, nitro, methyl, trifluoromethyl, 2-fluoroethyl, methoxyethoxymethyl, trifluoromethoxymethyl, methyl-(O)ₙS, aryl, isoxazolinyl, morpholinyl or methyl-(O)ₙS-amino, where the heterocyclyl groups and aryl groups are substituted by s radicals from the group consisting of methyl, trifluoromethyl and chlorine;
A represents a direct bond or (C₁-C₄)-alkylene;
R⁵ represents (C₁-C₆)-alkyl, (C₃-C₆)-cycloalkyl, (C₃-C₆) -cycloalkyl- (C₁-C₆) -alkyl, (C₁-C₆) -alkoxy- (C₁-C₆)-alkyl;
X¹ represents CR⁶;
X² represents CR⁷;
X³ represents CR⁸;
R⁶ and R⁷ represent hydrogen;
R⁸ represents hydrogen, halogen, (C₁-C₆)-alkyl, halo-(C₁-C₆) -alkyl, (C₂-C₆) -alkenyl, (C₂-C₆) -alkynyl or (C₁-C₆)-alkyl-(O)ₙS;
n represents 0, 1 or 2;
s represents 0, 1, 2 or 3.

4. 2-(Hetero)arylpyridazinones according to any of Claims 1 to 3 in which
R¹ represents methyl or vinyl;
R² represents hydrogen;
R³ represents hydrogen;
R⁴ represents methyl, chlorine, trifluoromethyl or methyl-(O)ₙS;
A represents a direct bond, -CH₂- or -CH₂CH₂-;
R⁵ represents methyl, ethyl, cyclopropyl, cyclopropylmethyl, methoxyethyl;
X¹ represents CR⁶;
X² represents CR⁷;
X³ represents CR⁸;
R⁶ and R⁷ represent hydrogen,
R⁸ represents methyl, ethyl, chlorine, trifluoromethyl or methyl-(O)nS;
n represents 0, 1 or 2.

5. Herbicidal compositions **characterized by** a herbicidally active content of at least one compound of the formula (I) according to any of Claims 1 to 4.

6. Herbicidal compositions according to Claim 5 in a mixture with formulation auxiliaries.

7. Herbicidal compositions according to Claim 5 or 6, comprising at least one further pesticidally active substance from the group consisting of insecticides, acaricides, herbicides, fungicides, safeners, and growth regulators.

8. Method for controlling unwanted plants, **characterized in that** an effective amount of at least one compound of the formula (I) according to any of Claims 1 to 4 or of a herbicidal composition according to any of Claims 5 to 7 is applied to the plants or to the site of the unwanted vegetation.

9. Use of compounds of the formula (I) according to any of Claims 1 to 4 or of herbicidal compositions according to any of Claims 5 to 7 for controlling unwanted plants.

10. Use according to Claim 9, **characterized in that** the compounds of the formula (I) are used for controlling unwanted plants in crops of useful plants.

11. Use according to Claim 10, **characterized in that** the useful plants are transgenic useful plants.

## Revendications

1. 2-(Hétéro)aryl-pyridazinones de formule (I) ou leurs sels 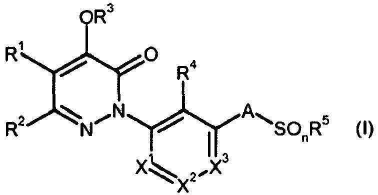 dans lesquelles
R¹ signifie hydrogène, halogène, cyano, alkyle en (C₁-C₆), cycloalkyle en (C₃-C₆), alcényle en (C₂-C₆), cycloalcényle en (C₄-C₆), alcynyle en (C₂-C₆), halogéno-alkyle en (C₁-C₆), alcoxy en (C₁-C₆), alcoxy en (C₁-C₆)-alkyle en (C₁-C₃), alcoxy en (C₁-C₆) -alcoxy en (C₂-C₆), alcoxy en (C₁-C₆) -alcoxy en (C₂-C₆) -alkyle en (C₁-C₃), cycloalkyle en (C₃-C₆) -alkyle en (C₁-C₃), amino, alkylamino en (C₁-C₆), di-alkylamino en (C₁-C₆), alkyle en (C₁-C₃)-(O)C-amino-alkyle en (C₁-C₄), alkyle en (C₁-C₆)-(O)ₙS, alkyle en (C₁-C₆)-(O)ₙS-alkyle en (C₁-C₃), halogéno-alkyle en (C₁-C₆)-(O)ₙS ou halogéno-alkyle en (C₁-C₆)-(O)ₙS-alkyle en (C₁-C₃) ;
R² signifie hydrogène, hydroxy, halogène, nitro, amino, cyano, alkyle en (C₁-C₆), alcoxy en (C₁-C₃), cycloalkyle en (C₃-C₆), alcényle en (C₂-C₆), alcynyle en (C₂-C₆), halogéno-alkyle en (C₁-C₆), alcoxy en (C₁-C₆)-alkyle en (C₁-C₃), cycloalkyle en (C₃-C₆)-alkyle en (C₁-C₃), alkyle en (C₁-C₆)-(O)ₙS, alkyle en (C₁-C₆)-(O)ₙS-alkyle en (C₁-C₃), halogéno-alkyle en (C₁-C₆)-(O)ₙS, halogéno-alkyle en (C₁-C₆)-(O)ₙS-alkyle en (C₁-C₃), alkylamino en (C₁-C₃) ou di-alkylamino en (C₁-C₃) ;
R³ signifie hydrogène, alkyle en (C₁-C₆) -(O)C, aryl-(O)C, alcoxy en (C₁-C₆)-(O)C, alkyle en (C₁-C₆)-(O)ₙS, alkyle en (C₁-C₆)-(O)ₙS(O)C ou aryl-(O)ₙS, les groupes aryle étant chacun substitués par s radicaux R⁹ ;
R⁴ signifie hydroxy, halogène, cyano, nitro, alkyle en (C₁-C₆), cycloalkyle en (C₃-C₆), halogéno-alkyle en (C₁-C₆), alcényle en (C₂-C₆), halogéno-alcényle en (C₂-C₆), alcynyle en (C₂-C₆), alcoxy en (C₁-C₆), alcényloxy en (C₂-C₆), cycloalkyle en (C₃-C₆)-alkyle en (C₁-C₃), alcoxy en (C₁-C₆)-alkyle en (C₁-C₃), alcoxy en (C₁-C₆)-alcoxy en (C₂-C₆), alcoxy en (C₁-C₆)-alcoxy en (C₂-C₆)-alkyle en (C₁-C₃), halogéno-alcoxy en (C₁-C₆), halogéno-alcoxy en (C₁-C₆) -alkyle en (C₁-C₃), alkyle en (C₁-C₆)-(O)ₙS, halogéno-alkyle en (C₁-C₆)-(O)ₙS, aryle, aryl-(O)ₙS, hétérocyclyle, hétérocyclyl-(O)ₙS, aryloxy, aryl-alkyle en (C₂-C₆), aryl-alcoxy en (C₁-C₆), hétérocyclyloxy, hétérocyclyl-alcoxy en (C₁-C₃)-alkyle en (C₁-C₃), HO(O)C, HO(O)C-alcoxy en (C₁-C₃), alcoxy en (C₁-C₃) - (O)C, alcoxy en (C₁-C₃) - (O)C-alcoxy en (C₁-C₃), alkylamino en (C₁-C₃), di-alkylamino en (C₁-C₃), alkylamino en (C₁-C₃)-(O)ₙS, alkylamino en (C₁-C₃)-(O)ₙS-alkyle en (C₁-C₃), di-alkylamino en (C₁-C₃)-(O)ₙS, di-alkylamino en (C₁-C₃)-(O)ₙS-alkyle en (C₁-C₃), alkylamino en (C₁-C₃) - (O)C, alkylamino en (C₁-C₃)-(O)C-alkyle en (C₁-C₃), di-alkylamino en (C₁-C₃)-(O)C, di-alkylamino en (C₁-C₃) - (O)C-alkyle en (C₁-C₃), alkyle en (C₁-C₃) - (O)C-amino, alkyle en (C₁-C₃)- (O)ₙS-amino, alkyle en (C₁-C₃)-(O)ₙS-alkylamino en (C₁-C₃) ou alkyle en (C₁-C₃)-(O)ₙS-amino-alkyle en (C₁-C₃), les groupes hétérocyclyle et les groupes aryle étant substitués par s radicaux du groupe constitué par alkyle en (C₁-C₃), halogéno-alkyle en (C₁-C₃), alcoxy en (C₁-C₃), halogéno-alcoxy en (C₁-C₃), phényle, cyano, nitro et halogène ;
A signifie une liaison directe ou alkylène en (C₁-C₄), les groupes méthylène dans l'alkylène en (C₁-C₄) pouvant porter indépendamment les uns des autres n radicaux du groupe constitué par halogène, alkyle en (C₁-C₄), halogéno-alkyle en (C₁-C₄), alcoxy en (C₁-C₄), halogéno-alcoxy en (C₁-C₄) ou alcoxy en (C₁-C₄)-alkyle en (C₁-C₄) ;
R⁵ signifie alkyle en (C₁-C₆), cycloalkyle en (C₃-C₆), cycloalkyle en (C₃-C₆)-alkyle en (C₁-C₆), alcoxy en (C₁-C₆)-alkyle en (C₁-C₆) ;
X¹ signifie N ou CR⁶ ;
X² signifie N ou CR⁷ ;
X³ signifie N ou CR⁸ ;
R⁶ signifie hydrogène, halogène, alkyle en (C₁-C₃), alcoxy en (C₁-C₃), alcényle en (C₂-C₃), alcynyle en (C₂-C₃), halogéno-alkyle en (C₁-C₃), halogéno-alcoxy en (C₁-C₃) ;
R⁷ signifie hydrogène, halogène, alkyle en (C₁-C₃) ;
R⁸ signifie hydrogène, hydroxy, halogène, cyano, nitro, alkyle en (C₁-C₆), cycloalkyle en (C₃-C₆), halogéno-alkyle en (C₁-C₆), alcényle en (C₂-C₆), halogéno-alcényle en (C₂-C₆), alcynyle en (C₂-C₆), alcoxy en (C₁-C₆), alcényloxy en (C₂-C₆), cycloalkyle en (C₃-C₆)-alkyle en (C₁-C₃), alcoxy en (C₁-C₆) -alkyle en (C₁-C₃), alcoxy en (C₁-C₆)-alcoxy en (C₂-C₆), alcoxy en (C₁-C₆)-alcoxy en (C₂-C₆)-alkyle en (C₁-C₃), halogéno-alcoxy en (C₁-C₆), halogéno-alcoxy en (C₁-C₆)-alkyle en (C₁-C₃), alkyle en (C₁-C₆) - (O)ₙS, halogéno-alkyle en (C₁-C₆)-(O)ₙS, aryle, aryl-(O)ₙS, hétérocyclyle, hétérocyclyl-(O)ₙS, aryloxy, aryl-alkyle en (C₂-C₆), aryl-alcoxy en (C₁-C₆), hétérocyclyloxy, hétérocyclyl-alcoxy en (C₁-C₃)-alkyle en (C₁-C₃), HO(O)C, HO(O)C-alcoxy en (C₁-C₃), alcoxy en (C₁-C₃)-(O)C, alcoxy en (C₁-C₃)-(O)C-alcoxy en (C₁-C₃), alkylamino en (C₁-C₃), di-alkylamino en (C₁-C₃), alkylamino en (C₁-C₃)-(O)ₙS, alkylamino en (C₁-C₃)-(O)ₙS-alkyle en (C₁-C₃), di-alkylamino en (C₁-C₃) - (O) ₙS, di-alkylamino en (C₁-C₃)-(O)ₙS-alkyle en (C₁-C₃), alkylamino en (C₁-C₃) - (O)C, alkylamino en (C₁-C₃)-(O)C-alkyle en (C₁-C₃), di-alkylamino en (C₁-C₃)-(O)C, di-alkylamino en (C₁-C₃) - (O)C-alkyle en (C₁-C₃), alkyle en (C₁-C₃)-(O)C-amino, alkyle en (C₁-C₃)-(O)ₙS-amino, alkyle en (C₁-C₃)-(O)ₙS-alkylamino en (C₁-C₃) ou alkyle en (C₁-C₃)-(O)ₙS-amino-alkyle en (C₁-C₃), les groupes hétérocyclyle et les groupes aryle étant substitués par s radicaux du groupe constitué par alkyle en (C₁-C₃), halogéno-alkyle en (C₁-C₃), alcoxy en (C₁-C₃), halogéno-alcoxy en (C₁-C₃), alkyle en (C₁-C₆)-(O)ₙS, phényle, cyano, nitro et halogène ;
ou
R⁷ et R⁸ forment ensemble avec les atomes de carbone auxquels ils sont reliés un cycle insaturé à cinq ou six chaînons, qui contient s atomes d'azote et est substitué par s radicaux R¹⁰ ;
R⁹ signifie halogène, alkyle en (C₁-C₃), halogéno-alkyle en (C₁-C₃), alcoxy en (C₁-C₆) ;
R¹⁰ signifie cyano, halogène, alkyle en (C₁-C₃)-(O)ₙS, alkyle en (C₁-C₃), alcényle en (C₂-C₃), alcynyle en (C₂-C₃), halogéno-alkyle en (C₁-C₃) ou morpholinyle ;
n signifie 0, 1 ou 2 ;
s signifie 0, 1, 2 ou 3,
à condition que R⁵ ne signifie pas alkyle en (C₁-C₆) lorsqu'A est une liaison directe.

2. 2-(Hétéro)aryl-pyridazinones selon la revendication 1, dans lesquelles
R¹ signifie hydrogène, halogène, cyano, alkyle en (C₁-C₆), cycloalkyle en (C₃-C₆), alcényle en (C₂-C₆), alcynyle en (C₂-C₆), halogéno-alkyle en (C₁-C₆), alcoxy en (C₁-C₆)-alkyle en (C₁-C₃), cycloalkyle en (C₃-C₆)-alkyle en (C₁-C₃), amino ou alkyle en (C₁-C₆)-(O)ₙS ;
R² signifie hydrogène, halogène, cyano, alkyle en (C₁-C₆), alcényle en (C₂-C₆), alcynyle en (C₂-C₆), halogéno-alkyle en (C₁-C₆) ou alkyle en (C₁-C₆)-(O)ₙS ;
R³ signifie hydrogène ;
R⁴ signifie hydroxy, halogène, cyano, nitro, alkyle en (C₁-C₆), cycloalkyle en (C₃-C₆), halogéno-alkyle en (C₁-C₆) , alcényle en (C₂-C₆), alcynyle en (C₂-C₆), alcoxy en (C₁-C₆), alcoxy en (C₁-C₆)-alkyle en (C₁-C₃), alcoxy en (C₁-C₆)-alcoxy en (C₂-C₆), alcoxy en (C₁-C₆)-alcoxy en (C₂-C₆) -alkyle en (C₁-C₃), halogéno-alcoxy en (C₁-C₆), halogéno-alcoxy en (C₁-C₆) -alkyle en (C₁-C₃), alkyle en (C₁-C₆)-(O)ₙS, halogéno-alkyle en (C₁-C₆)-(O)ₙS, aryle, hétérocyclyle, aryloxy, hétérocyclyl-alcoxy en (C₁-C₃)-alkyle en (C₁-C₃), alkylamino en (C₁-C₃), di-alkylamino en (C₁-C₃), alkylamino en (C₁-C₃)-(O)ₙS, alkylamino en (C₁-C₆) - (O)ₙS-alkyle en (C₁-C₃), di-alkylamino en (C₁-C₃)-(O)ₙS, di-alkylamino en (C₁-C₃)-(O)ₙS-alkyle en (C₁-C₃), alkylamino en (C₁-C₃)-(O)C, di-alkylamino en (C₁-C₃)-(O)C, di-alkylamino en (C₁-C₃) - (O)C-alkyle en (C₁-C₃), alkyle en (C₁-C₃)-(O)C-amino ou alkyle en (C₁-C₃)-(O)ₙS-amino, les groupes hétérocyclyle et les groupes aryle étant substitués par s radicaux du groupe constitué par alkyle en (C₁-C₃), halogéno-alkyle en (C₁-C₃), alcoxy en (C₁-C₃), halogéno-alcoxy en (C₁-C₃), cyano, nitro et halogène ;
A signifie une liaison directe ou alkylène en (C₁-C₄) ;
R⁵ signifie alkyle en (C₁-C₆), cycloalkyle en (C₃-C₆), cycloalkyle en (C₃-C₆) -alkyle en (C₁-C₆), alcoxy en (C₁-C₆)-alkyle en (C₁-C₆) ;
X¹ signifie CR⁶ ;
X² signifie CR⁷ ;
X³ signifie CR⁸ ;
R⁶ et R7 signifient indépendamment l'un de l'autre hydrogène, halogène ou alkyle en (C₁-C₃) ;
R⁸ signifie hydrogène, halogène, nitro, alkyle en (C₁-C₆), cycloalkyle en (C₃-C₆), halogéno-alkyle en (C₁-C₆), alcényle en (C₂-C₆), halogéno-alcényle en (C₂-C₆), alcynyle en (C₂-C₆), alcoxy en (C₁-C₆), alcényloxy en (C₂-C₆), cycloalkyle en (C₃-C₆)-alkyle en (C₁-C₃), alcoxy en (C₁-C₆)-alkyle en (C₁-C₃), alcoxy en (C₁-C₆)-alcoxy en (C₂-C₆), halogéno-alcoxy en (C₁-C₆), alkyle en (C₁-C₆)-(O)ₙS ou phényle, le groupe phényle étant substitué par s radicaux du groupe constitué par alkyle en (C₁-C₃), halogéno-alkyle en (C₁-C₃), alcoxy en (C₁-C₃), halogéno-alcoxy en (C₁-C₃), alkyle en (C₁-C₆) - (O)ₙS, phényle, cyano, nitro et halogène ;
n signifie 0, 1 ou 2 ;
s signifie 0, 1, 2 ou 3.

3. 2-(Hétéro)aryl-pyridazinones selon la revendication 1 ou 2, dans lesquelles
R¹ signifie hydrogène, amino, chlore, brome, cyano, méthyle, éthyle, isopropyle, cyclopropyle, vinyle, propargyle, isopropényle ou méthyl-(O)ₙS ;
R² signifie hydrogène, halogène ou alkyle en (C₁-C₆) ; R³ signifie hydrogène ;
R⁴ signifie fluor, chlore, cyano, nitro, méthyle, trifluorométhyle, 2-fluoroéthyle, méthoxyéthoxyméthyle, trifluorométhoxyméthyle, méthyl-(O)ₙS, aryle, isoxazolinyle, morpholinyle ou méthyl-(O)ₙS-amino, les groupes hétérocyclyle et les groupes aryle étant substitués par s radicaux du groupe constitué par méthyle, trifluorométhyle et chlore ;
A signifie une liaison directe ou alkylène en (C₁-C₄) ;
R⁵ signifie alkyle en (C₁-C₆), cycloalkyle en (C₃-C₆), cycloalkyle en (C₃-C₆)-alkyle en (C₁-C₆), alcoxy en (C₁-C₆)-alkyle en (C₁-C₆) ;
X¹ signifie CR⁶ ;
X² signifie CR⁷ ;
X³ signifie CR⁸ ;
R⁶ et R⁷ signifient hydrogène ;
R⁸ signifie hydrogène, halogène, alkyle en (C₁-C₆), halogéno-alkyle en (C₁-C₆), alcényle en (C₂-C₆), alcynyle en (C₂-C₆) ou alkyle en (C₁-C₆) - (O) ₙS ;
n signifie 0, 1 ou 2 ;
s signifie 0, 1, 2 ou 3.

4. 2-(Hétéro)aryl-pyridazinones selon l'une quelconque des revendications 1 à 3, dans lesquelles R¹ signifie méthyle ou vinyle ;
R² signifie hydrogène ;
R³ signifie hydrogène ;
R⁴ signifie méthyle, chlore, trifluorométhyle ou méthyl-(O)ₙS ;
A signifie une liaison directe, -CH₂- ou -CH₂CH₂- ;
R⁵ signifie méthyle, éthyle, cyclopropyle, cyclopropylméthyle, méthoxyéthyle ;
X¹ signifie CR⁶ ;
X² signifie CR⁷ ;
X³ signifie CR⁸ ;
R⁶ et R⁷ signifient hydrogène ;
R⁸ signifie méthyle, éthyle, chlore, trifluorométhyle ou méthyl-(O)ₙS ;
n signifie 0, 1 ou 2.

5. Agents herbicides, **caractérisés par** une teneur herbicide efficace en au moins un composé de formule (I) selon l'une quelconque des revendications 1 à 4.

6. Agents herbicides selon la revendication 5, en mélange avec des adjuvants de formulation.

7. Agents herbicides selon la revendication 5 ou 6, contenant au moins une substance à effet pesticide supplémentaire du groupe constitué par les insecticides, les acaricides, les herbicides, les fongicides, les agents protecteurs et les régulateurs de croissance.

8. Procédé de lutte contre des plantes indésirables, **caractérisé en ce qu'**une quantité efficace d'au moins un composé de formule (I) selon l'une quelconque des revendications 1 à 4 ou d'un agent herbicide selon l'une quelconque des revendications 5 à 7 est appliquée sur les plantes ou à l'emplacement de la végétation indésirable.

9. Utilisation de composés de formule (I) selon l'une quelconque des revendications 1 à 4 ou d'agents herbicides selon l'une quelconque des revendications 5 à 7 pour lutter contre des plantes indésirables.

10. Utilisation selon la revendication 9, **caractérisée en ce que** les composés de formule (I) sont utilisés pour lutter contre des plantes indésirables dans des cultures de plantes utiles.

11. Utilisation selon la revendication 10, **caractérisée en ce que** les plantes utiles sont des plantes utiles transgéniques.
